(19) Europäisches Patentamt
European Patent Office
Office européen des brevets

(11) Numéro de publication: **0 246 126**
**A1**

(12) # DEMANDE DE BREVET EUROPEEN

(21) Numéro de dépôt: **87400875.8**

(22) Date de dépôt: **16.04.87**

(51) Int. Cl.³: **C 07 D 235/28**
C 07 D 401/12, C 07 D 413/1- 2
C 07 D 471/04, C 07 D 513/0- 4
C 07 D 513/14, C 07 D 403/1- 2

(30) Priorité: **21.04.86 FR 8605721**

(43) Date de publication de la demande:
**19.11.87 Bulletin 87/47**

(84) Etats contractants désignés:
**AT BE CH DE FR GB GR IT LI LU NL SE**

(71) Demandeur: **LABORATORIOS DEL DR. ESTEVE, S.A.**
**Av. Mare de Deu de Montserrat, 221**
**E-08026 Barcelona(ES)**

(72) Inventeur: **Frigola Constansa, Jordi**
**Av. Daigonal, 299 at.1a**
**ES-08013 Barcelone(ES)**

(72) Inventeur: **Pares Corominas, Juan**
**Padilla, 349 3o 3a**
**ES-08025 Barcelone(ES)**

(72) Inventeur: **Colombo Pinol, Augusto**
**Av. Ch... , 36, 4o 1a**
**ES-08032 Barcelone(ES)**

(74) Mandataire: **Ahner, Francis et al,**
**CABINET REGIMBEAU 26, avenue Kléber**
**F-75116 Paris(FR)**

(54) **Benzimidazoles et imidazopyridines sulfonamides, leur préparation et leur application en tant que médicaments.**

(57) Dérivés de benzimidazole-2-sulfonamide et d'imidazopyridine-2-sulfonamide répondant à la formule générale I

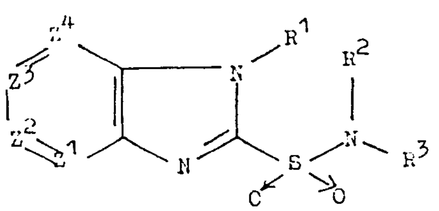

I

et leurs sels pharmaceutiquement acceptables, notamment formule dans laquelle :

$Z^1$ à $Z^4$ représentent un atome d'azote ou un atome de carbone substitué ou non substitué,

$R^1$ et $R^2$ représentent un atome d'hydrogène, un radical alkyle, alkylaryle, alkylcarbonylalkyle, alkylcarbonylaryle, alkylcarbonylhétéroaryle, cycloalkyle, carboxyalkyle, acyle, nitro, ou bien $R^1$ à $R^2$ peuvent former ensemble une liaison représentée par un groupe X;

$R^3$ représente un atome d'hydrogène, un radical alkyle, aryle, hétéroaryle, alkylaryle, hydroxyalkyle, alkylhétéroaryle, mono-, bi- ou tricycloalkyle, alkylamino, N-alkylalkylamino, alkylcarboxyalkyle, acyle, alkylthio (ou sulfinyl, ou sulfonyl)alkyle, alkylthio (ou sulfinyl, ou sulfonyl)aryle, alkylthio (ou sulfinyl, ou sulfonyl)hétéroaryle, alkylthio (ou sulfinyl, ou sulfonyl)alkylaryle, alkylthio (ou sulfinyl, ou sulfonyl)alkylhétéroaryle, haloalkyle, cyanoalkyle, N-alkylarylalkylamino, N-alkyl-N-alkylaryl-alkylamino, N,N-dialkylalkylamino, alkylpipérazinyle, alkylpipéridinyle ou alkylmorpholinyle;

X représente $-(CH\ R^8)_n-(CH\ R^9)_m-$ ou $-CR^8=CR^9-$;

$R^8$ et $R^9$ représentent un atome d'hydrogène, un radical alkyle inférieur, aryle ou hétéroaryle.

$R^{10}$ représente un atome d'hydrogène, un radical $-CH_2OH$, un radical $-CH_2Cl$ ou un radical méthylène $(-CH_2-)_{10}$;

$R^1$ peut former une liaison avec $R^{10}$ dans le cas où $R^{10}$ représente un radical méthylène $(-CH_2-)$ et lui-même ($R^1$) représente une liaison;

$R^{11}$ représente un atome d'hydrogène, un radical alkyle ou un radical hydroxyalkyle.

EP 0 246 126 A1

# BENZIMIDAZOLES ET IMIDAZOPYRIDINES SULFONAMIDES, LEUR PREPARATION ET LEUR APPLICATION EN TANT QUE MEDICAMENTS.

La présente invention se rapporte à de nouveaux dérivés de benzimidazole-2-sulfonamide et d'imidazopyridine-2-sulfonamide, leur procédé de préparation, ainsi que leur application en tant que médicaments.

Les composés objet de la présente invention peuvent également être utilisés dans l'industrie pharmaceutique comme intermédiaires et pour la préparation de médicaments.

Les sulfonamides liées à la position 2 du benzimidazole ou des imidazopyridines ont été très peu étudiées. Autant que l'on sache il n'existe qu'un petit nombre de publications dans la littérature scientifique qui se rapportent à ce genre de composés. Dans deux publications des années soixante [B. Stanovuik et M. Tisler, Arch. Pharm., 1965, **298**, 357; B. Stanovuik et M. Tisler, Arch. Pharm. (Weinheim), 1967, **300**, 322] on décrit les benzimidazole-2-sulfonamides N-substituées par les radicaux suivants : *m*-chlorophényle, phényle, *m*-tolyle, morpholinyle, éthyle, propyle, n-octyle, n-décyle, n-hexadécyle, n-octadécyle et n-méthyléthoxyméthylène. On décrit également dans la seconde référence citée, le produit de cyclisation 1,2,4-thiadiazolo[4,5-a]benzimidazole 1,1-dioxyde. Cependant dans ces travaux, on ne décrit, ni ne suggère l'usage de ces composés pour le traitement thérapeutique humain ou animal.

On a décrit également [S.M. Deshpande et K.C. Datta, J. Indian Chem. Soc., 1976, 53, 320] des bis-benzimidazole-2-sulfonamides liées par des chaînes aliphatiques de 2 à 12 atomes de carbone, comme étant des agents antidiabétiques potentiels. Dans les brevets U.S. 2 603 649 (1952 et DE 27 26 625 (1977) on revendique seulement le benzimidazole-2-sulfonamide et la N,N-diméthylbenzimidazole-2-sulfonamide, respectivement, sans faire mention de l'usage de ces composés pour le traitement thérapeutique des humains ou des animaux. Finalement, dans les brevets britanniques GB 687,760 et GB 833,049, on a décrit également le benzimidazole 2-sulfonamide.

Pour notre part nous avons découvert des séries de dérivés de benzimidazole-2-sulfonamide et d'imidazopyridine-2-sulfonamide qui possèdent des activités pharmaceutiques, en particulier une activité antiulcéreuse et/ou une activité antisécrétoire et qui par conséquent sont utiles comme agents antiulcéreux ou pour le traitement de l'hypersécrétion gastrique. En particulier les composés sont appropriés pour prévenir ou traiter les maladies gastrointestinales des mammifères, l'homme inclus, principalement la sécrétion de l'acide gastrique et la capacité cytoprotectrice. Les composés sont également utiles comme intermédiaires pour la préparation d'autres composés de ces séries.

La présente invention se rapporte à des composés de formule générale I :

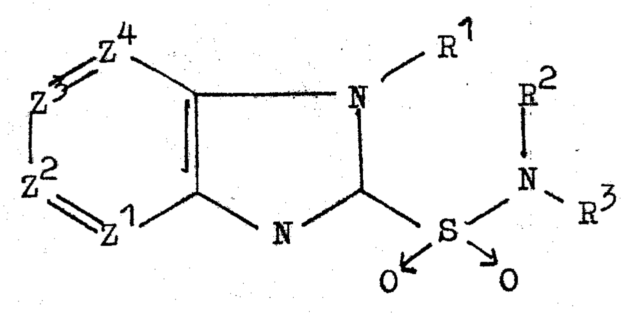

I

et leurs sels pharmaceutiquement acceptables, formule dans laquelle :

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$ peut représenter un atome d'azote;

$R^1$ et $R^2$ représentent indépendamment un atome d'hydrogène, un radical alkyle linéaire ou ramifié, un radical alkylaryle, un radical alkylcarbonylalkyle, un radical alkylcarbonylaryle, un radical alkylcarbonylhétéroaryle, un radical cycloalkyle, un radical carboxyalkyle, un radical acyle, un radical nitro, ou bien $R^1$ et $R^2$ peuvent former ensemble une liaison représentée par un groupe X;

$R^3$ représente un atome d'hydrogène, un radical alkyle linéaire ou ramifié, un radical aryle, un radical hétéroaryle, un radical alkylaryle, un radical hydroxyalkyle, un radical alkylhétéroaryle, un radical mono-, bi- ou tricycloalkyle, un radical alkylamino, un radical N-alkyl-alkylamino, un radical alkylcarboxyalkyle, un radical acyle, un radical alkylthio (ou sulfinyl, ou sulfonyl)alkyle, un radical alkylthio (ou sulfinyl, ou sulfonyl)aryle, un radical alkylthio (ou sulfinyl, ou sulfonyl)hétéroaryle, un radical alkylthio (ou sulfinyl, ou sulfonyl)alkylaryle, un radical alkylthio (ou sulfinyl, ou sulfonyle)alkylhétéroaryle, un radical haloalkyle, un radical cyanoalkyle, un radical N-alkylaryl-alkylamino, un radical N-alkyl-N-alkylaryl-alkylamino, un radical N,N-di-

alkyl-alkylamino, un radical alkylpipérazinyle, un radical alkylpipéridinyle ou un radical alkylmorpholinyle;

X représente $-(CH\ R^8)_n-(CH\ R^9)_m-$ ou $-CR^8=CR^9-$;

$R^2$ et $R^3$ peuvent former ensemble une liaison représentée par un groupe $-(CH_2)_n-Y_m-CH\ R^{10}-$;

Y représente $-CH_2-O-CH_2-$ ou $-CH_2-NR^{11}-CH_2-$;

n représente 1, 2, 3 ou 4;

m représente 0 ou 1;

$R^4$ et $R^7$ représentent indépendamment un atome d'hydrogène, un atome d'halogène, un radical nitro, un radical amino ou acylamino (tel que acétylamino);

$R^5$ et $R^6$ représentent indépendamment un atome d'hydrogène, un atome d'halogène, un radical nitro, un radical alkyle inférieur, un radical trifluorométhyle, un radical alcoxy, un radical aryloxy, un radical mercapto, un radical alkylthio, un radical alkylsulfinyl, un radical alkylsulfonyl, un radical isothiocyanate, un radical sulfamoyle, un radical alkylcarbonyle, un radical arylcarbonyle, un radical acylamino (tel que acétylamino), un radical cyano, un radical carboxylique, un radical carboxamido ou un radical carboxyalkyle;

$R^8$ et $R^9$ représentent indépendamment un atome d'hydrogène, un radical alkyle inférieur, un radical aryle ou un radical hétéroaryle;

$R^{10}$ représente un atome d'hydrogène, un radical $-CH_2OH$, un radical $-CH_2Cl$ ou un radical méthylène ($-CH_2-$);

$R^1$ peut former une liaison avec $R^{10}$ dans le cas où $R^{10}$ représente un radical méthylène ($-CH_2-$) et lui-même ($R^1$) représente une liaison;

$R^{11}$ représente un atome d'hydrogène, un radical alkyle ou un radical hydroxyalkyle;

Aryle représente un groupe phényle ou un groupe phényle substitué;

Hétéroaryle représente un groupe hétérocyclique aromatique dans lequel l'hétéroatome ou atomes du cycle est/sont sélectionnés parmi O et N; le groupe hétérocyclique peut être substitué.

On doit noter que la formule générale I, peut se dessiner, dans le cas où $R^1$ représente un atome d'hydrogène, comme une forme tautomère alternative de formule générale I', comme il est indiqué, et les références qui soient faites concernant la formule générale I, devront inclure, lorsque ce sera nécessaire, la forme tautomère alternative I'.

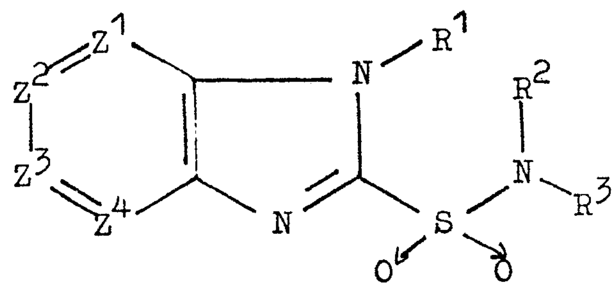

I'

Les composés de formule générale I et leurs sels physiologiquement acceptables sont de préférence administrés sous forme de formulations pharmaceutiques.

Un groupe préféré de composés de formule générale I est celui qui correspond aux composés de formule générale Ia.

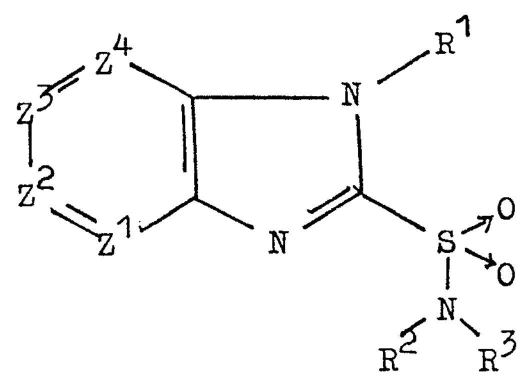

Ia

dans laquelle :

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$, peut représenter un atome d'azote,

$R^1$ représente un atome d'hydrogène, un radical alkyle linéaire ou ramifié, un radical alkylaryle, un radical alkylcarbonylalkyle, un radical alkylcarbonylaryle, un radical alkylcarbonylhétéroaryle, un radical cycloalkyle ou un radical carboxyalkyle;

$R^2$ représente un atome d'hydrogène ou un radical alkyle inférieur linéaire ou ramifié en $C_1$ à $C_5$, un radical nitro ou un radical acyle;

$R^3$ représente un atome d'hydrogène, un radical alkyle linéaire ou ramifié, un radical aryle, un radical hétéroaryle, un radical alkylaryle, un radical alkylhétéroaryle, un radical mono-, bi- ou tricycloalkyle, un radical alkylamino, un radical N-alkyl-alkylamino, un radical alkylcarboxyalkyle, un radical acyle, un radical alkylthio (ou sulfinyl, ou sulfonyl)alkyle, un radical alkylthio (ou sulfinyl, ou sulfonyl)aryle, un radical alkylthio (ou sulfinyl, ou sulfonyl)hétéroaryle, un radical alkylthio (ou sulfinyl, ou sulfonyl)alkylaryle, un radical alkylthio (ou sulfinyl, ou sulfonyl)alkylhétéroaryle, un radical haloalkyle, un radidal cyanoalkyle, un radical N-alkylaryl-alkylamino, un radical N-alkyl-N-alkylaryl-alkylamino, un radical N,N-dialkyl-alkylamino, un radical hydroxyalkyle, un radical alkylpipérazinyle, un radical alkylpipéridinyle ou un radical alkylmorpholinyle;

$R^4$ et $R^7$ représentent indépendamment un atome d'hydrogène, un atome d'halogène, un radical nitro, un radical amino ou acylamino (tel que acétylamino);

$R^5$ et $R^6$ représentent indépendamment un atome d'hydrogène, un atome d'halogène, un radical amino, un radical nitro, un radical alkyle inférieur, un radical trifluorométhyle, un radical alcoxy, un radical aryloxy, un radical mercapto, un radical alkylthio, un radical alkylsulfinyle, un radical alkylsulfonyle, un radical sulfamoyle, un radical isothiocyanate, un radical alkylcarbonyle, un radical arylcarbonyle, un radical acylamino (tel que acétylamino), un radical cyano, un groupe carboxylique, un groupe carboxamido ou un groupe carboxyalkyle.

Un second groupe préféré de composés de formule générale I sont ceux qui correspondent à la formule générale Ib

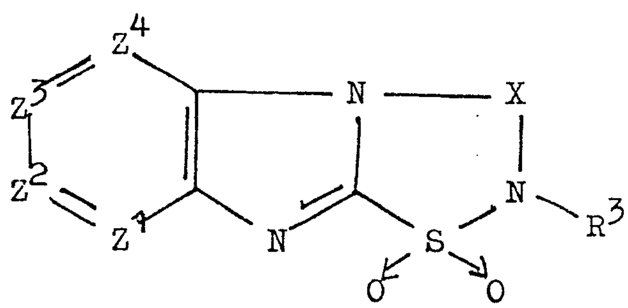

Ib

dans laquelle :

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$, peut représenter un atome d'azote;

$R^3$ représente un atome d'hydrogène, un radical alkyle linéaire ou ramifié, un radical aryle, un radical hétéroaryle, un radical alkylaryle, un radical alkylhétéroaryle, un radical mono-, bi- ou tricycloalkyle, un radical alkylamino, un radical N,N-dialkyl-alkylamino, un radical N-alkyl-alkylamino, un radical alkylcarboxyalkyle, un radical acyle, un radical alkylthio (ou sulfinyl, ou sulfonyl)alkyle, un radical alkylthio (ou sulfinyl, ou sulfonyl)aryle, un radical alkylthio (ou sulfinyl, ou sulfonyl)hétéroaryle, un radical alkylthio (ou sulfinyl, ou sulfonyl)alkylaryle, un radical alkylthio (ou sulfinyl, ou sulfonyl)alkylhétéroaryle, un radical haloalkyle, un radical cyanoalkyle, un radical, N-alkylaryl-alkylamino, un radical N-alkyl-N-alkylaryl-alkylamino, un radical hydroxyalkyle, un radical alkylpipérazinyle, un radical alkylpipéridinyle ou un radical alkylmorpholinyle;

$R^4$ et $R^7$ représentent indépendamment un atome d'hydrogène, un atome d'halogène, un radical nitro, un radical amino ou acylamino (tel que acétylamino);

$R^5$ et $R^6$ représentent indépendamment un atome d'hydrogène, un atome d'halogène, un radical amino, un radical nitro, un radical alkyle inférieur, un radical trifluorométhyle, un radical alcoxy, un radical aryloxy, un radical mercapto, un radical alkylthio,un radical alkylsulfinyle, un radical alkylsulfonyle, un radical sulfamoyle, un radical isothiocyanate, un radical alkylcarbonyle, un radical arylcarbonyle, un radical acylamino (tel que acétylamino), un radical cyano, un groupe carboxylique, un groupe carboxamido ou un groupe carboxyalkyle;

X représente $-(CH\ R^8)_n-(CH\ R^9)_m-$ ou $-CR^8=CR^9-$;

n représente 1, 2, 3 ou 4;

m représente 0 ou 1;

$R^8$ et $R^9$ représentent indépendamment un atome d'hydrogène, un radical alkyle inférieur, un radical aryle ou un radical hétéroaryle.

Un troisième groupe préféré de composés de formule générale I est celui qui correspond aux composés de formule générale Ic

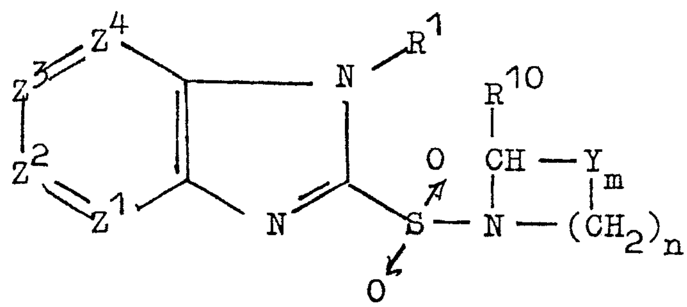

Ic

dans laquelle :

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$, peut représenter un atome d'azote;

$R^1$ représente un atome d'hydrogène, un radical alkyle linéaire ou ramifié, un radical alkylaryle, ou un radical carboxyalkyle;

Y représente $-CH_2-O-CH_2-$ ou $-CH_2-NR^{11}-CH_2-$;

n représente 1, 2, 3 ou 4;

m représente 0 ou 1;

$R^4$ et $R^7$ représentent indépendamment un atome d'hydrogène, un atome d'halogène, un radical nitro, un radical amino ou acylamino (tel que acétylamino);

$R^5$ et $R^6$ représentent indépendamment un atome d'hydrogène, un atome d'halogène, un radical amino, un radical nitro, un radical alkyle inférieur, un radical trifluorométhyle, un radical alcoxy, un radical aryloxy, un radical mercapto, un radical alkylthio, un radical alkylsulfinyle, un radical alkylsulfonyle, un radical sulfamoyle, un radical isothiocyanate, un radical alkylcarbonyle, un radical arylcarbonyle, un radical acylamino (tel que acétylamino), un radical cyano, un groupe carboxylique, un groupe carboxamido ou un groupe carboxyalkyle;

$R^{10}$ représente un atome d'hydrogène, un radical hydroxyalkyle, un radical haloalkyle ou un radical carboxyalkyle;

$R^{11}$ représente un atome d'hydrogène, un radical alkyle inférieur ou un radical hydroxyalkyle.

Un quatrième groupe préféré de composés de formule générale I est celui qui correspond aux composés de formule générale Id

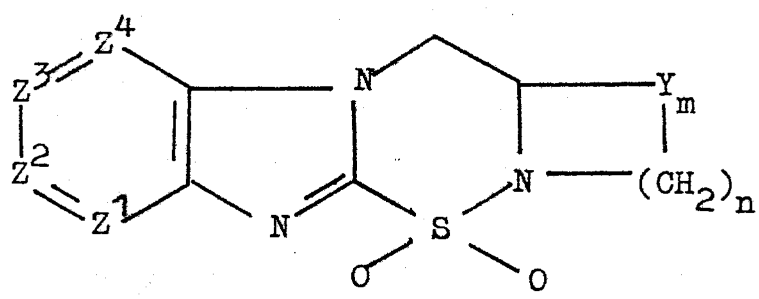

Id

dans laquelle :

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$, peut représenter un atome d'azote;

Y représente $-CH_2-O-CH_2-$ ou $-CH_2-NR^{11}-CH_2-$;

n représente 1, 2, 3 ou 4;

m représente 0 ou 1;

$R^4$ et $R^7$ représentent indépendamment un atome d'hydrogène, un atome d'halogène, un radical nitro, un radical amino ou acylamino (tel que acétylamino);

$R^5$ et $R^6$ représentent indépendamment un atome d'hydrogène, un atome d'halogène, un radical amino, un radical nitro, un radical alkyle inférieur, un radical trifluorométhyle, un radical alcoxy, un radical aryloxy, un radical mercapto, un radical alkylthio, un radical alkylsulfinyle, un radical alkylsulfonyle, un radical sulfamoyle, un radical isothiocyanate, un radical alkylcarbonyle, un radical arylcarbonyle, un radical acylamino (tel que acétylamino), un radical cyano, un groupe carboxylique, un groupe carboxamido ou un groupe carboxyalkyle;

$R^{11}$ représente un atome d'hydrogène, un radical alkyle inférieur ou un radical hydroxyalkyle.

Les classes de composés de formule générale I particulièrement préférés comprennent les composés suivants :

- 5-benzoyl-N-éthyl-1H-benzimidazole-2-sulfonamide,
-N-méthyl-1H-benzimidazole-2-sulfonamide,
-N-(2-pyridyl)-1H-benzimidazole-2-sulfonamide,
-N-[2-(4-morpholinoéthyl)]-1H-benzimidazole-2-sulfonamide,
-N-(4-aminobutyl)-1H-benzimidazole-2-sulfonamide,
-N-[3-(4-morpholinopropyl)]-1H-benzimidazole-2-sulfonamide,
-5-chloro-N-éthyl-1H-benzimidazole-2-sulfonamide,
-5-phénoxy-N-éthyl-1H-benzimidazole-2-sulfonamide,
-N-[2-(3,4-diméthoxyphényléthyl)]-1H-benzimidazole-2-sulfonamide,

- N,N-diéthyl-1H-benzimidazole-2-sulfonamide,
- N-butyl-1H-benzimidazole-2-sulfonamide,
- N-éthyl-4,7-dichloro-5,6-diméthyl-1H-benzimidazole-2-sulfonamide,
- N-(4-chlorophényl)-1H(benzimidazole-2-sulfonamide,
- 5-chloro-N-(4-chlorophényl)-1H-benzimidazole-2-sulfonamide,
- 5-chloro-N-(4-diéthylamino-1-méthylbutyl)-1H-benzimidazole-2-sulfonamide,
- 4-chloro-5,6-diméthyl-N-éthyl-1H-benzimidazole-2-sulfonamide,
- 4-nitro-1H-benzimidazole-2-sulfonamide,
- N-éthyl-4-nitro-1H-benzimidazole-2-sulfonamide,
- N-(4-diéthylamino-1-méthylbutyl)-1H-benzimidazole-2-sulfonamide,
- N-(cyanométhyl)-1H-benzimidazole-2-sulfonamide,
- N-cyclohexyl-1H-benzimidazole-2-sulfonamide,
- N-(1-naphtyl)-1H-benzimidazole-2-sulfonamide,
- N-éthyl-5-trifluorométhyl-1H-benzimidazole-2-sulfonamide,
- N-(1-adamantyl)-1H-benzimidazole-2-sulfonamide,
- 2-(sulfonyl-2-hydroxyméthyl-1-pipéridinyl)-1H-benzimidazole,
- N-(4-méthoxybenzyl)-1H-benzimidazole-2-sulfonamide,
- N-(5-méthyl-3-isoxazolyl)-1H-benzimidazole-2-sulfonamide,
- N-(2-pyridylméthyl)-1H-benzimidazole-2-sulfonamide,
- N-[2-(2-pyridyléthyl)]-1H-benzimidazole-2-sulfonamide,
- 5-benzoyl-N-(4-diéthylamino-1-méthylbutyl)-1H-benzimidazole-2-sulfonamide,
- 5-nitro-1H-benzimidazole-2-sulfonamide,
- N-éthyl-5-nitro-1H-benzimidazole-2-sulfonamide,
- N-[2-(4-morpholinoéthyl)]-5-nitro-1H-benzimidazole-2-sulfonamide,
- N,N-diéthyl-5-nitro-1H-benzimidazole-2-sulfonamide,

- N-butyl-4-nitro-1H-benzimidazole-2-sulfonamide,
- N-(2-hydroxy-1-éthyl)-4-nitro-1H-benzimidazole-2-sulfonamide,
- N-(carboxyéthylméthyl)-4-nitro-1H-benzimidazole-2-sulfonamide,
- N-éthyl-N,4-dinitro-1H-benzimidazole-2-sulfonamide,
- N-[2-(4-morpholinoéthyl)]-4-nitro-1H-benzimidazole-2-sulfonamide,
- N-acétyl-N-éthyl-4-nitro-1H-benzimidazole-2-sulfonamide,
- N-acétyl-4-nitro-1H-benzimidazole-2-sulfonamide,
- 2-[sulfonyl-4-(2-hydroxy-1-éthyl)-pipérazinyl]-4-nitro-1H-benzimidazole,
- N-(4-fluorophényl)-4-nitro-1H-benzimidazole-2-sulfonamide,
- 2-[sulfonyl-2-(carboxyméthyl)-pyrrolidinyl]-4-nitro-1H-benzimidazole,
- N-méthyl-4-nitro-1H-benzimidazole-2-sulfonamide,
- N-propyl-4-nitro-1H-benzimidazole-2-sulfonamide,
- N-isopropyl-4-nitro-1H-benzimidazole-2-sulfonamide,
- N-éthylthioéthyl-4-nitro-1H-benzimidazole-2-sulfonamide,
- N-éthylsulfonyléthyl-4-nitro-1H-benzimidazole-2-sulfonamide,
- N-[3-(2-oxohexaméthyleniminyl)]-4-nitro-1H-benzimidazole-2-sulfonamide,
- N-[1-[1,3-bis(carboxyéthyl)propyl]]-4-nitro-1H-benzimidazole-2-sulfonamide,
- 2-[sulfonyl-4-méthyl-pipérazinyl]-1H-benzimidazole,
- 5-chloro-N-cyclohexyl-1H-benzimidazole-2-sulfonamide,
- N-(2-diéthylamino-1-éthyl)-1H-benzimidazole-2-sulfonamide,
- N-éthyl-1H-imidazo[4,5-b]pyridine-2-sulfonamide,
- N-butyl-1H-imidazo[4,5-b]pyridine-2-sulfonamide,
- N-butyl-1H-imidazo[4,5-c]pyridine-2-sulfonamide,
- 1,N-diéthyl-1H-benzimidazole-2-sulfonamide,

- 1,N,N-triéthyl-1H-benzimidazole-2-sulfonamide,
- 1,N-diméthyl-N-éthyl-1H-benzimidazole-2-sulfonamide,
- 1-benzoylméthyl-N-éthyl-1H-benzimidazole-2-sulfonamide,
- 1-acétylméthyl-N-éthyl-1H-benzimidazole-2-sulfonamide,
- N-éthyl-1-(2-pyridylcarbonylméthyl)-1H-benzimidazole-2-sulfonamide,
- 5-chloro-N-(4-diéthylamino-1-méthylbutyl)-1-éthyl-1H-benzimidazole-2-sulfonamide,
- 6-chloro-N-(4-diéthylamino-1-méthylbutyl)-1-éthyl-1H-benzimidazole-2-sulfonamide,
- 1,N-diéthyl-4-nitro-1H-benzimidazole-2-sulfonamide,
- 1,N,N-triéthyl-6-nitro-1H-benzimidazole-2-sulfonamide,
- 1,N,N-triéthyl-5-nitro-1H-benzimidazole-2-sulfonamide,
- 5-chloro-1,N-diéthyl-N-(4-diéthylamino-1-méthylbutyl)-1H-benzimidazole-2-sulfonamide,
- 6-chloro-1,N-diéthyl-N-(4-diéthylamino-1-méthylbutyl)-1H-benzimidazole-2-sulfonamide,
- 1,N,N-triéthyl-4-nitro-1H-benzimidazole-2-sulfonamide,
- 1,N,N-triéthyl-7-nitro-1H-benzimidazole-2-sulfonamide,
- 1,N-diéthyl-5,N-dinitro-1H-benzimidazole-2-sulfonamide,
- 1,N-diéthyl-6,N-dinitro-1H-benzimidazole-2-sulfonamide,
- 1,N-diéthyl-5-nitro-1H-benzimidazole-2-sulfonamide,
- 1,N-diéthyl-6-nitro-1H-benzimidazole-2-sulfonamide,
- 5-amino-1H-benzimidazole-2-sulfonamide,
- 4-amino-1H-benzimidazole-2-sulfonamide,
- 4-amino-N-(carboxyéthylméthyl)-1H-benzimidazole-2-sulfonamide,
- 4-amino-N-éthyl-1H-benzimidazole-2-sulfonamide,
- 4-acétylamino-N-éthyl-1H-benzimidazole-2-sulfonamide,
- 3,4-dihydro-2H-2-éthyl-[1,2,5]-thiadiazino[5,6-a]-benzimidazole 1,1-dioxyde,
- 3,4-dihydro-2H-[1,2,5]-thiadiazino[5,6-a]benzimidazole 1,1-dioxyde,
- 3,4-dihydro-2H-2-méthyl-[1,2,5]-thiadiazino[5,6-a]-benzimidazole 1,1-dioxyde,

- 3,4,5-trihydro-2H-2-méthyl-[1,2,6]-thiadiazépino-[6,7-a]benzimidazole 1,1-dioxyde,
- 3,4,5-trihydro-2H-2-éthyl-[1,2,6]-thiadiazépino[6,7-a]benzimidazole 1,1-dioxyde,
- 3,4,5,6-tétrahydro-2H-2-éthyl-[1,2,7]-thiadiazocino-[7,8-a]benzimidazole 1,1-dioxyde,
- 3,4-dihydro-2H-[3-(4-morpholinopropyl)]-[1,2,5]-thiadiazino[5,6-a]benzimidazole 1,1-dioxyde
- 3,4,5-trihydro-2H-[2-(4-morpholinoéthyl)]-[1,2,6]-thiadiazépino[6,7-a]benzimidazole 1,1-dioxyde.
- 3,4-dihydro-2H-2-éthyl-8-phénoxy-[1,2,5]-thiadiazino-[5,6-a]benzimidazole 1,1-dioxyde,
- 3,4-dihydro-2H-2-éthyl-7-phénoxy-[1,2,5]-thiadiazino-[5,6-a]benzimidazole 1,1-dioxyde,
- 8-chloro-3,4-dihydro-2H-2-éthyl-7-phénoxy-[1,2,5]-thiadiazino[5,6-a]benzimidazole 1,1-dioxyde,
- 7-chloro-3,4-dihydro-2H-2-éthyl-7-phénoxy-[1,2,5]-thiadiazino[5,6-a]benzimidazole 1,1-dioxyde,
- 3,4,5-trihydro-2H-[1,2,6]-thiadiazépino[6,7-a]benzimidazole 1,1-dioxyde.
- 3,4-dihydro-2H-2-[2-(3,4-diméthoxyphényléthyl)]-[1,2,5]-thiadiazino[5,6-a]benzimidazole 1,1-dioxyde,
- 2H-2-butyl-3,4-dihydro-[1,2,5]-thiadiazino[5,6-a]-benzimidazole 1,1-dioxyde,
- 6,9-dichloro-3,4-dihydro-7,8-diméthyl-2H-2-éthyl-[1,2,5]-thiadiazino[5,6-a]benzimidazole 1,1-dioxyde,
- 8-chloro-3,4-dihydro-2H-2-(4-diéthylamino-1-méthylbutyl)-[1,2,5]-thiadiazino[5,6-a]benzimidazole 1,1-dioxyde,
- 7-chloro-3,4-dihydro-2H-2-(4-diéthylamino-1-méthylbutyl)-[1,2,5]-thiadiazino[5,6-a]benzimidazole 1,1-dioxyde,
- 2H-2-(4-chlorophényl)-3,4-dihydro-[1,2,5]-thiadiazino-[5,6-a]benzimidazole 1,1-dioxyde,
- 3,4-dihydro-2H-2-(5-méthyl-3-isoxazolyl)-[1,2,5]-thiadiazino [5,6-a]benzimidazole 1,1-dioxyde,

- 2H-2-(4-diéthylamino-1-méthylbutyl)-3,4-dihydro-[1,2,5]-thiadiazino[5,6-a]benzimidazole 1,1-dioxyde,
- 3,4-dihydro-2H-2-éthyl-9-nitro-[1,2,5]-thiadiazino-[5,6-a]benzimidazole 1,1-dioxyde,
- 3,4-dihydro-2H-2-éthyl-6-nitro-[1,2,5]-thiadiazino-[5,6-a]benzimidazole 1,1-dioxyde,
- 3-hydro-2H-2-méthyl-6-nitro-[1,2,4]-thiadiazolo-[4,5-a]benzimidazole 1,1-dioxyde,
- 2H-2-cyclohexyl-3,4-dihydro-[1,2,5]-thiadiazino-[5,6-a]benzimidazole 1,1-dioxyde,
- 3,4-dihydro-2H-2-(1-naphtyl)-[1,2,5]-thiadiazino-[5,6-a]benzimidazole 1,1-dioxyde,
- 3,4-dihydro-2H-2-éthyl-7-trifluorométhyl-[1,2,5]-thiadiazino[5,6-a]benzimidazole 1,1-dioxyde,
- 3,4-dihydro-2H-2-éthyl-8-trifluorométhyl-[1,2,5]-thiadiazino[5,6-a]benzimidazole 1,1-dioxyde,
- 2H-2-(1-adamantyl)-3,4-dihydro-[1,2,5]-thiadiazino-[5,6-a]benzimidazole 1,1-dioxyde,
- 7-benzoyl-3,4-dihydro-2H-2-éthyl-[1,2,5]-thiadiazino-[5,6-a]benzimidazole 1,1-dioxyde,
- 8-benzoyl-3,4-dihydro-2H-2-éthyl-[1,2,5]-thiadiazino-[5,6-a]benzimidazole 1,1-dioxyde,
- 3,4-dihydro-2H-2-(4-méthoxybenzyl)-[1,2,5]-thiadiazino[5,6-a]benzimidazole 1,1-dioxyde,
- 3,4-dihydro-2H-2-(2-pyridylméthyl)-[1,2,5]-thiadiazino-[5,6-a]benzimidazole 1,1-dioxyde,
- 3,4-dihydro-2H-2-[2-(2-pyridyléthyl)]-[1,2,5]-thiadiazino[5,6-a]benzimidazole 1,1-dioxyde,
- 8-chloro-3,4-dihydro-2H-2-cyclohexyl-[1,2,5]-thiadiazino[5,6-a]benzimidazole 1,1-dioxyde,
- 7-chloro-3,4-dihydro-2H-2-cyclohexyl-[1,2,5]-thiadiazino[5,6-a]benzimidazole 1,1-dioxyde,
- 2-(2-diéthylamino-1-éthyl)-3,4-dihydro-2H-[1,2,5]-thiadiazino[5,6-a]benzimidazole 1,1-dioxyde,

- 8-chloro-3,4-dihydro-2H-[1,2,5]-thiadiazino[5,6-a]-benzimidazole 1,1-dioxyde,
- 7-chloro-3,4-dihydro-2H-[1,2,5]-thiadiazino[5,6-a]-benzimidazole 1,1-dioxyde,
- 7,8-dichloro-3,4-dihydro-2H-[1,2,5]-thiadiazino[5,6-a] benzimidazole 1,1-dioxyde,
- 2-butyl-7,8-dichloro-3,4-dihydro-2H-9-nitro-[1,2,5]-thiadiazino[5,6-a]benzimidazole 1,1-dioxyde,
- 3,4-dihydro-2H-2-éthyl-7-nitro-[1,2,5]-thiadiazino-[5,6-a]benzimidazole 1,1-dioxyde,
- 3,4-dihydro-2H-2-éthyl-8-nitro-[1,2,5]-thiadiazino-[5,6-a]benzimidazole 1,1-dioxyde,
- 3,4-dihydro-2H-2-méthyl-7-nitro-[1,2,5]-thiadiazino-[5,6-a]benzimidazole 1,1-dioxyde,
- 3,4-dihydro-2H-2-méthyl-8-nitro-[1,2,5]-thiadiazino-[5,6-a]benzimidazole 1,1-dioxyde,
- 3,4,5-trihydro-2H-2-éthyl-8-nitro-[1,2,6]-thiadiazépino[6,7-a]benzimidazole 1,1-dioxyde,
- 3,4,5-trihydro-2H-2-éthyl-9-nitro-[1,2,6]-thiadiazépino[6,7-a]benzimidazole 1,1-dioxyde,
- 2H-2-butyl-3,4-dihydro-7-nitro-[1,2,5]-thiadiazino-[5,6-a]benzimidazole 1,1-dioxyde,
- 2H-2-butyl-3,4-dihydro-8-nitro-[1,2,5]-thiadiazino-[5,6-a]benzimidazole 1,1-dioxyde,
- 3,4-dihydro-2H-8-nitro-2-[2-(2-pyridyléthyl)]-[1,2,5]-thiadiazino[5,6-a]benzimidazole 1,1-dioxyde,
- 3,4-dihydro-2H-7-nitro-2-[2-(2-pyridyléthyl)]-[1,2,5]-thiadiazino[5,6-a]benzimidazole 1,1-dioxyde,
- 3,4-dihydro-2H-8-nitro-(2-pyridylméthyl)-[1,2,5]-thiadiazino[5,6-a]-benzimidazole 1,1-dioxyde,
- 3,4-dihydro-2H-7-nitro-(2-pyridylméthyl)-[1,2,5]-thiadiazino[5,6-a]benzimidazole 1,1-dioxyde,
- 3,4,5,6-tétrahydro-2H-2-éthyl-9-nitro-[1,2,7]-thiadiazozino[7,8-a]benzimidazole 1,1-dioxyde,
- 3,4,5,6-tétrahydro-2H-2-éthyl-10-nitro-[1,2,7]-thiadia-

zozino[7,8-a]benzimidazole 1,1-dioxyde,

- 3,4-dihydro-2H-2-[3-(4-morpholinopropyl)]-7-nitro-[1,2,5]-thiadiazino[5,6-a]benzimidazole 1,1-dioxyde,
- 3,4-dihydro-2H-2-[3-(4-morpholinopropyl)]-8-nitro-[1,2,5]-thiadiazino[5,6-a]benzimidazole 1,1-dioxyde,
- 2H-2-(4-diéthylamino-1-méthylbutyl)-3,4-dihydro-7-nitro-[1,2,5]-thiadiazino[5,6-a]benzimidazole 1,1-dioxyde,
- 2H-2-(4-diéthylamino-1-méthylbutyl)-3,4-dihydro-8-nitro-[1,2,5]-thiadiazino[5,6-a]benzimidazole 1,1-dioxyde,
- 3,4-dihydro-2H-7-nitro-[1,2,5]-thiadiazino[5,6-a]-benzimidazole 1,1-dioxyde,
- 3,4-dihydro-2H-8-nitro-[1,2,5]-thiadiazino[5,6-a]-benzimidazole 1,1-dioxyde,
- 2H-2-(1-adamantyl)-3,4-dihydro-7-nitro-[1,2,5]-thiadiazino[5,6-a]benzimidazole 1,1-dioxyde,
- 2H-2-(1-adamantyl)-3,4-dihydro-8-nitro-[1,2,5]-thiadiazino[5,6-a]benzimidazole 1,1-dioxyde,
- 7-amino-3,4-dihydro-2H-2-éthyl-[1,2,5]-thiadiazino-[5,6-a]benzimidazole 1,1-dioxyde,
- 3,4-dihydro-2H-2-éthyl-7-isothiocyanato-[1,2,5]-thiadiazino[5,6-a]benzimidazole 1,1-dioxyde,
- 7-acétylamino-3,4-dihydro-2H-2-éthyl-[1,2,5]-thiadiazino[5,6-a]benzimidazole 1,1-dioxyde,
- 2-carboxyéthylméthyl-8-chloro-3,4-dihydro-2H-[1,2,5]-thiadiazino[5,6-a]benzimidazole 1,1-dioxyde,
- 2-carboxyéthylméthyl-7-chloro-3,4-dihydro-2H-[1,2,5]-thiadiazino[5,6-a]benzimidazole 1,1-dioxyde,
- 8-chloro-2-(6-chloro-1-hexyl)-3,4-dihydro-2H-[1,2,5]-thiadiazino[5,6-a]benzimidazole 1,1-dioxyde,
- 7-chloro-2-(6-chloro-2-hexyl)-3,4-dihydro-2H-[1,2,5]-thiadiazino[5,6-a]benzimidazole 1,1-dioxyde,
- 8-chloro-2-(2-chloro-1-éthyl)-3,4-dihydro-2H-[1,2,5]-thiadiazino[5,6-a]benzimidazole 1,1-dioxyde,

- 7-chloro-2-(2-chloro-1-éthyl)-3,4-dihydro-2H-[1,2,5]-thiadiazino[5,6-a]benzimidazole 1,1-dioxyde,
- 2-(3-chloro-1-propyl)-3,4-dihydro-2H-7-nitro-[1,2,5]-thiadiazino[5,6-a]benzimidazole 1,1-dioxyde,
- 8-chloro-2-(4-cyano-1-butyl)-3,4-dihydro-2H-[1,2,5]-thiadiazino[5,6-a]benzimidazole 1,1-dioxyde,
- 7-chloro-2-(4-cyano-1-butyl)-3,4-dihydro-2H-[1,2,5]-thiadiazino[5,6-a]benzimidazole 1,1-dioxyde,
- 2-{3-[N-méthyl-N-[2-(3,4-diméthoxyphényl)éthyl]]-propylamino}-3,4-dihydro-2H-7-nitro-[1,2,5]-thiadiazino[5,6-a]benzimidazole 1,1-dioxyde,
- 8-chloro-2-(6-diéthylamino-1-hexyl)-3,4-dihydro-2H-[1,2,5]-thiadiazino[5,6-a]benzimidazole 1,1-dioxyde,
- 7-chloro-2-(6-diéthylamino-1-hexyl)-3,4-dihydro-2H-[1,2,5]-thiadiazino[5,6-a]benzimidazole 1,1-dioxyde,
- 8-chloro-2-cyanométhyl-3,4-dihydro-2H-[1,2,5]-thiadiazino[5,6-a]benzimidazole 1,1-dioxyde,
- 7-chloro-2-cyanométhyl-3,4-dihydro-2H-[1,2,5]-thiadiazino[5,6-a]benzimidazole 1,1-dioxyde,
- 8-chloro-2-(2-diéthylamino-1-éthyl)-3,4-dihydro-2H-[1,2,5]-thiadiazino[5,6-a]benzimidazole 1,1-dioxyde,
- 7-chloro-2-(2-diéthylamino-1-éthyl)-3,4-dihydro-2H-[1,2,5]-thiadiazino[5,6-a]benzimidazole 1,1-dioxyde,
- 7-chloro-2-(6-éthylamino-1-hexyl)-3,4-dihydro-2H-[1,2,5]-thiadiazino[5,6-a]benzimidazole 1,1-dioxyde,
- 7-chloro-2-(2-pyridyl)-3,4-dihydro-2H-[1,2,5]-thiadiazino[5,6-a]benzimidazole 1,1-dioxyde.
- 3,4-dihydro-2H-2-éthyl-3-méthyl-[1,2,5]-thiadiazino-[5,6-a]benzimidazole 1,1-dioxyde,
- 3,4-dihydro-2H-2-éthyl-3-méthyl-7-nitro-[1,2,5]-thiadiazino[5,6-a]benzimidazole 1,1-dioxyde,
- 3,4-dihydro-2H-2-éthyl-3-méthyl-8-nitro-[1,2,5]-thiadiazino[5,6-a]benzimidazole 1,1-dioxyde,
- 2H-2-butyl-3,4-dihydro-pyrido[3',2':4,5]imidazo[1,2-e]-[1,2,5]thiadiazine 1,1-dioxyde,

- 2-butyl-3,4-dihydro-2H-pyrido[3',4':4,5]imidazo[1,2-e][1,2,5]thiadiazine 1,1-dioxyde,
- 1,2,3,4,13,14-hexahydro-pyrido[1',2':2,3][1,2,5]-thiadiazino[5,6-a]benzimidazole 6,6-dioxyde,
- 1,2,3,4,13,14-hexahydro-9-nitro-pyrido[1',2':2,3][1,2,5]-thiadiazino[5,6-a]benzimidazole 6,6-dioxyde,
- 1,2,3,4,13,14-hexahydro-10-nitro-pyrido[1',2':2,3]-[1,2,5]-thiadiazino[5,6-a]benzimidazole 6,6-dioxyde,
- 2H-2-éthyl-[1,2,5]-thiadiazino[5,6-a]benzimidazole 1,1-dioxyde,
- 2H-2-éthyl-3-méthyl-[1,2,5]-thiadiazino[5,6-a]benzimidazole 1,1-dioxyde,
- 2H-2-éthyl-3-phényl-[1,2,5]-thiadiazino[5,6-a]benzimidazole 1,1-dioxyde,
- 2H-2-éthyl-7-nitro-3-(4-nitrophényl)-[1,2,5]-thiadiazino[5,6-a]benzimidazole 1,1-dioxyde.

Les nouveaux dérivés de formule générale I peuvent être préparés conformément à l'invention, selon les méthodes suivantes :

Méthode A

Par réaction d'un composé de formule générale II en suspension dans l'eau ou dans l'acide acétique de 10 à 90 % dans l'eau

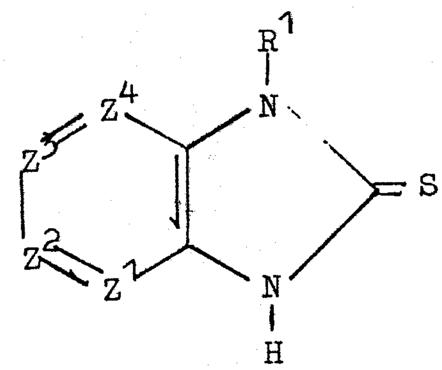

II

dans laquelle

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$, peut représenter un atome d'azote;

$R^1$ représente un atome d'hydrogène, un radical alkyle linéaire ou ramifié, un radical alkylaryle, un radical alkylcarbonylalkyle, un radical alkylcarbonylaryle, un radical alkylcarbonylhétéroaryle ou un radical cycloalkyle;

$R^4$ et $R^7$ représentent indépendamment un atome d'hydrogène, un atome d'halogène, un radical nitro, un radical amino ou acylamino (tel que acétylamino);

$R^5$ et $R^6$ représentent indépendamment un atome d'hydrogène, un atome d'halogène, un radical amino, un radical nitro, un radical alkyle inférieur, un radical trifluorométhyle, un radical alcoxy, un radical aryloxy, un radical mercapto, un radical alkylthio, un radical alkylsulfinyle, un radical alkylsulfonyle, un radical sulfamoyle, un radical isothiocyanate, un radical alkylcarbonyle, un radical arylcarbonyle, un radical acylamino (tel que acétylamino), un radical cyano, un groupe carboxylique, un groupe carboxamido ou un groupe carboxyalkyle;

avec un courant de chlore gazeux pendant un temps compris entre 10 minutes et deux heures on obtient le chlorure d'acide sulfonique correspondant. La réaction s'effectue à des températures comprises entre 0°C et 15°C, en pouvant utiliser dans certains cas comme catalyseur un acide de Lewis, tel que le chlorure ferrique, le chlorure de zinc ou le chlorure d'étain (IV). On filtre le chlorure de l'acide formé et immédiatement on l'additionne à une solution du composé de formule générale III dans un solvant approprié, tel que l'eau, les alcools, l'acétone ou les mélanges de ceux-ci

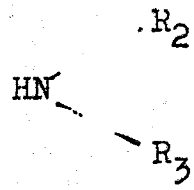

III

dans laquelle

$R^2$ représente un atome d'hydrogène ou un radical alkyle inférieur linéaire ou ramifié en $C_1$ à $C_5$;

$R^3$ représente un atome d'hydrogène, un radical alkyle linéaire ou ramifié, un radical aryle, un radical hétéroaryle, un radical alkylaryle, un radical alkylhétéroaryle, un radical mono-, bi- ou tricycloalkyle, un radical alkylamino, un radical N-alkyl-alkylamino, un radical alkylcarboxyalkyle, un radical acyle, un radical alkylthio (ou sulfinyl, ou sulfonyl)alkyle, un radical alkylthio (ou sulfinyl, ou sulfonyl)aryle, un radical alkylthio (ou sulfinyl, ou sulfonyl)hétéroaryle, un radical alkylthio (ou sulfinyl, ou sulfonyl)alkylaryle, un radical alkylthio (ou sulfinyl, ou sulfonyl)alkylhétéroaryle, un radical haloalkyle, un radical cyanoalkyle, un radical N-alkylaryl-alkylamino, un radical N-alkyl-N-alkylaryl-alkylamino, un radical N,N-dialkyl-alkylamino,un radical hydroxyalkyle, un radical alkylpipérazinyle, un radical alkylpipéridinyle ou un radical alkylmorpholinyle;

$R^2$ et $R^3$ peuvent former ensemble une liaison représentée par un groupe $-(CH_2)_n-Y_m-CH\ R^{10}-$;

Y représente $-CH_2-O-CH_2-$ ou $-CH_2-NR^{11}-CH_2-$;

n représente 1, 2, 3 ou 4;

m représente 0 ou 1;

$R^{10}$ représente un atome d'hydrogène, un radical hydroxyalkyle, un radical haloalkyle ou un radical carboxyalkyle;

$R^{11}$ représente un atome d'hydrogène, un radical alkyle inférieur ou un radical hydroxyalkyle.

La réaction s'effectue à des températures comprises entre -10°C et 40°C pendant 1 à 4 heures. Dans les composés préparés de formules générales Ia et Ic, $Z^1$, $Z^2$, $Z^3$, $Z^4$, $R^1$ à $R^7$, Y, n, m, $R^{10}$ et $R^{11}$ ont les significations mentionnées précédemment.

Méthode B

Par réaction d'un composé de formule générale Ia, dans laquelle $Z^1$, $Z^2$, $Z^3$, $Z^4$, $R^3$ à $R^7$ ont les significations mentionnées précédemment et $R^1$ et $R^2$ représentent un atome d'hydrogène, avec un composé de formule générale IV ou avec un composé de formule générale V

$$A^1-(CHR^8)_m-(CHR^8)_{n-1}-(CHR^9)_m-A^2 \qquad IV$$

$$A^1 - CR^8 = CR^9 - A^2 \qquad V$$

dans lesquelles

$A^1$ et $A^2$ représentent indépendamment un atome de chlore, un atome de brome ou un atome d'iode;

$R^8$ et $R^9$ représentent indépendamment un atome d'hydrogène, un radical alkyle inférieur, un radical aryle ou un radical hétéroaryle;

n représente 1, 2, 3 ou 4;

m représente 0 ou 1.

Dans les composés préparés, de formule générale Ib, $Z^1$, $Z^2$, $Z^3$, $Z^4$, X et $R^3$ à $R^9$ ont les significations mentionnées précédemment.

La réaction s'effectue en présence d'un solvant approprié, par exemple le diméthylsulfoxyde, des alcools tels que le méthanol ou l'éthanol, l'acétone, l'acétonitrile, des éthers tels que le tétrahydrofuranne ou le dioxanne ou des mélanges de ces solvants avec de l'eau. Cette réaction est avantageusement conduite en présence d'une base appropriée, tant des bases minérales

comme le carbonate de potassium, l'hydroxyde de potassium ou un mélange des deux, comme des bases organiques telles que les amines tertiaires ou secondaires. On peut utiliser un catalyseur choisi parmi un sel d'ammonium quaternaire tel que le chlorure de benzyltriéthylammonium, le bromure de tétrabutylammonium, le sulfate acide de tétrabutylammonium ou un sel de phosphonium tel que le chlorure de benzyltriphénylphosphonium ou le bromure de 2-diméthylaminoéthyltriphénylphosphonium. Les températures les plus appropriées oscillent entre environ -5°C et la température de reflux du solvant, le temps de réaction étant compris entre 1 heure et 24 heures.

Méthode C

Par réaction d'un composé de formule générale Ic, dans laquelle $Z^1$, $Z^2$, $Z^3$, $Z^4$, Y, n, m, $R^4$ à $R^7$ et $R^{11}$ ont les significations mentionnées précédemment; $R^1$ représente un atome d'hydrogène et $R^{10}$ représente un groupe hydroxyméthyle ($CH_2OH$), avec un composé deshydratant tel que l'acide polyphosphorique ou le chlorure de thionyle. Les températures les plus adéquates oscillent entre 30°C et 140°C, le temps de réaction étant compris entre 1 heure et 5 heures.

Dans les composés préparés de formule générale Id, $Z^1$, $Z^2$, $Z^3$, $Z^4$, Y, n, m, $R^4$ à $R^7$ et $R^{11}$ ont les significations mentionnées précédemment.

Méthode D

Par réaction d'un composé de formule générale Ia, dans laquelle $Z^1$, $Z^2$, $Z^3$, $Z^4$, Y, n, m, $R^3$ à $R^7$ et $R^{11}$ ont les significations mentionnées précédemment; $R^1$ représente un radical alkylcarbonylalkyle, un radical alkylcarbonylaryle ou un radical alkylcarbonylhétéroaryle et $R^2$ représente un atome d'hydrogène, avec un composé déshydratant tel que l'acide polyphosphorique, le chlorure de thionyle, l'acide p-toluènesulfonique, le tétrachlorure de titanium ou l'oxychlorure de phosphore.

On peut effectuer la réaction dans un solvant tel que toluène, xylène ou en absence de solvant. Les températures les plus adéquates oscillent entre 50°C et 150°C, le temps de réaction étant compris entre 1 heure et 8 heures.

Dans les composés préparés de formule générale Ib, $Z^1$, $Z^2$, $Z^3$, $Z^4$, Y, n, m, $R^3$ à $R^7$ et $R^{11}$ ont les significations mentionnées précédemment, X représente $-CR^8=CR^9-$, $R^8$ représente un atome d'hydrogène et $R^9$ représente un radical alkyle inférieur, un radical aryle ou un radical hétéroaryle.

Méthode E

Par réaction d'un composé de formule générale Ia, dans laquelle $Z^1$ à $Z^4$ et $R^2$ à $R^7$ ont les significations mentionnées précédemment et $R^1$ représente un atome d'hydrogène, avec un composé de formule générale VI

$$A^1 - R^1 \qquad \text{VI}$$

dans laquelle

$A^1$ représente un atome de chlore, un atome de brome ou un atome d'iode;

$R^1$ représente un radical alkyle linéaire ou ramifié en $C_1$ à $C_5$, un radical cycloalkyle, un radical alkylaryle, un radical alkylcarbonylalkyle, un radical carboxyalkyle, un radical alkylcarbonylaryle ou un radical alkylcarbonylhétéroaryle.

La réaction s'effectue en présence d'un solvant adéquat, par exemple le diméthylsulfoxyde, le diméthylformamide, des alcools, de l'acétone ou des mélanges de ces solvants avec de l'eau. Cette réaction est avantageusement conduite en présence d'une base adéquate, tant des bases minérales telles que le carbonate de sodium comme des bases organiques telles que les amines tertiaires. Les températures les plus adéquates oscillent entre 20°C et la température de reflux du solvant, le

temps de réaction étant compris entre 2 heures et 24 heures.

Dans les composés préparés de formule générale Ia, $Z^1$ à $Z^4$ et $R^2$ à $R^7$ ont les significations mentionnées précédemment et $R^1$ a les significations mentionnées excepté hydrogène.

Méthode F

Par réaction d'un composé de formule générale Ia, dans laquelle $Z^1$ à $Z^4$ et $R^3$ à $R^7$ ont les significations mentionnées précédemment, $R^2$ représente un atome d'hydrogène et $R^1$ représente un atome d'hydrogène, un radical alkyle linéaire ou ramifié en $C_1$ à $C_5$, un radical cycloalkyle, un radical alkylaryle, un radical alkylcarbonylalkyle, un radical carboxyalkyle, un radical alkylcarbonylaryle ou un radical alkylcarbonylhétéroaryle, avec un composé de formule générale VII

$$A^1 - R^2 \qquad \text{VII}$$

dans laquelle

$A^1$ représente un atome de chlore, un atome de brome, un atome d'iode, ou un radical acyloxy;

$R^2$ représente un radical alkyle inférieur linéaire ou ramifié en $C_1$ à $C_5$, ou un radical acyle.

La réaction s'effectue en présence d'un solvant adéquat, par exemple le diméthylformamide, des alcools, de l'acétone ou des mélanges de ces solvants avec de l'eau. Cette réaction est avantageusement conduite en présence d'une base adéquate telle que l'hydroxyde de sodium, le carbonate de sodium ou l'hydrure de sodium. Les températures les plus adéquates oscillent entre 10°C et 60°C, le temps de réaction étant compris entre 2 heures et 24 heures.

Dans les composés préparés de formule générale Ia, $Z^1$ à $Z^4$ et $R^3$ à $R^7$ ont les significations mentionnées précédemment et $R^1$ et $R^2$ ont les significations mention-

nées précédemment excepté hydrogène.

Méthode G

Par réaction d'un composé de formule générale Ib, dans laquelle $Z^1$ à $Z^4$ et $R^4$ à $R^9$ ont les significations mentionnées précédemment, X représente $-(CHR^8)_n-(CHR^9)_m-$, n représente 1, 2, 3 ou 4, m représente 0 ou 1 et $R^3$ représente un atome d'hydrogène, avec un composé de formule générale VIII

$$A^1 - R^3 \qquad VIII$$

dans laquelle

$A^1$ représente un atome de fluor, un atome de chlore, un atome de brome ou un atome d'iode;

$R^3$ représente un radical alkyle linéaire ou ramifié, un radical hétéroaryle, un radical alkylaryle, un radical alkylhétéroaryle, un radical mono-, bi- ou tricycloalkyle, un radical alkylamino, un radical N-alkyl-alkylamino, un radical alkylcarboxyalkyle, un radical acyle, un radical alkylthio (ou sulfinyl, ou sulfonyl) alkyle, un radical alkylthio (ou sulfinyl, ou sulfonyl)aryle, un radical alkylthio (ou sulfinyl, ou sulfonyl)hétéroaryle, un radical alkylthio (ou sulfinyl, ou sulfonyl)alkylaryle, un radical alkylthio (ou sulfinyl, ou sulfonyl)alkylhétéroaryle, un radical haloalkyle, un radical cyanoalkyle, un radical, N-alkylaryl-alkylamino, un radical N-alkyl-N-alkylaryl-alkylamino, un radical N,N-dialkyl-alkylamino, un radical hydroxyalkyle, un radical alkylpipérazinyle, un radical alkylpipéridinyle ou un radical alkylmorpholinyle.

La réaction s'effectue en présence d'un solvant adéquat, par exemple le diméthylsulfoxyde, le diméthylformamide, des alcools, de l'acétone ou des mélanges de ces solvants avec de l'eau. Cette réaction est avantageusement conduite en présence d'une base adéquate, tant des

bases minérales comme l'hydrure de sodium, le carbonate de potassium, l'hydroxyde de potassium ou un mélange de ces deux derniers, comme des bases organiques telles que les amines tertiaires. On peut utiliser un catalyseur choisi parmi un sel d'ammonium quaternaire ou un sel de phosphonium. Les températures les plus adéquates oscillent entre environ -5°C et la température de reflux du solvant, le temps de réaction étant compris entre 1 heure et 24 heures.

Dans les composés préparés de formule générale Ib, $Z^1$ à $Z^4$ et $R^4$ à $R^9$ ont les significations mentionnées précédemment et $R^3$ a les significations mentionnées précédemment excepté hydrogène.

Méthode H

Par nitration d'un composé de formule générale I, dans laquelle $Z^1$ représente $C-R^4$, $Z^2$ représente $C-R^5$, $Z^3$ représente $C-R^6$, $Z^4$ représente $C-R^7$, l'un des deux radicaux $R^5$ et $R^6$ représente un atome d'hydrogène et l'autre représente un atome d'hydrogène, un atome d'halogène, un radical nitro, un radical alkyle inférieur, un radical trifluorométhyle, un radical alcoxy, un radical aryloxy ou un radical acylamino et $R^1$ à $R^4$, $R^7$ à $R^{11}$, X, Y, n et m ont les significations mentionnées précédemment, avec un agent de nitration tel qu'un mélange d'acide nitrique et un autre acide fort comme par exemple l'acide sulfurique ou avec le tétrafluoroborate de nitronium.

La réaction s'effectue sans solvant ou avec un solvant adéquat tel que le tétraméthylène sulfone. Les températures les plus adéquates oscillent entre -10°C et 120°C, le temps de réaction étant compris entre 15 minutes et 6 heures.

Dans les composés préparés de formule générale I, $Z^1$ représente $C-R^4$, $Z^2$ représente $C-R^5$, $Z^3$ représente $C-R^6$, $Z^4$ représente $C-R^7$, l'un des deux radicaux $R^5$ et

$R^6$ représente un groupe nitro et l'autre représente un atome d'hydrogène, un atome d'halogène, un radical nitro, un radical alkyle inférieur, un radical trifluorométhyle, un radical alcoxy, un radical aryloxy ou un radical acylamino et $R^1$ à $R^4$, $R^7$ à $R^{11}$, X, Y, n et m ont les significations mentionnées précédemment.

Méthode J

Par réduction d'un composé de formule générale I, dans laquelle $Z^1$ représente C-$R^4$, $Z^2$ représente C-$R^5$, $Z^3$ représente C-$R^6$, $Z^4$ représente C-$R^7$, l'un des quatre radicaux $R^4$, $R^5$, $R^6$ ou $R^7$ représente un groupe nitro et les autres représentent un atome d'hydrogène et $R^1$ à $R^3$, $R^8$ à $R^{11}$, X, Y, n et m ont les significations mentionnées précédemment, avec un agent réducteur approprié.

Parmi les nombreux agents réducteurs susceptibles d'être utilisés pour réduire un groupe nitro à amino, on peut citer les suivants : hydrogénation catalytique en utilisant comme catalyseurs le nickel de Raney ou le palladium sur le carbone, le sulfure de sodium, le sulfure d'ammonium, le sulfate ferreux/hydroxyde d'ammonium, le zinc/acétate d'ammonium, l'amalgame de zinc/acide chlorhydrique, etc. La réaction s'effectue au sein d'un alcool tel que le méthanol ou l'éthanol ou bien de mélanges d'un alcool avec de l'eau. Les températures les plus appropriées oscillent entre 10°C et 60°C, le temps de réaction étant compris entre 2 heures et 24 heures.

Dans les composés préparés de formule générale I, $Z^1$ représente C-$R^4$, $Z^2$ représente C-$R^5$, $Z^3$ représente C-$R^6$, $Z^4$ représente C-$R^7$, l'un des quatre radicaux $R^4$, $R^5$, $R^6$ ou $R^7$ représente un groupe amino et les autres représentent un atome d'hydrogène et $R^1$ à $R^3$, $R^8$ à $R^{11}$, X, Y, n et m ont les significations mentionnées précédemment.

Méthode K

Par acylation d'un composé de formule générale I, dans laquelle $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un groupe amino et les autres représentent un atome d'hydrogène et $R^1$ à $R^3$, $R^8$ à $R^{11}$, X, Y,n et m ont les significations mentionnées précédemment, avec un halogénure d'acide ou un anhydride.

La réaction s'effectue sans solvant ou avec un solvant adéquat tel qu'une amine tertiaire, comme par exemple la pyridine. Les températures les plus adéquates oscillent entre 0°C et 60°C, le temps de réaction étant compris entre 1 heure et 12 heures.

Dans les composés préparés de formule générale I, $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un groupe acylamino et les autres représentent un atome d'hydrogène et $R^1$ à $R^3$, $R^8$ à $R^{11}$, X, Y, n et m ont les significations mentionnées précédemment.

Méthode L

Par réaction d'un composé de formule générale I, dans laquelle $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un groupe amino et les autres représentent un atome d'hydrogène et $R^1$ à $R^3$, $R^8$ à $R^{11}$, X, Y, n et m ont les significations mentionnées précédemment, avec le thiophosgène ($Cl_2CS$).

La réaction s'effectue dans un mélange d'acide chlorhydrique et un solvant préféremment chloré tel que le chloroforme. Les températures les plus adéquates oscillent entre 10°C et la température de reflux du solvant, le temps de réaction étant compris entre 6 heures et 24 heures.

Dans les composés préparés de formule générale I; $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un groupe isothiocyanate (-N=C=S) et les autres représentent un atome d'hydrogène et $R^1$ à $R^3$, $R^8$ à $R^{11}$, X, Y, n et m ont les significations mentionnées précédemment.

Méthode M

Par réaction d'un composé de formule générale I, dans laquelle $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un groupe amino et les autres représentent un atome d'hydrogène et $R^1$ à $R^3$, $R^8$ à $R^{11}$, X, Y, n et m ont les significations mentionnées précédemment, avec le nitrite de sodium dans une solution aqueuse d'acide chlorhydrique ou tétrafluoroborique ou dans de l'acide acétique saturé d'acide chlorhydrique gazeux à une température entre -10°C et 5°C, on obtient le correspondant sel de diazonium que l'on verse sur une solution aqueuse de chlorure cuivreux ou de cyanure cuivreux ou bien sur une solution d'acide acétique saturé d'anhydride sulfureux et une quantité catalytique de chlorure cuivreux et de chlorure cuivrique. Dans ce dernier cas on fait réagir le chlorure de l'acide sulfonique obtenu avec de l'ammoniaque dans les conditions habituelles de formation d'une sulfamide. On maintient les solutions correspondantes entre 5°C et la température ambiante pendant des temps qui oscillent entre 1 heure et 4 heures.

Dans les composés préparés de formule générale I, $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un atome de chlore, un groupe cyano (-C≡N) ou un groupe sulfamoyle ($-SO_2NH_2$) et les autres représentent un atome d'hydrogène, et $R^1$ à $R^3$,

$R^8$ à $R^{11}$, X, Y, n et m ont les significations mentionnées précédemment.

Pour les composés de formule générale I, où $Z^1$ est différent de $Z^4$ ou $Z^2$ est différent de $Z^3$, et $R^1$ est différent de l'hydrogène, on obtient, dans certains cas, deux isomères de position qui peuvent être séparés par cristallisation fractionnée ou par des méthodes chromatographiques.

Les composés de formule générale I, conformément à l'invention, peuvent être synthétisés tant sous forme de base libre comme d'un sel, suivant les conditions de la réaction et la nature des produits de départ. Les sels peuvent se transformer en base libre en utilisant des agents basiques tels que les alcalis et les carbonates alcalins ou par échange ionique. D'autre part, les bases libres synthétisées peuvent á leur tour former des sels avec des acides minéraux ou organiques.

Les composés de formules générales II à VIII sont connus ou peuvent être préparés par des procédés analogues à des procédés connus à partir de composés facilement accessibles. Les composés de formule générale II sont connus ou peuvent être préparés à partir des correspondantes diamines de formule générale IX suivant les méthodes décrites dans la littérature chimique [J.A. Van Allan et B.D. Deacon, Organic Syntheses, Coll. Vol. IV, p. 569, et les références qui y sont citées].

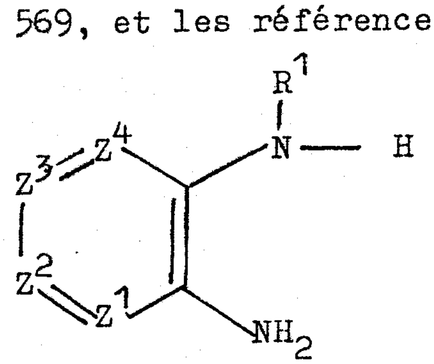

IX

dans laquelle :

$Z^1$ représente un atome d'azote ou un atome de carbone

lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$, peut représenter un atome d'azote.

$R^1$ représente un atome d'hydrogène, un radical alkyle linéaire ou ramifié, un radical alkylaryle, un radical alkylcarbonylalkyle, un radical alkylcarbonylaryle, un radical alkylcarbonylhétéroaryle ou un radical cycloalkyle.

Dans les exemples suivants on indiquera la préparation de nouveaux dérivés selon l'invention. On décrira également quelques formes d'emploi typiques pour les différents domaines d'application.

Les exemples ci-après, donnés à simple titre d'illustration, ne doivent cependant en aucune façon limiter l'étendue de l'invention.

Exemple 1

Méthode A : Préparation de 5-benzoyl-N-éthyl-1H-benzimidazole-2-sulfonamide.

On fait bouillonner du chlore pendant une heure dans une suspension de 50,9 g (0,2 mole) de 2-mercapto-5-benzoyl-1H-benzimidazole dans 1200 ml d'acide acétique à 20 % dans l'eau, de façon que la température n'excède pas les 7°C. On filtre le chlorure d'acide obtenu, on le lave avec de l'eau froide et on l'ajoute immédiatement en petites portions à 200 ml d'éthylamine à 17 % dans l'eau, préalablement refroidie à 5°C. On maintient l'agitation jusqu'à atteindre la température ambiante et on agite alors pendant 1 heure. On porte la solution à pH 5 avec de l'acide chlorhydrique, on filtre

et on lave avec de l'eau. Une fois recristallisé avec de l'acétate d'éthyle on obtient 51,3 g (78 %) de 5-benzoyl-N-éthyl-1H-benzimidazole-2-sulfonamide, de point de fusion 250-3°C.

Les données pour l'identification du produit se présentent dans les tableaux 1 et 2.

Tableau I

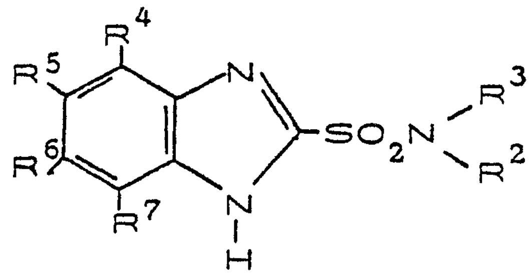

| Exemple nº | $R^2$ | $R^3$ | $R^4$ | $R^5$ | $R^6$ | $R^7$ | Pf (ºC) | IR (KBr) |
|---|---|---|---|---|---|---|---|---|
| 1 | H | $-CH_2CH_3$ | H | $C_6H_5\overset{O}{\overset{\|}{C}}-$ | H | H | 250-3 | 1645;1340;1150 |
| 2 | H | H | H | H | H | H | 211-3 | 1345;1150 |
| 3 | H | $-CH_3$ | H | H | H | H | 196-7 | 1350;1165 |
| 4 | H | $-CH_2CH_3$ | H | H | H | H | 215-8 | 1330;1150 |
| 5 | H | 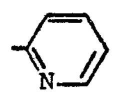 | H | H | H | H | 259-60 | 1285;1175 |
| 6 | H | $-CH_2CH_2$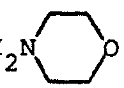 | H | H | H | H | 180-3 | 1340;1160 |
| 7 | H | $-CH_2CH_2CH_2CH_2NH_2$ | H | H | H | H | 198-203 | 1330;1140 |

Tableau I (suite)

| Exemple n° | $R^2$ | $R^3$ | $R^4$ | $R^5$ | $R^6$ | $R^7$ | Pf(°C) | IR (KBr) |
|---|---|---|---|---|---|---|---|---|
| 8 | H | $-CH_2CH_2CH_2$ 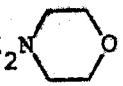 | H | H | H | H | 171-4 | 1355;1155 |
| 9 | H | $-CH_2CH_3$ | H | Cl | H | H | 219-220 | 1335;1150 |
| 10 | H | $-CH_2CH_3$ | H | $C_6H_5O-$ | H | H | 253-8 | 1340;1155 |
| 11 | H | $-CH_2CH_2-$ 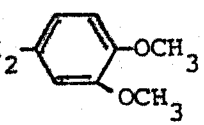 | H | H | H | H | 95-99 | 1345;1155 |
| 12 | $-CH_2CH_3$ | $-CH_2CH_3$ | H | H | H | H | 127-9 | 1370;1150 |
| 13 | $-CH_2CH_2-O-CH_2CH_2-$ | | H | H | H | H | 162-9 | 1380;1160 |
| 14 | H | $-CH_2CH_2CH_2CH_3$ | H | H | H | H | 180-5 | 1350;1160 |
| 15 | H | $-CH_2CH_3$ | Cl | $-CH_3$ | $-CH_3$ | Cl | 310-2 | 1335;1150 |
| 16 | H | 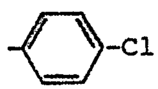 | H | Cl | H | H | 235-7 | 1355;1155 |
| 17 | H | 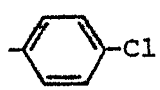 | H | H | H | H | 233-7 | 1360;1160 |
| 18 | H | $-CH(CH_3)(CH_2)_3N(CH_2CH_3)_2$ | H | Cl | H | H | 219-22 | 1320;1130 |

Tableau I (Suite)

| Exemple nº | $R^2$ | $R^3$ | $R^4$ | $R^5$ | $R^6$ | $R^7$ | Pf (ºC) | IR (KBr) |
|---|---|---|---|---|---|---|---|---|
| 19 | H | $-CH_2CH_3$ | Cl | $-CH_3$ | $-CH_3$ | H | 253-5 | 1350;1160 |
| 20 | H | H | $NO_2$ | H | H | H | > 300 | 1520;1370;1338;1160 |
| 21 | H | $-CH_2CH_3$ | $NO_2$ | H | H | H | 204-5 | 1530;1360;1350;1170 |
| 22 | H | $-CH(CH_3)(CH_2)_3N(CH_2CH_3)_2$ | H | H | H | H | 183-4 | 1310;1135 |
| 23 | H | $-CH_2CN$ | H | H | H | H | 195-7 | 2045;1370;1160 |
| 24 | H | Cyclohexyl | H | H | H | H | 238-40 | 1360;1155 |
| 25 | H | 1-Naphtyl | H | H | H | H | 212-8 | 1330;1145 |
| 26 | H | $-CH_2CH_3$ | H | $-CF_3$ | H | H | 221-2 | 1335;1150 |
| 27 | H | 1-Adamantyl | H | H | H | H | 236-46 | 1335;1150 |
| 28 | $-CH_2CH_2CH_2CH_2CH(CH_2OH)-$ | | H | H | H | H | 175-6 | 1335;1150 |
| 29 | H | 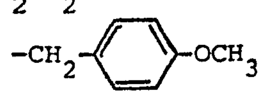 | H | H | H | H | 206-9 | 1335;1145 |
| 30 | H | 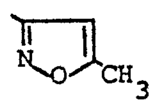 | H | H | H | H | 195-8 | 1355;1160 |

Tableau I (Suite)

| Exemple n° | $R^2$ | $R^3$ | $R^4$ | $R^5$ | $R^6$ | $R^7$ | Pf(°C) | IR (KBr) |
|---|---|---|---|---|---|---|---|---|
| 31 | H | $-CH_2-$ 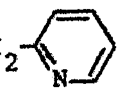 | H | H | H | H | 212-3 | 1360;1160 |
| 32 | H | $-CH_2CH_2-$ 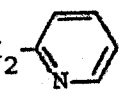 | H | H | H | H | 200-2 | 1345;1160 |
| 33 | H | $-CH(CH_2)_3N(CH_2CH_3)_2$ <br> $CH_3$ | H | $C_6H_5\overset{O}{\overset{\|\|}{C}}-$ | H | H | 236-40 | 1650;1320;1135 |
| 34 | H | H | H | $NO_2$ | H | H | >300 | 1535;1350;1145 |
| 35 | H | $-CH_2CH_3$ | H | $NO_2$ | H | H | 240-5 | 1525;1345;1155 |
| 36 | H | $-CH_2CH_2$ 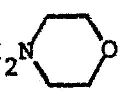 | H | $NO_2$ | H | H | 208-10 | 1515;1365;1320;1150 |
| 37 | $-CH_2CH_3$ | $-CH_2CH_3$ | H | $NO_2$ | H | H | 135-7 | 1520;1350;1145 |
| 38 | H | $-CH_2CH_2CH_2$ 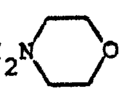 | H | $NO_2$ | H | H | 219-22 | 1500;1370;1330;1145 |

Tableau 1 (suite)

| Exemple nº | $R^2$ | $R^3$ | $R^4$ | $R^5$ | $R^6$ | $R^7$ | Pf (ºC) | IR (KBr) |
|---|---|---|---|---|---|---|---|---|
| 39 | H | $-CH_2CH_2CH_2CH_3$ | $NO_2$ | H | H | H | 181-3 | 1535;1360;1175 |
| 40 | H | $-CH_2CH_2OH$ | $NO_2$ | H | H | H | 173-5 | 1530;1350;1340;1155 |
| 41 | H | $-CH_2CO_2CH_2CH_3$ | $NO_2$ | H | H | H | 167-9 | 1750;1545;1370;1350; 1190 |
| 42 | $NO_2$ | $CH_2CH_3$ | $NO_2$ | H | H | H | 149-50 | 1595;1535;1400;1390; 1350;1185 |
| 43 | H | $-CH_2CH_2$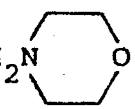 | $NO_2$ | H | H | H | 172-6 | 1535;1330;1160 |
| 44 | $-COCH_3$ | $-CH_2CH_3$ | $NO_2$ | H | H | H | 166-8 | 1710;1525;1380; 1340;1180 |
| 45 | H | $-COCH_3$ | $NO_2$ | H | H | H | >320 | 1730;1520;1370; 1340;1180 |
| 46 | 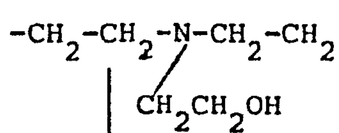 | | $NO_2$ | H | H | H | 175-7 | 1350;1160 |

Tableau 1 (Suite)

| Exemple n° | $R^2$ | $R^3$ | $R^4$ | $R^5$ | $R^6$ | $R^7$ | Pf(°C) | IR (KBr) |
|---|---|---|---|---|---|---|---|---|
| 47 | H | 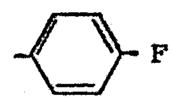 | $NO_2$ | H | H | H | 217-27 | 1535;1370;1345;1180 |
| 48 | $-CH_2-CH_2-CH_2-CH(CO_2CH_3)-$ | | $NO_2$ | H | H | H | 167-8 | 1735;1535;1355;1345; 1150 |
| 49 | H | $-CH_3$ | $NO_2$ | H | H | H | 215-7 | 1535;1335;1170 |
| 50 | H | $-CH_2CH_2CH_3$ | $NO_2$ | H | H | H | 190-2 | 1535;1360;1180 |
| 51 | H | $-CH(CH_3)-CH_3$ | $NO_2$ | H | H | H | 218-20 | 1545;1340;1180 |
| 52 | H | $-CH_2CH_2-S-CH_2-CH_3$ | $NO_2$ | H | H | H | 165-6 | 1535;1355;1175 |
| 53 | H | $-CH_2CH_2-S(\rightarrow O)_2-CH_2-CH_3$ | $NO_2$ | H | H | H | 162-5 | 1540;1360;1175 |

Tableau 1 (suite)

| Exemple nº | $R^2$ | $R^3$ | $R^4$ | $R^5$ | $R^6$ | $R^7$ | Pf (ºC) | IR (KBr) |
|---|---|---|---|---|---|---|---|---|
| 54 | H | 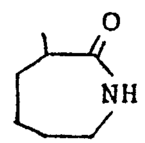 | $NO_2$ | H | H | H | 242-3 | 1650;1535;1340;1175 |
| 55 | H | $-CH(CO_2-CH_2-CH_3)-CH_2-CH_2-CO_2-CH_2-CH_3$ | $NO_2$ | H | H | H | 167-70 | 1740;1720;1535;1375;<br>1340;1185 |
| 56 | $-CH_2-CH_2-N(CH_3)-CH_2CH_2$ | | H | H | H | H | 208-10 | 1350;1150 |
| 57 | H | Cyclohexyl | H | Cl | H | H | 242-4 | 1350;1190 |
| 58 | H | $-CH_2-CH_2-N(CH_2-CH_3)_2$ | H | H | H | H | 180-2 | 1320;1150 |

Tableau 2

| Exemple nº | $^{1}H$ - RMN (DMSO-$d_6$) δ |
|---|---|
| 1 | 1,09(t,3H) ; 3,14(quint.,2H) ; 7,70(m,7H) ; 8,04(s,1H) ; 8,41(t,1H) ; 13,86(é,1H) |
| 2 | 7,27(m,2H) ; 7,60(m,2H) ; 8,03(é,2H) ; 10,92(é,1H) |
| 3 | 2,75(s,3H) ; 7,27(m,2H) ; 7,58(m,2H) ; 9,60(é,2H) |
| 4 | 1,10(t,3H) ; 3,13(quint.,2H) ; 7,33(m,2H) ; 7,66(m,2H) ; 8,24(t,1H) ; 10,52(é,1H) |
| 5 | 6,98(m,3H) ; 7,19(dd,1H) ; 7,51(m,2H) ; 7,81(m,1H) ; 9,34(dd,1H) ; 12,30(é,2H) |
| 6 | 2,57(t,4H) ; 2,72(t,2H) ; 3,51(t,2H) ; 3,72(t,4H) ; 7,60(m,2H) ; 7,93(m,2H) ; 9,60(é,2H) |
| 7 | 1,52(m,4H) ; 2,88(m,4H) ; 6,65(m,4H) ; 7,11(m,2H) ; 7,60(m,2H) |
| 8 | 1,59(quint.,2H) ; 2,24(m,6H) ; 3,12(t,2H) ; 3,48(m,4H) ; 7,32(m,2H) ; 7,67(m,2H) ; 10,55(é,2H) |

Tableau 2 (suite)

| Exemple n° | $^1H$ RMN (DMSO-$d_6$) δ |
|---|---|
| 9 | 1,05(t,3H) ; 3,10(quint.,2H) ; 7,35(dd,1H) ; 7,67(d,1H) ; 7,71(d,1H) ; 8,30(t,1H) ; 13,69(é,1H) |
| 10 | 1,08(t,3H) ; 3,12(quint.,2H) ; 6,90(m,2H) ; 7,28(m,5H) ; 7,61(d,1H) ; 8,38(t,1H) ; 13,90(é,1H) |
| 11 | 2,70(t,2H) ; 3,34(t,2H) ; 3,69(s,6H) ; 6,80(m,3H) ; 7,38(m,2H) ; 7,69(m,2H) ; 8,42(é,1H) ; 13,60(é,1H) |
| 12 | 1,11(t,6H) ; 3,36(q,4H) ; 7,34(m,2H) ; 7,67(m,2H) ; 12,50(é,1H) |
| 13 | 3,25(t,4H) ; 3,71(t,4H) ; 7,38(m,2H) ; 7,72(m,2H) ; 12,75(é,1H) |
| 14 | 0,80(t,3H) ; 1,42(m,4H) ; 3,10(q,2H) ; 7,31(m,2H) ; 7,67(m,2H) ; 8,24(t,1H) ; 13,10(é,1H) |
| 15 | 1,10(t,3H) ; 2,32(s,6H) ; 3,12(quint.,2H) ; 8,30(t,1H) ; 13,20(é,1H) |
| 16 | 7,28(m,5H) ; 7,63(m,2H) ; 12,20(é,2H) ; |

Tableau 2 (suite)

| Exemple n° | $^1H$ RMN (DMSO-$d_6$) δ |
|---|---|
| 17 | 7,10(m,6H) ; 7,43(m,2H) ; 10,90(é,2H) ; |
| 18 | 0,95(d,3H) ; 1,02(t,6H) ; 1,35(m,4H) ; 2,42(q,4H) ; 2,48(t,2H) ; 3,46(Sext.,1H) ; 7,29(dd,1H) ; 7,64(d,1H) ; 7,65(d,1H) ; 9,18(é,2H) |
| 19 | 1,51(t,3H) ; 2,82(s,6H) ; 3,55(quint.,2H) ; 7,75(s,1H) ; 8,80(t,1H) ; 14,10(é,1H) |
| 20 | 7,58(t,1H) ; 8,22(m,4H) ; 10,20(é,1H) ; |
| 21 | 1,10(t,3H) ; 3,14(quint.,2H) ; 7,47(t,1H) ; 8,10(m,2H) ; 8,34(t,1H) ; 12,50(é,1H) |
| 22 | 0,86(t,6H) ; 1,05(d,3H) ; 1,33(m,4H) ; 2,26(q,4H) ; 2,37(t,2H) ; 3,46(Sext.,1H) ; 7,28(m,2H) ; 7,62(m,2H) ; 9,27(é,2H) |
| 23 | 4,30(s,2H) ; 7,24(m,2H) ; 7,59(m,2H) ; 9,10(é,1H) ; 13,10(é,1H) |
| 24 | 1,30(m,10H) ; 3,26(é,1H) ; 7,31(m,2H) ; 7,67(m,2H) ; 8,97(é,1H) ; 13,51(é,1H) |

Tableau 2 (suite)

| Exemple n° | $^1H$ RMN (DMSO-$d_6$) δ |
|---|---|
| 25 | 7,15–8,27(m,11H) ; 13,40(é,2H) |
| 26 | 1,06(t,3H) ; 3,13(quint.,2H) ; 7,62(dd,1H) ; 7,86(d,1H) ; 8,04(d,1H) ; 8,40(t,1H) ; 13,97(é,1H) |
| 27 | 1,49(é,6H) ; 1,82(é,6H) ; 1,89(é,3H) ; 7,29(m,2H) ; 7,66(m,2H) ; 7,98(s,1H) ; 13,20(é,1H) |
| 28 | 1,38(m,6H) ; 3,07(m,1H) ; 3,45(é,1H) ; 3,58(d,2H) ; 3,88(m,2H) ; 7,32(m,2H) ; 7,67(m,2H) ; 12,60(é,1H) |
| 29 | 3,66(s,3H) ; 4,25(d,2H) ; 6,81(d,2H) ; 7,24(d,2H) ; 7,36(m,2H) ; 7,69(m,2H) ; 8,90(t,1H) ; 12,10(é,1H) |
| 30 | 2,27(s,3H) ; 6,10(s,1H) ; 7,18(m,2H) ; 7,55(m,2H) ; 11,20(é,2H) |
| 31 | 4,44(s,2H) ; 7,33(m,4H) ; 7,68(m,3H) ; 8,42(d,1H) ; 9,09(é,1H) ; 13,52(é,1H) |
| 32 | 2,97(t,2H) ; 3,52(t,2H) ; 7,33(m,4H) ; 7,62(m,3H) ; 8,43(m,1H) ; 8,80(é,1H) ; 13,52(é,1H) |

Tableau 2 (suite)

| Exemple n° | $^1H$ RMN (DMSO-$d_6$) δ |
|---|---|
| 33 | 0,91(t,6H) ; 1,09(d,3H) ; 1,41(m,4H) ; 2,42(m,6H) ; 3,53 (sext.,1H) ; 6,90(é,2H) ; 7,66(m,7H) ; 8,01(s,1H) |
| 34 | 7,70(d,1H) ; 8,18(dd,1H) ; 8,20(s,2H) ; 8,43(d,1H) ; 10,57(é,1H) |
| 35 | 1,09(t,3H) ; 3,13(quint.,2H) ; 7,71(d,1H) ; 8,11(dd,1H) ; 8,43(m,3H) |
| 36 | 2,43(t,4H) ; 2,55(t,2H) ; 3,33(t,2H) ; 3,50(t,4H) ; 7,86(d,1H) ; 8,23(dd,1H) ; 8,61(d,1H) ; 10,37(é,2H) |
| 37 | 1,11(t,6H) ; 3,35(q,4H) ; 7,79(d,1H) ; 8,16(dd,1H) ; 8,52(d,1H) ; 12,60(é,1H) |
| 38 | 1,76(quint.,2H) ; 2,50(t,6H) ; 3,27(t,2H) ; 3,64(t,4H) ; 7,89(d,1H) ; 8,27(dd,1H) ; 8,54(d,1H) ; 10,37(é,2H) |

Tableau 2 (suite)

| Exemple nº | $^1H$ RMN (DMSO-$d_6$) $\delta$ |
|---|---|
| 39 | 0,80(t,3H); 1,39(m,4H); 3,09(t,2H); 7,53(t,1H); 8,13(d,1H); 8,20(d,1H); 8,29 (é,2H) |
| 40 | 3,22(t,2H); 3,51(t,2H); 7,46(t,1H); 8,11(d,1H); 8,14(d,1H); 8,44(é,3H) |
| 41 | 1,02(t,3H); 3,94(q,2H); 4,04(s,2H); 7,55(t,1H); 8,16 (d,1H); 8,24(d,1H); 9,02(é,2H) |
| 42 | 1,44(t,3H); 4,40(q,2H); 7,62(t,1H); 8,24(d,1H); 8,32(d,1H); 8,85(é,1H) |
| 43 | 2,33(t,4H); 2,47(t,2H); 3,25(t,2H); 3,39(t,4H); 7,54(t,1H); 8,02(é,2H); 8,14(d,1H); 8,22(d,1H) |
| 44 | 1,30(t,3H); 2,33(s,1H); 2,42(s,3H); 3,94(q,2H); 7,59(t,1H); 8,19(d,1H); 9,27(d,1H) |
| 45 | 2,05(s,3H); 3,59(é, 2H); 7,61(t,1H); 8,15(d,1H); 8,28(d,1H) |

Tableau 2 (suite)

| Exemple n° | $^1H$ RMN (DMSO-$d_6$) δ |
|---|---|
| 46 | 2,82(m,6H) ; 3,50(m,6H) ; 6,60(é, 2H) ; 7,43(t,1H) ; 8,12(d,1H) ; 8,16(d,1H) |
| 47 | 7,21(m,4H) ; 7,50(t,1H) ; 8,09(d,1H) ; 8,19(d,1H) |
| 48 | 1,98(m,4H) ; 3,59(t,2H) ; 3,67(s,3H) ; 4,66(t,1H) ; 7,56(t,1H) ; 7,56(t,1H) ; 8,16(d,1H) ; 8,23(d,1H) ; 14,00(é,1H) |
| 49 | 2,74(d,3H) ; 7,52(t,1H) ; 8,12(d,1H) ; 8,19(d,1H) ; 8,40(é,1H) ; 12,53(é,1H) |
| 50 | 0,81(t,3H) ; 1,47(sext.,2H) ; 3,04(q,2H) ; 7,52(t,1H) ; 8,12(d,1H) ; 8,20(d,1H) ; 8,39(t,1H) ; 13,28(é,1H) |
| 51 | 1,07(d,6H) ; 3,61(quint., 1H) ; 7,50(t,1H) ; 8,10(d,1H) ; 8,17(d,1H) ; 8,28(d,1H) ; 13,01(é,1H) |
| 52 | 1,14(t,3H) ; 2,50 (q,2H) ; 2,70(t,2H) ; 3,30(q,2H) ; 7,59(t,1H) ; 8,19(d,1H) ; 8,27(d,1H) ; 8,61(t,1H) ; 13,32(é,1H) |

Tableau 2 (suite)

| Exemple n° | $^1H$ RMN (DMSO-$d_6$) δ |
|---|---|
| 53 | 1,22(t,3H) ; 3,15(q,2H) ; 3,46(m,4H) ; 7,58(t,1H) ; 8,18(d,1H) ; 8,26(d,1H) ; 8,78(t,1H) ; 10,31(é,1H) |
| 54 | 1,22(m,2H) ; 1,74(m,4H) ; 3,09(m,2H) ; 3,40(é,1H) ; 4,43(m,1H) ; 7,50(t,1H) ; 7,94(é,1H) ; 8,22(d,1H) ; 8,37(d,1H) ; 14,02(é,1H) |
| 55 | 0,96(t,3H) ; 1,12(t,3H) ; 1,99(m,2H) ; 2,45(t,2H) ; 3,89(q,2H) ; 3,95(q,2H) ; 4,24(é,2H) ; 7,57(t,1H) ; 8,14(d,1H) ; 8,24(d,1H) ; 9,07(é,1H) |
| 56 | 2,08(s,3H) ; 2,33(t,4H) ; 3,25(t,4H) ; 7,33(m,2H) ; 7,71(m,2H) ; 12,70(é,1H) |
| 57 | 1,18(m,4H) ; 1,63(m,6H) ; 3,25(m,1H) ; 7,35(d,1H) ; 7,68(d,1H) ; 7,72(s,1H) ; 8,20(é,2H) |
| 58 | 0,81(t,6H) ; 2,39(q,4H) ; 2,45(t,2H) ; 3,10(t,2H) ; 7,23(m,2H) ; 7,58(m,2H) ; 8,22(é,2H) |

Tableau 3

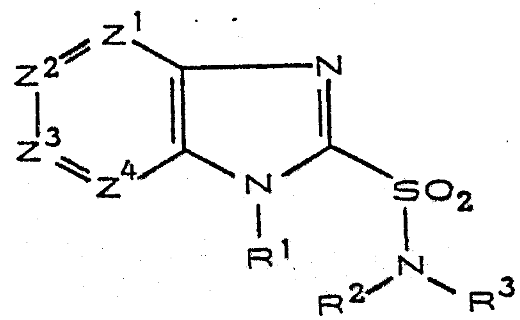

| Exemple nº | $R^1$ | $R^2$ | $R^3$ | $Z^1$ | $Z^2$ | $Z^3$ | $Z^4$ | Pf(ºC) | IR (KBr) |
|---|---|---|---|---|---|---|---|---|---|
| 59 | H | H | $-CH_2CH_3$ | CH | CH | CH | N | 229-30 | 1350;1150 |
| 60 | H | H | $-(CH_2)_3CH_3$ | CH | CH | CH | N | 222 | 1355;1155 |
| 61 | H | H | $-(CH_2)_3CH_3$ | CH | CH | N | CH | 185-95 | 1340;1150 |
| 62 | $-CH_2CH_3$ | H | $-CH_2CH_3$ | CH | CH | CH | CH | 135-9 | 1340;1165 |
| 63 | $-CH_2CH_3$ | $CH_2CH_3$ | $-CH_2CH_3$ | CH | CH | CH | CH | 50-3 | 1330;1150 |
| 64 | $-CH_3$ | $-CH_3$ | $-CH_2CH_3$ | CH | CH | CH | CH | 68-74 | 1335;1160 |
| 65 | $-CH_2\overset{O}{\overset{\|}{C}}C_6H_5$ | H | $-CH_2CH_3$ | CH | CH | CH | CH | 185-95 | 1690;1355;1165 |
| 66 | $-CH_2\overset{O}{\overset{\|}{C}}CH_3$ | H | $-CH_2CH_3$ | CH | CH | CH | CH | 188-90 | 1735;1355;1165 |

Tableau 3 (suite)

| Exemple n° | $R^1$ | $R^2$ | $R^3$ | $Z^1$ | $Z^2$ | $Z^3$ | $Z^4$ | Pf(°C) | IR (KBr) |
|---|---|---|---|---|---|---|---|---|---|
| 67 | $-CH_2\overset{\overset{O}{\|}}{C}C_6H_5N$ | H | $-CH_2CH_3$ | CH | CH | CH | CH | 170-2 | 1730;1350;1160 |
| 68 | $-CH_2CH_3$ | H | $-\overset{\overset{CH_3}{\|}}{C}H(CH_2)_3N(CH_2CH_3)_2$ | CH | CCl | CH | CH | 60-71 | 1340;1160 |
| 69 | $-CH_2CH_3$ | H | $-\overset{\overset{CH_3}{\|}}{C}H(CH_2)_3N(CH_2CH_3)_2$ | CH | CH | CCl | CH | | |
| 70 | $-CH_2CH_3$ | H | $-CH_2CH_3$ | $CNO_2$ | CH | CH | CH | 128-30 | 1530;1360;1330;1165 |
| 71 | $-CH_2CH_3$ | $CH_2CH_3$ | $-CH_2CH_3$ | CH | CH | $CNO_2$ | CH | 90-1 | 1520;1360;1340;1155 |
| 72 | $-CH_2CH_3$ | $CH_2CH_3$ | $-CH_2CH_3$ | CH | $CNO_2$ | CH | CH | 85-100 | 1525;1345;1150 |
| 73 | $-CH_2CH_3$ | $CH_2CH_3$ | $-\overset{\overset{CH_3}{\|}}{C}H(CH_2)_3N(CH_2CH_3)_2$ | CH | CCl | CH | CH | 35-43 | 1340;1150 |
| 74 | $-CH_2CH_3$ | $CH_2CH_3$ | $-\overset{\overset{CH_3}{\|}}{C}H(CH_2)_3N(CH_2CH_3)_2$ | CH | CH | CCl | CH | | |
| 75 | $-CH_2CH_3$ | $CH_2CH_3$ | $-CH_2CH_3$ | $CNO_2$ | CH | CH | CH | 59-60 | 1530;1335;1150 |
| 76 | $-CH_2CH_3$ | $CH_2CH_3$ | $-CH_2CH_3$ | CH | CH | CH | $CNO_2$ | 125-7 | 1530;1340;1155 |
| 77 | $-CH_2CH_3$ | $HNO_2$ | $-CH_2CH_3$ | CH | $CNO_2$ | CH | CH | 108-9 | 1590;1525;1350;1180 |

Tableau 3 (suite)

| Exemple n° | $R^1$ | $R^2$ | $R^3$ | $Z^1$ | $Z^2$ | $Z^3$ | $Z^4$ | Pf(°C) | IR (KBr) |
|---|---|---|---|---|---|---|---|---|---|
| 78 | $-CH_2CH_3$ | $HNO_2$ | $-CH_2CH_3$ | CH | CH | $CNO_2$ | CH | 144-5 | 1590;1525;1350;1185 |
| 79 | $-CH_2CH_3$ | H | $-CH_2CH_3$ | CH | $CNO_2$ | CH | CH | 127-35 | 1520;1350;1165 |
| 80 | $-CH_2CH_3$ | H | $-CH_2CH_3$ | CH | CH | $CNO_2$ | CH | 156-9 | 1525;1350;1170 |
| 81 | H | H | H | CH | $CNH_2$ | CH | CH | 235-7 | 1350;1150 |
| 82 | H | H | H | $CNH_2$ | CH | CH | CH | 192-5 | 1360;1150 |
| 83 | H | H | $-CH_2CO_2CH_2CH_3$ | $CNH_2$ | H | H | H | 160 | 1760;1350;1155 |
| 84 | H | H | $-CH_2CH_3$ | $CNH_2$ | H | H | H | 175-80 | 1360;1155(HCl) |
| 85 | H | H | $-CH_2CH_3$ | $CNHCOH_3$ | H | H | H | 210-2 | 1680;1350;1155 |

Tableau 4

| Exemple n° | $^1H$ RMN (DMSO-$d_6$) $\delta$ |
|---|---|
| 59 | 1,05(t,3H) ; 3,13(quint.,2H) ; 7,36(dd,1H) ; 8,11(dd,1H) ; 8,36(t,1H) ; 8,50(dd,1H) ; 10,21(é,1H) |
| 60 | 0,80(t,3H) ; 1,36(m,4H) ; 3,06(q,2H) ; 7,28(dd,1H) ; 8,01(dd,1H) ; 8,26(t,1H) ; 8,42(dd,1H) ; 12,03(é,1H) |
| 61 | 0,81(t,3H) ; 1,36(m,4H) ; 3,06(t,2H) ; 7,78(é,1H) ; 8,27(é,1H) ; 8,60(é,2H) ; 9,12(é,1H) |
| 62 | 1,14(t,3H) ; 1,40(t,3H) ; 3,23(q,2H) ; 4,58(q,2H) ; 7,37(m,2H) ; 7,76(m,2H) ; 8,61(s,1H) |
| 63 | 1,20(t,6H) ; 1,40(t,3H) ; 3,48(q,4H) ; 4,56(q,2H) ; 7,39(m,2H) ; 7,74(m,2H) |
| 64 | 1,18(t,3H); 3,04(s,3H); 3,43(q,2H); 4,03(s,3H); 7,38(m,2H); 7,70(m,2H) |

Tableau 4 (suite)

| Exemple n° | $^1$H RMN (DMSO-$d_6$) δ |
|---|---|
| 65 | 1,09(t,3H) ; 3,14(q,2H) ; 6,25(s,2H) ; 7,68(m,7H) ; 8,10(m,3H) |
| 66 | 1,09(t,3H) ; 2,26(s,3H) ; 3,08(q,2H) ; 5,53(s,2H) ; 7,60(m,4H) ; 8,49(é,1H) |
| 67 | 1,10(t,3H) ; 3,16(q,2H) ; 6,40(s,2H) ; 7,40(m,2H) ; 7,80(m,3H) ; 8,03(m,2H) ; 8,61(é,1H) ; 8,87(d,1H) |
| 68 | 1,10(d,3H) ; 1,14(t,6H) ; 1,37(t,3H) ; 1,51(m,4H) ; 3,02(m,6H) ; 3,45(m,1H) ; 4,58(q,2H) ; 7,43(dd,1H) ; 7,79(d,1H) ; 7,84(d,1H) ; 8,20(é,1H) |
| 69 | 1,10(d,3H) ; 1,14(t,6H) ; 1,37(t,3H) ; 1,51(m,4H) ; 3,02(m,6H) ; 3,45(m,1H) ; 4,58(q,2H) ; 7,33(dd,1H) ; 7,87(d,1H) ; 7,92(d,1H) ; 8,03(é,1H) |
| 70 | 1,17(t,3H) ; 1,41(t,3H) ; 3,24(q,2H) ; 4,65(q,2H) ; 7,58(t,1H) ; 8,12(d,1H) ; 8,19(d,1H) ; 8,85(s,1H) |
| 71 | 1,22(t,6H) ; 1,44(t,3H) ; 3,50(q,4H) ; 4,69(q,2H) ; 7,92(d,1H) ; 8,14(d,1H) ; 8,74(s,1H) |

Tableau 4 (suite)

| Exemple n° | $^{1}H$ RMN (DMSO-$d_6$) δ |
| --- | --- |
| 72 | 1,22(t,6H) ; 1,42(t,3H) ; 3,49(q,4H) ; 4,62(q,2H) ; 7,98(d,1H) ; 8,28(d,1H) ; 8,61(s,1H) |
| 73 | 1,03(t,6H) ; 1,35(m,4H) ; 1,49(d,3H) ; 1,52(t,6H) ; 2,53(m,6H) ; 3,55(q,2H) ; 4,04(m,1H) ; 4,71(q,2H) ; 7,55(dd,1H) ; 7,89(d,1H) ; 7,94(d,1H) |
| 74 | 1,03(t,6H) ; 1,35(m,4H) ; 1,49(d,3H) ; 1,52(t,6H) ; 2,53(m,6H) ; 3,55(q,2H) ; 4,04(m,1H) ; 4,71(q,2H) ; 7,45(dd,1H) ; 7,98(d,1H) ; 8,03(d,1H) |
| 75 | 1,25(t,6H) ; 1,43(t,3H) ; 3,51(q,4H) ; 4,65(q,2H) ; 7,69(t,1H) ; 8,17(t,1H) ; 8,24(d,1H) |
| 76 | 1,25(t,6H) ; 1,32(t,3H) ; 3,52(q,4H) ; 4,62(q,2H) ; 7,54(t,1H) ; 8,13(dd,1H) ; 8,21(dd,1H) |
| 77 | 1,46(t,6H) ; 4,43(q,2H) ; 4,74(q,2H) ; 8,11(d,1H) ; 8,35(dd,1H) 8,71(d,1H) ; |

Tableau 4 (suite)

| Exemple n° | $^1H$ RMN $\delta$ (DMSO-$d_6$) |
|---|---|
| 78 | 1,47(t,3H); 1,48(t,3H); 4,43(q,2H); 4,81(q,2H); 8,04(d,1H); 8,24(dd,1H); 8,90(d,1H) |
| 79 | 1,13(t,3H); 1,41(t,3H); 3,21(q,2H); 4,66(q,2H); 8,05(d,1H); 8,25(dd,1H); 8,64(d,1H); 8,91(é,1H) |
| 80 | 1,13(t,3H; 1,42(t,3H); 3,24(q,2H); 4,70(q,2H); 7,97(d,1H); 8,19(dd,1H); 8,79(d,1H); 8,90(é,1H) |
| 81 | 5,07(é,2H); 6,60(m,3H); 3,30(m,2H); 7,80(é,1H) |
| 82 | 5,31(é,2H); 6,41(d,1H); 6,85(m,3H); 8,15(é,2H) |
| 83 | 1,08(t,3H); 3,45(é,H); 3,94(s,2H); 3,98(q,2H); 5,46(é,2H); 6,44(d,1H); 6,74(d,1H); 7,03(t,1H) |
| 84 | 1,09(t,3H); 3,13(q,2H); 5,35(é,2H); 6,48(d,1H); 6,86(d,1H); 7,07(t,1H); 8,04(é,1H); 12,90(é,1H) |
| 85 | 1,10(t,3H); 2,16(s,3H); 3,10(quint.,2H); 7,26(t,1H); 7,30(d,1H); 7,89(d,1H); 8,24(t,1H); 9,81(s,1H); 13,39(é,1H) |

Exemples 2 à 61

Les composés identifiés par les exemples 2 à 61 dans les tableaux 1, 2, 3 et 4 sont préparés par la même méthode de préparation décrite dans l'exemple 1.

Exemple 86

Méthode B : Préparation de 3,4-dihydro-2H-2-éthyl-[1,2,5]-thiadiazino[5,6-a]benzimidazole 1,1-dioxyde.

A une solution de 45 g (0,2 mole) de N-éthyl-1H-benzimidazole-2-sulfonamide dans 400 ml de diméthylsulfoxyde on ajoute 4 g de sulfate acide de tétrabutylammonium, 45 g (0,24 mole) de 1,2-dibromoéthane et 138 g (1 mole) de carbonate de potassium finement pulvérisé. On agite pendant 12 heures à température ambiante et on verse sur un mélange eau/glace. On filtre le précipité obtenu, on lave avec de l'eau et on obtient 44,5g (88 %) de 3,4-dihydro-2H-2-éthyl-[1,2,5]-thiadiazino-[5,6-a]benzimidazole 1,1-dioxyde, de point de fusion 176-8°C.

Les données pour l'identification du produit se présentent dans les tableaux 5 et 6.

Tableau 5

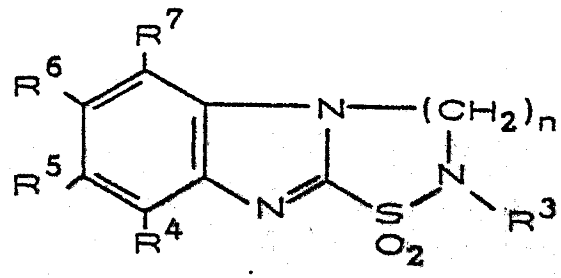

| Exemple nº | $R^3$ | n | $R^4$ | $R^5$ | $R^6$ | $R^7$ | Pf (ºC) | IR (KBr) |
|---|---|---|---|---|---|---|---|---|
| 86 | $-CH_2-CH_3$ | 2 | H | H | H | H | 176-8 | 1345;1160 |
| 87 | H | 2 | H | H | H | H | 227-30 | 1335;1170 |
| 88 | $-CH_3$ | 2 | H | H | H | H | 247-8 | 1345;1160 |
| 89 | $-CH_3$ | 3 | H | H | H | H | 177-9 | 1350;1160 |
| 90 | $-CH_2-CH_3$ | 3 | H | H | H | H | 120-1 | 1360;1160 |
| 91 | $-CH_2-CH_3$ | 4 | H | H | H | H | 155-7 | 1355;1155 |
| 92 | $-(CH_2)_3-$ 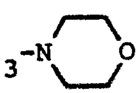 | 2 | H | H | H | H | 188-90 | 1345;1165 |
| 93 | $-(CH_2)_2-$ 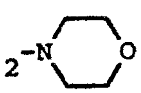 | 3 | H | H | H | H | 132-7 | 1345;1165 |

Tableau 5 (suite)

| Exemple n° | $R^3$ | n | $R^4$ | $R^5$ | $R^6$ | $R^7$ | Pf(°C) | IR (KBr) |
|---|---|---|---|---|---|---|---|---|
| 94 | $-CH_2-CH_3$ | 2 | H | $C_6H_5O-$ | H | H | 199-205 | 1345;1165 |
| 95 | $-CH_2-CH_3$ | 2 | H | H | $C_6H_5O-$ | H | | |
| 96 | $-CH_2-CH_3$ | 2 | H | Cl | H | H | 156-163 | 1360;1165 |
| 97 | $-CH_2-CH_3$ | 2 | H | H | Cl | H | | |
| 98 | H | 3 | H | H | H | H | 251-6 | 1360;1160 |
| 99 | 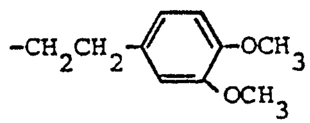 | 3 | H | H | H | H | 175-7 | 1345;1165 |
| 100 | $-CH_2CH_2CH_2CH_3$ | 2 | H | H | H | H | 181-3 | 1345;1170 |
| 101 | $-CH_2CH_3$ | 2 | Cl | $CH_3$ | $CH_3$ | Cl | 206-10 | 1350;1150 |
| 102 | $CH_3$<br>\|<br>$-CH(CH_2)_3N(CH_2CH_3)_2$ | 2 | H | Cl | H | H | 119-21 | 1330;1165 |
| 103 | $CH_3$<br>\|<br>$-CH(CH_2)_3N(CH_2CH_3)_2$ | 2 | H | H | Cl | H | 150-2 | 1320;1180 |

Tableau 5 (suite)

| Exemple nº | $R^3$ | n | $R^4$ | $R^5$ | $R^6$ | $R^7$ | Pf (ºC) | IR (KBr) |
|---|---|---|---|---|---|---|---|---|
| 104 | 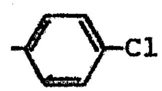 | 2 | H | H | H | H | 215-23 | 1330;1170 |
| 105 | 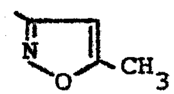 | 2 | H | H | H | H | 203-5 | 1370;1180 |
| 106 | $-CH(CH_3)(CH_2)_3N(CH_2CH_3)_2$ | 2 | H | H | H | H | 110-2 | 1320;1170 |
| 107 | $-CH_2CH_3$ | 2 | $NO_2$ | H | H | H | 218-20 | 1525;1335;1160 |
| 108 | $-CH_2CH_3$ | 2 | H | H | H | $NO_2$ | 184-5 | 1525;1355;1160 |
| 109 | $-CH_2-CH_3$ | 1 | H | H | H | H | 110-6 | 1330;1170 |
| 110 | Cyclohexyle | 2 | H | H | H | H | 222-3 | 1320;1175 |
| 111 | 1-Naphtyle | 2 | H | H | H | H | 274-5 | 1370;1180 |
| 112 | $-CH_2CH_3$ | 2 | H | $CF_3$ | H | H | 160-95 | 1350;1160 |
| 113 | $-CH_2CH_3$ | 2 | H | H | $CF_3$ | H | | |
| 114 | 1-Adamantyle | 2 | H | H | H | H | 300 | 1345;1170 |

Tableau 5 (suite)

| Exemple n° | $R^3$ | n | $R^4$ | $R^5$ | $R^6$ | $R^7$ | Pf(°C) | IR (KBr) |
|---|---|---|---|---|---|---|---|---|
| 115 | $-CH_2CH_3$ | 2 | H | $C_6H_5-\overset{\underset{\|}{O}}{C}-$ | H | H | 180-230 | 1660;1650;1355;1160 |
| 116 | $-CH_2CH_3$ | 2 | H | H | $C_6H_5-\overset{\underset{\|}{O}}{C}-$ | H | | |
| 117 | 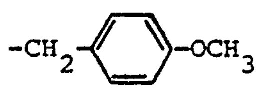 | 2 | H | H | H | H | 227-30 | 1355;1165 |
| 118 | 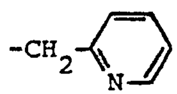 | 2 | H | H | H | H | 168-70 | 1345;1165 |
| 119 | 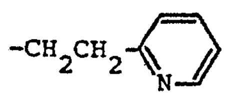 | 2 | H | H | H | H | 182-4 | 1340;1165 |
| 120 | Cyclohexyle | 2 | H | Cl | H | H | 242-4 | 1325;1165 |
| 121 | Cyclohexyle | 2 | H | H | Cl | H | 267-9 | 1315;1170 |
| 122 | $-CH_2CH_2N(CH_2CH_3)_2$ | 2 | H | H | H | H | 163-5 | 1330;1155 |
| 123 | H | 2 | H | Cl | H | H | 255-9 | 1360;1175 |

Tableau 5 (suite)

| Exemple n° | $R^3$ | n | $R^4$ | $R^5$ | $R^6$ | $R^7$ | Pf(°C) | IR (KBr) |
|---|---|---|---|---|---|---|---|---|
| 124 | H | 2 | H | H | Cl | H | 258-62 | 1350;1170 |
| 125 | H | 2 | H | Cl | Cl | H | 273-6 | 1350;1175 |
| 126 | $-CH_2CH_2CH_2CH_3$ | 2 | $NO_2$ | Cl | Cl | H | 215-7 | 1550;1355;1175;1165 |
| 127 | $-CH_2CH_3$ | 2 | H | H | $NO_2$ | H | 255-8 | 1520;1350;1155 |
| 128 | $-CH_2CH_3$ | 2 | H | $NO_2$ | H | H | 208-10 | 1525;1345;1165 |
| 129 | $-CH_3$ | 2 | H | $NO_2$ | H | H | 241-5 | 1520;1350;1340;1165 |
| 130 | $-CH_3$ | 2 | H | H | $NO_2$ | H | 257-65 | 1520;1345;1165 |
| 131 | $-CH_2CH_3$ | 3 | H | H | $NO_2$ | H | 238-45 | 1520;1365;1350;1160 |
| 132 | $-CH_2CH_3$ | 3 | H | $NO_2$ | H | H | 203-8 | 1520;1355;1160 |
| 133 | $-CH_2CH_2CH_2CH_3$ | 2 | H | $NO_2$ | H | H | 165-73 | 1525;1350;1170 |
| 134 | $-CH_2CH_2CH_2CH_3$ | 2 | H | H | $NO_2$ | H | 195-7 | 1525;1350;1170 |

Tableau 5 (Suite)

| Exemple n° | $R^3$ | n | $R^4$ | $R^5$ | $R^6$ | $R^7$ | Pf(°C) | IR (KBr) |
|---|---|---|---|---|---|---|---|---|
| 135 | $-CH_2CH_2-$ 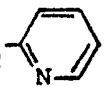 | 2 | H | $NO_2$ | H | H | 175-92 | 1525;1365;1345;1170 |
| 136 | $-CH_2CH_2-$ 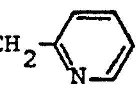 | 2 | H | H | $NO_2$ | H | | |
| 137 | $-CH_2-$ 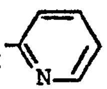 | 2 | H | $NO_2$ | H | H | 175-95 | 1525;1365;1345;1175 |
| 138 | $-CH_2-$ 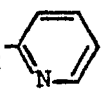 | 2 | H | H | $NO_2$ | H | | |
| 139 | $-CH_2CH_3$ | 4 | H | $NO_2$ | H | H | 207-10 | 1525;1350;1160 |
| 140 | $-CH_2CH_3$ | 4 | H | H | $NO_2$ | H | 240-7 | 1525;1350;1160 |
| 141 | $-(CH_2)_3-$ 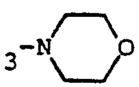 | 2 | H | $NO_2$ | H | H | 170-190 | 1525;1365;1345;1170 |
| 142 | $-(CH_2)_3-$ 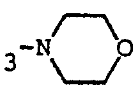 | 2 | H | H | $NO_2$ | H | | |

Tableau 5 (suite)

| Exemple n° | $R^3$ | n | $R^4$ | $R^5$ | $R^6$ | $R^7$ | Pf(°C) | IR (KBr) |
|---|---|---|---|---|---|---|---|---|
| 143 | $-\overset{CH_3}{\overset{\vert}{C}}H(CH_2)_3N(CH_2CH_3)_2$ | 2 | H | $NO_2$ | H | H | 130-7 | 1530;1350;1165 |
| 144 | $-\overset{CH_3}{\overset{\vert}{C}}H(CH_2)_3N(CH_2CH_3)_2$ | 2 | H | H | $NO_2$ | H | 145-7 | 1530;1350;1325;1170 |
| 145 | H | 2 | H | $NO_2$ | H | H | 240-5 | 1525;1345;1170 |
| 146 | H | 2 | H | H | $NO_2$ | H | 251(d) | 1525;1345;1170 |
| 147 | 1-Adamantyle | 2 | H | $NO_2$ | H | H | 217-23 | 1530;1350;1170 |
| 148 | 1-Adamantyle | 2 | H | H | $NO_2$ | H | | |
| 149 | $-CH_2CH_3$ | 2 | H | H | $NH_2$ | H | 232-4 | 3430;3330;1355;1155 |
| 150 | $-CH_2CH_3$ | 2 | H | H | $CH_3\overset{O}{\overset{\|}{C}}NH-$ | H | 245-8 | 1690;1350;1155 |
| 151 | $-CH_2CH_3$ | 2 | H | H | -NCS | H | 218-25 | 2100;1350;1160 |

Tableau 5 (suite)

| Exemple nº | $R^3$ | n | $R^4$ | $R^5$ | $R^6$ | $R^7$ | Pf (ºC) | IR (KBr) |
|---|---|---|---|---|---|---|---|---|
| 152 | $-CH_2CO_2CH_2CH_3$ | 2 | H | Cl | H | H | 192-4 | 1755;1340;1170 |
| 153 | $-CH_2CO_2CH_2CH_3$ | 2 | H | H | Cl | H | 163-5 | 1745;1350;1170 |
| 154 | $-(CH_2)_6-Cl$ | 2 | H | Cl | H | H | 154-7 | 1345;1170 |
| 155 | $-(CH_2)_6-Cl$ | 2 | H | H | Cl | H | 167-8 | 1350;1170 |
| 156 | $-CH_2CH_2Cl$ | 2 | H | Cl | H | H | 216 | 1350;1170 |
| 157 | $-CH_2CH_2Cl$ | 2 | H | H | Cl | H | 218-20 | 1345;1165 |
| 158 | $-CH_2CH_2CH_2Cl$ | 2 | H | H | $NO_2$ | H | 210-4 | 1345;1170 |
| 159 | $-(CH_2)_4-CN$ | 2 | H | Cl | H | H | 179-80 | 2240;1340;1160 |
| 160 | $-(CH_2)_4-CN$ | 2 | H | H | Cl | H | 192-6 | 2240;1355;1165 |
| 161 | 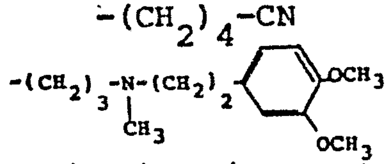 | 2 | H | H | $NO_2$ | H | 104-9 | 1520;1350;1335;1165 |
| 162 | $-(CH_2)_6-N(CH_2CH_3)_2$ | 2 | H | Cl | H | H | 125-32 (HCl) | 1370;1180 (HCl) |

Tableau 5 (suite)

| Exemple n° | $R^3$ | n | $R^4$ | $R^5$ | $R^6$ | $R^7$ | Pf(°C) | IR (KBr) |
|---|---|---|---|---|---|---|---|---|
| 163 | $-(CH_2)_6-N(CH_2CH_3)_2$ | 2 | H | H | Cl | H | 128-38 (HCl) | 1355;1175(HCl) |
| 164 | $-CH_2-CN$ | 2 | H | Cl | H | H | 185-7 | 2225;1370;1180 |
| 165 | $-CH_2-CN$ | 2 | H | H | Cl | H | 243-5 | 2225;1370;1180 |
| 166 | $-CH_2CH_2N(CH_2CH_3)_2$ | 2 | H | Cl | H | H | 212-3 | 1335;1160 |
| 167 | $-CH_2CH_2N(CH_2CH_3)_2$ | 2 | H | H | Cl | H | 193-6 | 1340;1165 |
| 168 | $-(CH_2)_6-NHCH_2CH_3$ | 2 | H | H | Cl | H | 241-3 (HCl) | 1335;1165(HCl) |
| 169 | 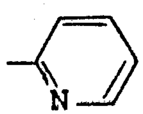 | 2 | H | H | Cl | H | 205-9 | 1370;1170 |

Tableau 6

| Exemple n° | $^{1}H$ RMN (DMSO-$d_6$) δ |
|---|---|
| 86 | 1,30(t,3H); 3,36(q,2H); 4,20(t,2H); 4,45(t,2H); 7,34-7,90(m,4H) |
| 87 | 3,95(t,2H); 4,34(t,2H); 7,36-7,85(m,4H); 8,28(ê,1H) |
| 88 | 2,99(s,3H); 4,17(t,2H); 4,48(t,2H); 7,35-7,88(m,4H) |
| 89 | 2,03(quint.,2H); 2,72(s,3H); 3,79(t,2H); 4,63(t,2H); 7,43(m,2H); 7,80(m,2H) |
| 90 | 1,06(t,3H); 1,93(quint.,2H); 2,96(q,2H); 3,73(t,2H); 4,59(t,2H); 7,38(m,2H); 7,75(m,2H) |
| 91 | 1,14(t,3H); 1,30(m,2H); 1,99(quint.,2H); 3,09(q,2H); 3,59(t,2H); 4,85(t,2H); 7,40(m,2H); 7,77(m,2H) |
| 92 | 1,95(quint.,2H); 2,52(m,6H); 3,49(t,2H); 3,71(m,4H); 4,33(t,2H); 4,57(t,2H); 7,48-8,05(m,4H) |
| 93 | 2,05(quint.,2H); 2,25(t,4H); 2,48(t,2H); 3,22(t,2H); 3,34(t,4H); 3,84(t,2H); 4,65(t,2H); 7,43(m,2H); 7,80(m,2H) |

Tableau 6 (suite)

| Exemple n° | $^{1}H$ RMN (DMSO-$d_6$) δ |
|---|---|
| 94 | 1,25(t,3H); 3,35(q,2H); 4,24(t,2H); 4,51(t,2H); 6,91(m,2H); 7,35(m,5H); 7,70(d,1H) |
| 95 | 1,27(t,3H); 3,38(q,2H); 4,24(t,2H); 4,51(t,2H); 6,89(m,2H); 7,36(m,5H); 7,74(d,1H) |
| 96 | 1,24(t,3H); 3,36(q,2H); 4,19(t,2H); 4,45(t,2H); 7,50(dd,1H); 7,72(d,1H); 7,90(d,1H) |
| 97 | 1,24(t,3H); 3,36(q,2H); 4,18(t,2H); 4,43(t,2H); 7,40(dd,1H); 7,81(d,1H); 7,82(d,1H) |
| 98 | 1,96(quint.,2H); 2,94(t,1H); 3,75(m,2H); 4,60(m,2H); 7,38(m,2H); 7,78(m,2H) |
| 99 | 2,90(t,2H); 3,53(t,2H); 3,72(s,6H); 4,16(t,2H); 4,43(t,2H); 6,85(m,3H); 7,35-7,88(m,4H) |
| 100 | 0,93(t,3H); 1,52(m,4H); 3,32(t,2H); 4,19(t,2H); 4,46(t,2H); 7,38-7,86(m,4H) |
| 101 | 1,28(t,3H); 2,40(s,6H); 3,35(q,2H); 4,14(t,2H); 4,77(t,2H) |
| 102 | 0,91(t,6H); 1,23(d,3H); 1,43(m,4H); 2,40(m,6H); 3,69(m,1H); 4,14(t,2H); 4,38(t,2H); 7,50(dd,1H); 7,74(d,1H); 7,90(d,1H) |

Tableau 6 (suite)

| Exemple n° | $^1H$ RMN (DMSO-$d_6$) $\delta$ |
|---|---|
| 103 | 0,86(t,6H) ; 1,19(d,3H) ; 1,39(m,4H) ; 2,34(m,6H) ; 3,42(m,1H) ; 4,10(t,2H) ; 4,34(t,2H) ;7,39(dd,1H) ; 7,81(d,1H) ; 7,83(d,1H) |
| 104 | 4,57(m,4H) ; 7,30-7,98(m,8H) |
| 105 | 2,38(s,3H) ; 4,69(m,4H) ; 6,42(s,1H) ; 7,55(m,4H) |
| 106 | 0,89(t,6H) ; 1,23(d,3H) ; 1,47(m,4H) ; 2,37(m,6H) ; 3,40(m,1H) ; 4,14(t,2H) ; 4,33(t,2H) ;7,39-7,87(m,4H) |
| 107 | 1,28(t,3H) ; 3,41(q,2H) ; 4,25(t,2H) ; 4,56(t,2H) ; 7,66(t,1H) ; 8,15(d,1H) ; 8,23(d,1H) |
| 108 | 1,28(t,3H) ; 3,43(q,2H) ; 4,15(t,2H) ; 4,57(t,2H) ; 7,56(t,1H) ; 8,16(d,1H) ; 8,24(d,1H) |
| 109 | 1,20(t,3H) ; 3,32(q,2H) ; 6,18(s,2H) ; 7,40-7,87(m,4H) |
| 110 | 1,00-1,76(m,10H) ; 3,86(m,1H) ; 4,17(t,2H) ; 4,36(t,2H) ; 7,30-7,85(m,4H) |

Tableau 6 (suite)

| Exemple n° | $^1H$ RMN (DMSO-$d_6$) δ |
|---|---|
| 111 | 4,63(m,4H) ; 7,40-8,35(m,11H) |
| 112 | 1,27(t,3H) ; 3,41(q,2H) ; 4,23(t,2H) ; 4,52(t,2H) ; 7,80(d,1H) ; 7,99(d,1H) ; 8,14(s,1H) |
| 113 | 1,27(t,3H) ; 3,41(q,2H) ; 4,23(t,2H) ; 4,52(t,2H) ; 7,66(d,1H) ; 7,80(d,1H) ; 8,15(s,1H) |
| 114 | 1,54(m,6H) ; 1,65(m,6H) ; 2,14(m,3H) ; 4,22(t,2H) ; 4,34(t,2H) ; 7,36(m,3H) ; 7,89(m,1H) [$CDCl_3$] |
| 115 | 1,26(t,3H) ; 3,39(q,2H) ; 4,21(t,2H) ; 4,53(t,2H) ; 7,51-7,87(m,7H) ; 8,03(d,1H) |
| 116 | 1,26(t,3H) ; 3,39(q,2H) ; 4,21(t,2H) ; 4,53(t,2H) ; 7,50-7,92(m,7H) ; 8,09(d,1H) |
| 117 | 3,77(s,3H) ; 4,05(t,2H) ; 4,40(t,2H) ; 4,48(s,2H) ; 6,93(d,2H) ; 7,36(d,2H) ; 7,47(m,4H) |

Tableau 6 (suite)

| Exemple n° | $^1H$ RMN δ (DMSO-$d_6$) |
|---|---|
| 118 | 4,23(t,2H) ; 4,47(t,2H) ; 4,68(s,2H) ; 7,20-7,95(m,7H) ; 8,48(ddd,1H) |
| 119 | 3,17(t,2H) ; 3,78(t,2H) ; 4,22(t,2H) ; 4,47(t,2H) ; 7,13-7,86(m,7H) ; 8,49(ddd,1H) |
| 120 | 1,25(m,2H) ; 1,68(m,8H) ; 3,84(m,1H) ; 4,16(t,2H) ; 4,36(t,2H) ; 7,46(dd,1H) ; 7,67(d,1H) ; 7,84(d,1H) |
| 121 | 1,24(m,2H) ; 1,67(m,8H) ; 3,84(m,1H) ; 4,16(t,2H) ; 4,36(t,2H) ; 7,39(dd,1H) ; 7,77(d,1H) ; 7,80(d,1H) |
| 122 | 0,95(t,6H) ; 2,50(q,4H) ; 2,69(t,2H) ; 3,40(t,2H) ; 4,27(t,2H) ; 4,48(t,2H) ; 7,30-7,87(m,4H) |
| 123 | 3,96(t,2H) ; 4,35(t,2H) ; 7,47(dd,1H) ; 7,71(d,1H) ; 7,88(d,1H) ; 8,35(é,1H) |
| 124 | 3,94(t,2H) ; 4,33(t,2H) ; 7,40(dd,1H) ; 7,82(d,1H) ; 7,83(d,1H) ; 8,26(é,1H) |
| 125 | 3,93(t,2H) ; 4,33(t,2H) ; 8,12(s,1H) ; 8,14(s,1H) ; 8,42(é,1H) |

Tableau 6 (suite)

| Exemple N° | $^{1}H$ RMN $\delta$ (DMSO-$d_6$) |
|---|---|
| 126 | 0,90(t,3H) ; 1,30(m,2H) ; 1,58(m,2H) ; 3,32(t,2H) ; 4,20(t,2H) ; 4,49(t,2H) ; 8,51(s,1H) |
| 127 | 1,25(t,3H) ; 3,38(q,2H) ; 4,23(t,2H) ; 4,58(t,2H) ; 8,02(d,1H) ; 8,25(dd,1H) ; 8,74(d,1H) |
| 128 | 1,27(t,3H) ; 3,40(q,2H) ; 4,23(t,2H) ; 4,54(t,2H) ; 7,88(d,1H) ; 8,31(dd,1H) ; 8,67(d,1H) |
| 129 | 3,03(s,3H) ; 4,21(t,2H) ; 4,58(t,2H) ; 7,88(d,1H) ; 8,31(dd,1H) ; 8,66(d,1H) |
| 130 | 3,02(s,3H) ; 4,22(t,2H) ; 4,64(t,2H) ; 8,00(dd,1H) ; 8,24(dd,1H) ; 8,68(dd,1H) |
| 131 | 1,10(t,3H) ; 2,00(quint.,2H) ; 3,04(q,2H) ; 3,79(t,2H) ; 4,77(t,2H) ; 8,00(d,1H) ; 8,21(dd,1H) ; 8,88(d,1H) |
| 132 | 1,11(t,3H) ; 2,04(quint.,2H) ; 3,10(q,2H) ; 3,79(t,2H) ; 4,71(t,2H) ; 7,94(d,1H) ; 8,27(dd,1H) ; 8,63(d,1H) |

Tableau 6 (suite)

| Exemple n° | $^1H$ RMN (DMSO-$d_6$) δ |
|---|---|
| 133 | 0,93(t,3H) ; 1,51(m;4H) ; 3,36(t,2H) ; 4,23(t,2H) ; 4,55(t,2H) ; 7,87(d,1H) ; 8,29(dd,1H) ; 8,64(d,1H) |
| 134 | 0,93(t,3H) ; 1,51(m,4H) ; 3,36(t,2H) ; 4,24(t,2H) ; 4,61(t,2H) ; 8,07(d,1H) ; 8,20(dd,1H) ; 8,65(d,1H) |
| 135 | 3,18(t,2H) ; 3,82(t,2H) ; 4,27(t,2H) ; 4,57(t,2H) ; 7,24(m,2H) ; 7,67(m,1H) ; 7,85(d,1H) ; 8,29(dd,1H) ; 8,46(d,1H) ; 8,63(d,1H) |
| 136 | 3,18(t,2H) ; 3,82(t,2H) ; 4,27(t,2H) ; 4,63(t,2H) ; 7,24(m,2H) ; 7,69(m,1H) ; 8,00(d,1H) ; 8,21(dd,1H) ; 8,46(d,1H) ; 8,65(d,1H) |
| 137 | 4,28(t,2H) ; 4,53(t,2H) ; 4,73(s,2H) ; 7,28(m,1H) ; 7,49(m,1H) ; 7,78(m,1H) ; 7,86(d,1H) ; 8,31(dd,1H) ; 8,38(m,1H) ; 8,65(d,1H) |
| 138 | 4,28(t,2H) ; 4,54(t,2H) ; 4,73(s,2H) ; 7,28(m,1H) ; 7,49(m,1H) ; 7,78(m,1H) ; 7,96(d,1H) ; 8,23(dd,1H) ; 8,38(m,1H) ; 8,70(d,1H) |
| 139 | 1,15(t,3H) ; 1,40(m,2H) ; 2,06(quint.,2H) ; 3,13(q,2H) ; 3,65(t,2H) ; 4,91(t,2H) ; 8,01(d,1H) ; 8,31(dd,1H) ; 8,72(d,1H) |

Tableau 6 (suite)

| Exemple n° | $^1H$ RMN (DMSO-$d_6$) δ |
|---|---|
| 140 | 1,15(t,3H) ; 1,40(m,2H) ; 2,04(quint.,2H) ; 3,14(q,2H) ; 3,62(t,2H) ; 4,97(t,2H) ; 8,01(d,1H) ; 8,22(dd,1H) ; 8,83(d,1H) |
| 141 | 2,02(quint.,2H) ; 3,04(m,6H) ; 3,43(t,2H) ; 3,78(m,4H) ; 4,25(t,2H) ; 4,56(t,2H) ; 7,92(d,1H) ; 8,34(dd,1H) ; 8,69(d,1H) |
| 142 | 2,02(quint.,2H) ; 3,04(m,6H) ; 3,43(t,2H) ; 3,78(m,4H) ; 4,25(t,2H) ; 4,62(t,2H) ; 8,01(d,1H) ; 8,25(dd,1H) ; 8,71(d,1H) |
| 143 | 0,90(t,6H) ; 1,23(d,3H) ; 1,48(m,4H) ; 2,40(q,6H) ; 3,43(m,1H) ; 4,16(m,2H) ; 4,46(t,2H) ; 7,89(d,1H) ; 8,32(dd,1H) ; 8,69(d,1H) |
| 144 | 0,90(t,6H) ; 1,24(d,3H) ; 1,45(m,4H) ; 2,39(q,6H) ; 3,43(m,1H) ; 4,17(t,2H) ; 4,53(t,2H) ; 8,02(d,1H) ; 8,27(dd,1H) ; 8,73(d,1H) |
| 145 | 4,00(t,2H) ; 4,44(t,2H) ; 7,88(d,1H) ; 8,30(dd,1H) ; 8,47(è,1H) ; 8,67(d,1H) |
| 146 | 4,01(t,2H) ; 4,51(t,2H) ; 8,01(d,1H) ; 8,26(dd,1H) ; 8,48(è,1H) ; 8,71(d,1H) |

Tableau 6 (suite)

| Exemple n° | $^1H$ RMN (DMSO-$d_6$) δ |
|---|---|
| 147 | 1,57(é,12H) ; 2,03(é,3H) ; 3,95(t,2H) ; 4,43(t,2H) ; 7,90(d,1H) ; 8,33(dd,1H) ; 8,73(d,1H) |
| 148 | 1,57(é,12H) ; 2,03(é,3H) ; 4,01(t,2H) ; 4,49(t,2H) ; 8,00(d,1H) ; 8,24(dd,1H) ; 8,69(d,1H) |
| 149 | 1,22(t,3H) ; 3,29(q,2H) ; 4,16(m,4H) ; 5,36(é,2H) ; 6,58(d,1H) ; 6,75(dd,1H) ; 7,45(d,1H) |
| 150 | 1,24(t,3H) ; 2,10(s,3H) ; 3,34(q,2H) ; 4,16(t,2H) ; 4,36(t,2H) ; 7,37(d,1H) ; 7,70(d,1H) ; 8,10(s,1H) ; 10,06(s,1H) |
| 151 | 1,25(t,3H) ; 3,36(q,2H) ; 4,19(t,2H) ; 4,41(t,2H) ; 7,39(dd,1H) ; 7,84(d,1H, J = 2 Hz) ; 7,84(d,1H, J = 8,6 Hz) |
| 152 | 1,10(t,3H) ; 4,07(q,2H) ; 4,30(m,4H) ; 4,34(s,2H) ; 7,49(dd,1H) ; 7,74(d,1H) ; 7,89(d,1H) |
| 153 | 1,11(t,3H) ; 4,08(q,2H) ; 4,31(m,4H) ; 4,33(s,2H) ; 7,41(dd,1H) ; 7,82(d,1H) ; 7,86(d,1H) |

Tableau 6 (suite)

| Exemple n° | $^1H$ RMN $\delta$ (DMSO-$d_6$) |
|---|---|
| 154 | 1,53(m,8H) ; 3,30(t,2H) ; 3,61(t,2H) ; 4,18(t,2H) ; 4,45(t,2H) ; 7,47(dd,1H) ; 7,71(d,1H) ; 7,87(d,1H) |
| 155 | 1,55(m,8H) ; 3,30(t,2H) ; 3,61(t,2H) ; 4,18(t,2H) ; 4,43(t,2H) ; 7,39(dd,1H) ; 7,79(d,1H) ; 7,83(d,1H) |
| 156 | 3,69(t,2H) ; 3,87(t,2H) ; 4,26(t,2H) ; 4,47(t,2H) ; 7,48(dd,1H) ; 7,73(d,1H) ; 7,89(d,1H) |
| 157 | 3,69(t,2H) ; 3,88(t,2H) ; 4,27(t,2H) ; 4,46(t,2H) ; 7,40(dd,1H) ; 7,82(d,1H) ; 7,86(d,1H) |
| 158 | 2,14(quint., 2H) ; 3,51(t,2H) ; 3,73(t,2H) ; 4,25(t,2H) ; 4,62(t,2H) ; 8,00(d,1H) ; 8,24(dd,1H) ; 8,71(d,1H) |
| 159 | 1,68(m,4H) ; 2,54(t,2H) ; 3,36(t,2H) ; 4,18(t,2H) ; 4,45(t,2H) ; 7,49(dd,1H) ; 7,73(d,1H) ; 7,90(d,1H) |
| 160 | 1,70(m,4H) ; 2,54(t,2H) ; 3,34(t,2H) ; 4,19(t,2H) ; 4,44(t,2H) ; 7,39(dd,1H) ; 7,81(d,1H) ; 7,84(d,1H) |

Tableau 6 (suite)

| Exemple n° | $^1H$ RMN (DMSO-$d_6$) δ |
|---|---|
| 161 | 1,87(quint., 2H) ; 2,29(s,3H) ; 2,64(m,6H) ; 3,29(t,2H) ; 3,86(s,6H) ; 4,15(m,4H) ; 6,76(m,3H) ; 7,83(d,1H) ; 8,19(dd,1H) ; 8,33(d,1H) |
| 162 | 0,91(t,6H) ; 1,45(m,8H) ; 2,36(t,2H) ; 2,39(q,4H) ; 3,28(t,2H) ; 4,18(t,2H) ; 4,44(t,2H) ; 7,46(dd,1H) ; 7,72(d,1H) ; 7,86(d,1H) |
| 163 | 0,91(t,6H) ; 1,45(m,8H) ; 2,37(t,2H) ; 2,40(q,4H) ; 3,28(t,2H) ; 4,18(t,2H) ; 4,44(t,2H) ; 7,38(dd,1H) ; 7,80(d,1H) ; 7,83(d,1H) |
| 164 | 4,40(t,2H) ; 4,47(t,2H) ; 4,71(s,2H) ; 7,51(dd,1H) ; 7,77(d,1H) ; 7,92(d,1H) |
| 165 | 4,42(m,4H) ; 4,71(s,2H) ; 7,44(dd,1H) ; 7,82(d,1H) ; 7,91(d,1H) |
| 166 | 0,95(t,6H) ; 2,51(q,4H) ; 2,70(t,2H) ; 3,40(t,2H) ; 4,24(t,2H) ; 4,46(t,2H) ; 7,45(dd,1H) ; 7,68(d,1H) ; 7,84(d,1H) |

Tableau 6 (suite)

| Exemple n° | $^{1}H$ RMN ($DMSO-d_6$) $\delta$ |
|---|---|
| 167 | 0,93(t,6H); 2,48(q,4H); 2,66(t,2H); 3,37(t,2H); 4,23(t,2H); 4,44(t,2H); 7,39(dd,1H); 7,80(d,1H); 7,83(d,1H) |
| 168 | 0,98(t,3H); 1,47(m,8H); 3,10(m,5H); 3,28(t,2H); 4,16(t,2H); 4,42(t,2H); 7,40(dd,1H); 7,81(d,1H); 7,83(d,1H) |
| 169 | 4,71(m,4H); 7,41(m,2H); 7,50(dd,1H); 7,84(d,1H); 7,92(m,2H); 8,38(d,1H) |

Exemples 87 à 126 et 170 à 176

Les composés identifiés par les exemples 87 à 126 et 170 à 176 dans les tableaux 5 à 10 sont préparés par la même méthode de préparation décrite dans l'exemple 86.

Exemple 180

Méthode C : Préparation de 1,2,3,4,13,14-hexahydro-pyrido[1',2':2,3][1,2,5]-thiadiazino[5,6-a]benzimidazole 6,6-dioxyde.

On met à reflux pendant 1 heure 2,95 g (0,01 mole) de 2-(sulfonyl-2-hydroxyméthyl-1-pipéridinyl)-1H-benzimidazole dans 20 ml de chlorure de thionyle. On évapore à sec, on recristallise le résidu dans un mélange éthanol/acétate d'éthyle (1:1) et on obtient 2,0 g (73 %) de 1,2,3,4,13,14-hexahydro-pyrido[1',2':2,3][1,2,5]-thiadiazino[5,6-a]benzimidazole 6,6-dioxyde, de point de fusion 246-8°C.

Les données pour l'identification du produit se présentent dans les tableaux 11 et 12.

Exemple 177

Méthode D : Préparation de 2H-2-éthyl-3-phényl-[1,2,5]-thiadiazino[5,6-a]benzimidazole 1,1-dioxyde.

On agite à 125°C pendant 4 heures un mélange de 13,7 g (0,04 mole) de 1-benzoylméthyl-N-éthyl-1H-benzimidazole-2-sulfonamide et 230 g d'acide polyphosphorique. On verse la solution résultante sur de la glace, on filtre, on lave avec de l'eau, puis avec une solution aqueuse de carbonate acide de sodium et à nouveau avec de l'eau et on obtient 8,7 g (67 %) de 2H-2-éthyl-3-phényl-[1,2,5]-thiadiazino[5,6-a]benzimidazole 1,1-dioxyde, de point de fusion 155-8°C.

Les données pour l'identification du produit se présentent dans les tableaux 9 et 10.

Tableau 7

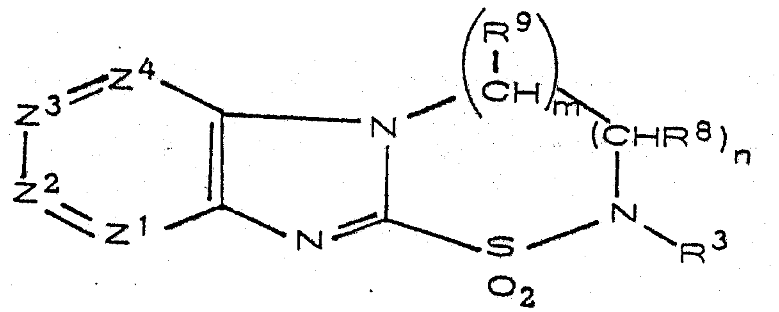

| Exemple nº | $R^3$ | n | m | $R^8$ | $R^9$ | $Z^1$ | $Z^2$ | $Z^3$ | $Z^4$ | Pf (ºC) | IR (KBr) |
|---|---|---|---|---|---|---|---|---|---|---|---|
| 170 | $-CH_2CH_3$ | 1 | 1 | $CH_3$ | H | CH | CH | CH | CH | 137-41 | 1340;1180;1155 |
| 171 | $-CH_2CH_3$ | 1 | 1 | $CH_3$ | H | CH | $C-NO_2$ | CH | CH | 170-200 | 1520;1350;1180;1155 |
| 172 | $-CH_2CH_3$ | 1 | 1 | $CH_3$ | H | CH | CH | $C-NO_2$ | CH | | |
| 173 | $-(CH_2)_3CH_3$ | 1 | 1 | H | H | CH | CH | CH | N | 143-5 | 1355;1175;1160 |
| 174 | $-(CH_2)_3CH_3$ | 1 | 1 | H | H | CH | N | CH | CH | 227-31 | 1355;1165 |
| 175 | $-CH_2CH_3$ | 1 | 0 | $C_6H_5$ | - | CH | CH | CH | CH | 120-2 | 1320;1170 |

Tableau 8

| Exemple n° | $^1H$ RMN (DMSO-$d_6$) δ |
|---|---|
| 170 | 1,20(t,3H) ; 1,49(d,3H) ; 3,25(q,2H) ; 4,37(d,2H) ; 4,67(m,1H) ; 7,31-7,85(m,4H) |
| 171 | 1,22(t,3H) ; 1,53(d,3H) ; 3,32(q,2H) ; 4,04-4,83(m,3H) ; 7,87(d,1H) ; 8,31(dd,1H) ; 8,67(d,1H) |
| 172 | 1,22(t,3H) ; 1,53(d,3H) ; 3,32(q,2H) ; 4,10-4,78(m,3H) ; 7,97(d,1H) ; 8,22(dd,1H) ; 8,72(d,1H) |
| 173 | 0,91(t,3H) ; 1,45(m,4H) ; 3,34(t,2H) ; 4,18(t,2H) ; 4,46(t,2H) ; 7,48(dd,1H) ; 8,27(dd,1H) ; 8,57(dd,1H) |
| 174 | 0,92(t,3H) ; 1,46(m,4H) ; 3,32(t,2H) ; 4,17(t,2H) ; 4,47(t,2H) ; 7,73(dd,1H) ; 8,52(d,1H) ; 9,12(dd,1H) |
| 175 | 1,18(t,3H) ; 3,28(q,2H) ; 7,01(s,1H) ; 6,90-7,46(m,3H) ; 7,55(s,5H) ; 7,88(m,1H) |

Tableau 9

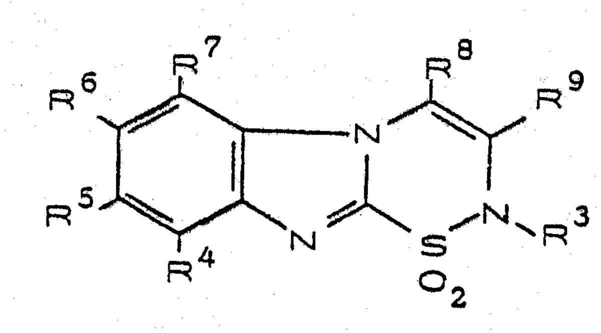

| Exemple nº | $R^8$ | $R^9$ | $R^3$ | $R^4$ | $R^5$ | $R^6$ | $R^7$ | Pf (ºC) | IR (KBr) |
|---|---|---|---|---|---|---|---|---|---|
| 176 | H | H | $-CH_2CH_3$ | H | H | H | H | 123-7 | 1350;1160 |
| 177 | H | 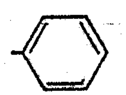 | $-CH_2CH_3$ | H | H | H | H | 155-8 | 1365;1180 |
| 178 | H | $-CH_3$ | $-CH_2CH_3$ | H | H | H | H | 153-5 | 1355;1180 |
| 179 | H | 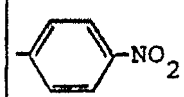 | $-CH_2CH_3$ | H | H | $NO_2$ | H | 307-15 | 1520;1380;1340;1185 |

Tableau 10

| Exemple nº | $^1H$ RMN (DMSO-$d_6$) δ |
|---|---|
| 176 | 1,26(t,3H); 3,78(q,2H); 6,76(d,1H); 7,52(m,2H); 7,73(d,1H); 7,95(m,2H) |
| 177 | 0,75(t,3H); 3,55(q,2H); 7,53(m,5H); 7,83(m,3H); 8,21(d,1H); 8,51(s,1H) |
| 178 | 1,00(t,3H); 2,20(s,3H); 3,75(q,2H); 7,47(m,2H); 7,76(s,1H); 7,90(m,2H) |
| 179 | 0,78(t,3H); 3,57(q,2H); 8,12(m,3H); 8,37(m,3H); 8,88(s,1H); 9,33(d,1H) |

Tableau 11

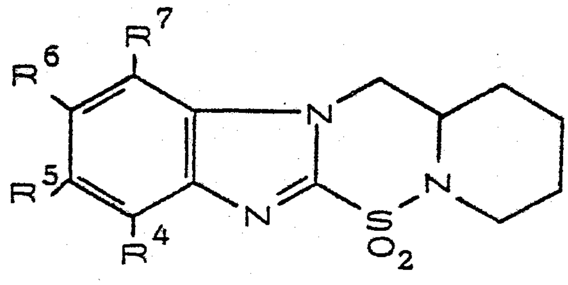

| Exemple nº | $R^4$ | $R^5$ | $R^6$ | $R^7$ | Pf (ºC) | IR (KBr) |
|---|---|---|---|---|---|---|
| 180 | H | H | H | H | 246-8 | 1350;1165 |
| 181 | H | $NO_2$ | H | H | 250-66 | 1520;1365;1350;1170 |
| 182 | H | H | $NO_2$ | H | | |

Tableau 12

| Exemple nº | $^1H$ RMN (DMSO-$d_6$) δ |
|---|---|
| 180 | 1,82(m,6H); 3,02(m,1H); 4,39(m,2H); 4,71(d,2H); 7,31-7,88(m,4H) |
| 181 | 1,80(m,6H); 3,38(m,1H); 4,50(m,2H); 4,65(d,2H); 7,80(d,1H); 8,34(dd,1H); 8,62(d,1H) |
| 182 | 1,85(m,6H); 3,52(m,1H); 4,60(m,2H); 4,73(d,2H); 8,01(d,1H); 8,24(dd,1H); 8,67(d,1H) |

Exemple 178

Le composé identifié par l'exemple 178 dans les tableaux 9 et 10, est préparé par la même méthode de préparation décrite dans l'exemple 177.

Exemple 62

Méthode E : Préparation de 1,N-diéthyl-1H-benzimidazole-2-sulfonamide.

On ajoute 46,8 g (0,3 mole) d'iodure d'éthyle à une suspension de 67,5 g (0,3 mole) de N-éthyl-1H-benzimidazole-2-sulfonamide et 47,7 g (0,45 mole) de carbonate de sodium dans 450 ml d'acétone et 20 ml d'eau. On laisse à reflux pendant 20 heures, on évapore l'acétone, on ajoute 200 ml d'eau et on extrait avec du chlorure de méthylène. On sèche ($Na_2SO_4$) la couche organique et on évapore. On recristallise le résidu avec un mélange éthanol/eau (2:1) et on obtient 56,8 g (75 %) de 1,N-diéthyl-1H-benzimidazole-2-sulfonamide de point de fusion 135-9°C.

Les données pour l'identification du produit se présentent dans les tableaux 3 et 4.

Exemples 63 à 70

Les composés identifiés par les exemples 63 à 70 dans les tableaux 3 et 4, sont préparés par la même méthode de préparation décrite dans l'exemple 62.

Exemple 71

Méthode F : Préparation de 6-nitro-1,N,N-triéthyl-1H-benzimidazole-2-sulfonamide.

On ajoute 2,6 g (0,11 mole) d'hydrure de sodium à une solution de 29,8 g (0,1 mole) de 6-nitro-1,N-diéthyl-1H-benzimidazole-2-sulfonamide dans 100 ml de diméthylformamide. On agite la solution pendant une heure à 40°C, on ajoute 10,8 g (0,1 mole) de bromure d'éthyle et on maintient l'agitation pendant 12 heures à 50°C. On verse sur de l'eau, on filtre et on lave avec de l'eau. On recristallise le précipité dans l'éthanol

et on obtient 25,6 g (78 %) de 6-nitro-1,N,N-triéthyl-1H-benzimidazole-2-sulfonamide, de point de fusion 90-1°C.

Les données pour l'identification du produit se présentent dans les tableaux 3 et 4.

Exemples 72 à 76

Les composés identifiés par les exemples 72 à 76 dans les tableaux 3 et 4, sont préparés par la même méthode de préparation décrite dans l'exemple 71.

Exemple 134

Méthode G : Préparation de 2H-2-butyl-3,4-dihydro-7-nitro-[1,2,5]-thiadiazino[5,6-a]benzimidazole 1,1-dioxyde.

On ajoute 2,6 g (0,11 mole) d'hydrure de sodium à une solution de 26,8 g (0,1 mole) de 3,4-dihydro-2H-7-nitro-[1,2,5]-thiadiazino[5,6-a] benzimidazole 1,1-dioxyde dans 200 ml de diméthylformamide. On agite la solution pendant une heure à température ambiante et on ajoute ensuite 13,6 g (0,1 mole) de bromure de butyle. On maintient l'agitation pendant 3 heures de plus à 40°C, on verse sur de l'eau, on filtre et on lave avec de l'eau. Le précipité recristallisé dans l'acétonitrile donne 29,2 g (90 %) de 2H-2-butyl-3,4-dihydro-7-nitro-[1,2,5]-thiadiazino[5,6-a]benzimidazole 1,1-dioxyde, de point de fusion 195-7°C.

Les données pour l'identification du produit se présentent dans les tableaux 5 et 6.

Exemples 86, 88 à 97, 115 à 122, 126 à 138 et 152 à 169

Les composés identifiés par les exemples 86, 88 à 97, 115 à 122, 126 à 138 et 152 à 169 dans les tableaux 5 et 6, sont préparés par la même méthode de préparation décrite dans l'exemple 134.

Exemples 127 et 128.

Méthode H : Préparation de 3,4-dihydro-2H-2-éthyl-7-nitro-[1,2,5]-thiadiazino[5,6-a]benzimidazole

1,1-dioxyde et de 3,4-dihydro-2H-2-éthyl-8-nitro-[1,2,5]-thiadiazino[5,6-a]benzimidazole 1,1-dioxyde.

A une solution de 100,4 g (0,4 mole) de 3,4-dihydro-2H-2-éthyl-[1,2,5]-thiadiazino[5,6-a]benzimidazole 1,1-dioxyde dans 500 ml d'acide sulfurique concentré à 5°C, on ajoute goutte à goutte une solution froide (entre 5°C et 10°C) formée par 45 ml d'acide sulfurique concentré et 55 ml d'acide nitrique à 65 %. Une fois l 'addition terminé on agite pendant 2 heures à température ambiante, on verse sur de la glace, on filtre, on lave avec de l'eau et avec de l'éthanol et on obtient 110,1 g (93 %) d'un mélange des deux isomères.Par recristallisation fractionnée dans le nitrométhane on obtient 51,3 g de 3,4-dihydro-2H-2-éthyl-7-nitro-[1,2,5]-thiadiazino[5,6-a]benzimidazole 1,1-dioxyde, de point de fusion 255-8°C et 47,8 g de 3,4-dihydro-2H-2-éthyl-8-nitro-[1,2,5]-thiadiazino[5,6-a]benzimidazole 1,1-dioxyde, de point de fusion 208-10°C.

Les données pour l'identification des produits se présentent dans les tableaux 5 et 6.

Exemples 34 à 38, 42, 71, 72, 77 à 80, 129 à 148, 171, 172, 179, 181 et 182

Les composés identifiés par les exemples 34 à 38, 42, 71, 72, 77 à 80, 129 à 148, 171, 172, 179, 181 et 182 dans les tableaux 1 à 12, sont préparés par la même méthode de préparation décrite dans les exemples 127 et 128.

Exemple 149

Méthode J : Préparation de 7-amino-3,4-dihydro-2H-2-éthyl-[1,2,5]-thiadiazino[5,6-a]benzimidazole 1,1-dioxyde.

On hydrogène à température ambiante pendant 12 heures à une pression de 60 psi une solution de 5,9 g (0,02 mole) de 3,4-dihydro-2H-2-éthyl-7-nitro-[1,2,5]-thiadiazino[5,6-a]benzimidazole 1,1-dioxyde dans 200 ml de méthanol et 0,2 g de palladium sur du charbon à 10 %. On filtre, on concentre le méthanol et on obtient 4,6 g (87 %) de 7-amino-3,4-dihydro-2H-2-éthyl[1,2,5]thiadiazino[5,6-a]benzimidazole 1,1-dioxyde, de point de fusion 232-4°C.

Les données pour l'identification du produit se présentent dans les tableaux 5 et 6.

Exemples 81 à 84

Les composés identifiés par les exemples 81 à 84 dans les tableaux 3 et 4, sont préparés par la même méthode de préparation décrite dans l'exemple 149.

Exemple 150

Méthode K : Préparation de 7-acétylamino-3,4-dihydro-2H-2-éthyl-[1,2,5]-thiadiazino[5,6-a]benzimidazole 1,1-dioxyde.

On ajoute lentement 1,6 g (0,02 mole) de chlorure d'acétyle à une solution de 5,3 g (0,02 mole) de 7-amino-3,4-dihydro-2H-2-éthyl-[1,2,5]-thiadiazino[5,6-a]-benzimidazole 1,1-dioxyde dans 25 ml de pyridine refroidie dans un bain eau/glace. Une fois l'addition terminée on agite pendant 2 heures à température ambiante et on verse sur de la glace. On filtre, on lave avec de l'eau, on sèche et on obtient 4,2 g (68 %) de 7-acétylamino-3,4-dihydro-2H-2-éthyl-[1,2,5]-thiadiazino[5,6-a]benzimidazole 1,1-dioxyde, de point de fusion 245-8°C.

Les données pour l'identification du produit se présentent dans les tableaux 5 et 6.

Exemples 44, 45 et 85

Les composés identifiés par les exemples 44, 45 et 85 dans les tableaux 1, 2, 3 et 4, sont préparés par la même méthode de préparation décrite dans l'exemple 150.

Exemple 151

Méthode L : Préparation de 3,4-dihydro-2H-2-éthyle-7-isothiocyanate-[1,2,5]-thiadiazino[5,6-a]-benzimidazole 1,1-dioxyde.

On ajoute sous agitation une solution de 4,5 g (0,017 mole) de 7-amino-3,4-dihydro-2H-2-éthyl-[1,2,5]-thiadiazino[5,6-a]benzimidazole 1,1-dioxyde dans 100 ml d'acide chlorhydrique 4N à une solution de 2 ml (0,02 mole) de thiophosgène dans 100 ml de chloroforme. On agite à température ambiante jusqu'à disparition de la couleur orange de la couche organique (environ 6 heures), on sépare cette dernière par décantation, on lave avec de l'eau, on sèche sur du sulfate de sodium et on évapore à sec. On recristallise le résidu dans un mélange acétone/eau (9:1) et on obtient 3,0 g (49 %) de 3,4-dihydro-2H-2-éthyl-7-isothiocyanate-[1,2,5]-thiadiazino[5,6-a]benzimidazole 1,1-dioxyde, de point de fusion 218-25°C.

Les données pour l'identification du produit se présentent dans les tableaux 5 et 6.

Exemple 103

Méthode M : Préparation de 7-chloro-3,4-dihydro-2H-2-(4-diéthylamino-1-méthylbutyl)-[1,2,5]-thiadiazino-[5,6-a]benzimidazole 1,1-dioxyde.

On ajoute goutte à goutte 1,5 g (0,022 mole) de nitrite de sodium dissous dans 5 ml d'eau, à une solution de 7,58 g (0,02 mole) de 7-amino-3,4-dihydro-2H-2-(4-diéthylamino-1-méthylbutyl)-[1,2,5]-thiadiazino-[5,6-a]benzimidazole 1,1-dioxyde dans 50 ml d'acide chlorhydrique 12N, en maintenant la température entre -5°C et 0°C. Une fois l'addition complétée, on agite 15 minutes à la même température et on verse le tout sur une solution de 4,4 g (0,044 mole) de chlorure cuivreux dans 25 ml d'acide chlorhydrique concentré, préalablement refroidie à 0°C, en plusieurs fois pendant

5 minutes en prenant la précaution d'éviter une effervescence excessive. Une fois l'addition terminée on laisse chauffer jusqu'à température ambiante en maintenant l'agitation et puis on chauffe jusqu'à une température de 70°C. On laisse refroidir jusqu'à une température ambiante et on ajoute 100 ml d'eau. On filtre, on lave avec de l'eau, on sèche et on obtient 2,5 g (31 %) de 7-chloro-3,4-dihydro-2H-2-(4-diéthylamino-1-méthyl-butyl)-[1,2,5]-thiadiazino[5,6-a]benzimidazole 1,1-dioxyde, de point de fusion 150-2°C.

Les données pour l'identification du produit se présentent dans les tableaux 5 et 6.

Activité inhibitrice de la sécrétion acide gastrique

Méthode de Shay [Shay, H.; Komarov, S.A.; Fels, S.S.; Merange, D.; Grvenstein, M.; Siplet, H. : Gastroenterology, 5, 43 (1945) - Visscher, F.E.; Seay, P.H.; Taxelaar, A.P.; Veldhamp, W.; Vander Brook, M.J. : J. Pharmac. Exp. Ther., 110, 188 (1954) - "Animal Experiments in Pharmacological Analysis", F.R. Domer; C.C. Thomas Pub, Springfield, Illinois, USA, 1970, p. 140].

Dans ce test on utilise des rats Wistar, mâles, de 200 à 250 grammes de poids, que l'on maintient à jeun depuis le jour antérieur à celui de l'essai, avec accès libre à l'eau. On utilise des lots de 4 animaux chacun, au minimum.

On anesthésie les rats avec de l'éther éthylique, on leur pratique une laparotomie et on leur ligature le pylore, puis on effectue la couture de l'incision abdominale. L'administration des produits, avec le véhicule pour le lot témoin, s'effectue par voie intraduodénale (i.d.) avant de coudre l'incision abdominale. La dose administrée pour le premier essai est de 40 mg/kg et on détermine également dans un deuxième essai la dose efficace cinquante (DE-50) par voie i.d.. Le véhicule utilisé est la gomme arabique à 5 % p/v dans

l'eau bidistillée.

Deux heures après la ligature du pylore, on sacrifie les rats par anesthésie prolongée avec de l'éther éthylique et on mesure le volume du suc gastrique, on détermine l'acidité totale moyennant un pH-mètre muni d'une burette automatique. Pour chaque produit et pour chaque dose essayés, on détermine le pourcentage d'inhibition de la sécrétion acide gastrique par rapport au lot témoin.

A titre d'exemples non limitatifs, on résume les résultats obtenus pour quelques uns des dérivés de la présente invention dans le tableau 13.

Tableau 13

Inhibition de la sécrétion acide gastrique chez le rat Shay.

| Exemple n° | Pourcentage d'inhibition de la sécrétion acide gastrique (Dose = 40 mg/kg, voie i.d.) | DE-50 (mg/kg, voie i.d.) |
|---|---|---|
| 1 | 78,7 | 22,4 |
| 5 | 96,4 | 10,8 |
| 11 | 48,8 | 42,0 |
| 14 | 61,4 | 30,6 |
| 16 | 48,0 | 42,6 |
| 18 | 41,0 | 53,5 |
| 26 | 53,0 | 38,5 |
| 28 | 51,4 | 38,5 |
| 29 | 54,2 | 37,9 |
| 34 | 63,0 | 29,9 |
| 36 | 40,0 | 65,6 |
| 62 | 52,4 | 31,0 |
| 63 | 52,0 | 49,2 |
| 68 | 53,8 | 39,7 |
| 69 | 54,3 | 39,2 |
| 73 | 77,5 | 18,5 |

Tableau 13 (Suite)

| Exemple n° | Pourcentage d'inhibition de la sécrétion acide gastrique (Dose = 40 mg/kg, voie i.d.) | DE-50 (mg/kg, voie i.d.) |
|---|---|---|
| 74 | 76,4 | 17,9 |
| 90 | 51,4 | 64,0 |
| 100 | 51,8 | 37,6 |
| 102 | 71,4 | 14,1 |
| 103 | 72,3 | 15,0 |
| 115 | 51,1 | 38,9 |
| 116 | 50,3 | 39,4 |
| 127 | 56,3 | 52,4 |
| 133 | 40,0 | 58,3 |
| 134 | 70,3 | 20,7 |
| 141 | 80,9 | 16,3 |
| 142 | 79,4 | 17,5 |
| 143 | 99,3 | 20,5 |
| 144 | 93,7 | 10,0 |
| 145 | 68,7 | 19,8 |
| 146 | 67,5 | 20,3 |
| 147 | 46,5 | 44,0 |
| 148 | 45,3 | 46,0 |
| 149 | 64,0 | 32,6 |
| 150 | 53,4 | 41,6 |
| 170 | 73,2 | 21,8 |
| 173 | 57,1 | 39,2 |
| 177 | 53,7 | 38,1 |
| 180 | 43,8 | 43,7 |
| 181 | 43,4 | 62,0 |
| 182 | 42,1 | 61,2 |

Activité antiulcéreuse

On démontre l'activité antiulcéreuse au moyen de l'étude de l'inhibition des lésions gastriques induites par le stress dû à l'immobilisation et le froid chez le rat, d'après la méthode de Senay et cols. (Senay, E.C.; Levine, R.J. : Proc. Soc. Exp. Biol. Med., 124, 1221-1227 (1967).

Dans ce test on utilise des rats Wistar, mâles, de 150 à 200 grammes de poids, maintenus à jeun dans des cages individuelles 24 heures avant le début de l'étude, mais avec de l'eau "ad libitum". On utilise des lots de 5 animaux chacun, au minimum.

Au début de l'étude, les rats reçoivent les produits décrits, ou bien le véhicule dans le cas du lot témoin, par voie orale, au moyen d'une sonde gastrique. Les doses administrées des produits décrits est de 40 mg/kg. Le véhicule utilisé est la gomme arabique à 5 % p/v dans l'eau bidistillée.

Immédiatement après le traitement, on introduit les rats dans des ceps en plastique en forme de tube d'un diamètre d'environ 5 cm et de longueur variable, adaptable à la taille du rat. Une heure après le traitement on introduit les rats dans leur cep, dans un congélateur à -5°C pendant 3 heures. Immédiatement après l'exposition au froid, on tue les rats par anesthésie prolongée avec de l'éther éthylique, on extrait les estomacs, on les ouvre le long de la courbature majeure et on évalue les lésions de la muqueuse de l'estomac. Pour cette évaluation on compte les points hémorragiques et on mesure la grandeur des ulcères nécrosées.

Pour chaque produit et pour chaque dose essayée on détermine le pourcentage d'inhibition des lésions gastriques, par rapport au lot témoin.

Dans le tableau 14 on résume à titre d'exemple non limitatif les résultats obtenus pour un des produits

décrits.

Tableau 14

| Produit | Dose (voie orale) | Nº de rats | Evaluation des lésions ($\bar{x}$ ± écart type de la moyenne) | Inhibition |
|---|---|---|---|---|
| Témoin (gomme arabique) | 10 ml/kg | 10 | 6,2 ± 1,0 | - |
| Exemple 144 | 40 mg/kg | 10 | 0 | 100% |

Cytoprotection gastrique

On démontre l'activité cytoprotectrice gastrique au moyen de l'étude de l'inhibition des lésions gastriques induites par l'éthanol chez le rat, d'après la méthode décrite par Robert et cols. (Robert, A.; Nezamis, J.E.; Lancaster, C. et Hanchar, A.J. : Gastroenterology, 77 : 433-443 (1979)).

Dans ce test on utilise des rats Wistar, mâles, de 150 à 200 grammes de poids, maintenus à jeun depuis 24 heures, mais avec de l'eau "ad libitum". On utilise des lots de 5 animaux chacun, au minimum.

Au début de l'étude, les rats reçoivent les produits décrits, ou le véhicule lorsqu'il s'agit des rats du lot témoin, par voie orale, au moyen d'une sonde gastrique. La dose administrée pour les produits décrits est de 40 mg/kg. Le véhicule employé est la gomme arabique à 5 % p/v dans l'eau bidistillée.

Trente minutes après le traitement, les rats reçoivent 1 ml d'éthanol absolu par voie orale, au moyen d'une sonde gastrique. Une heure après le traitement avec l'éthanol, on sacrifie les rats par anesthésie prolongée avec de l'éther éthylique, on leur extrait les estomacs, on les ouvre le long de la courbature majeure et on évalue les lésions de la muqueuse de l'estomac.

Pour cette évaluation on compte les points hémorragiques et on mesure la grandeur des ulcères nécrosées.

Pour chaque produit essayé, on détermine le pourcentage d'inhibition des lésions gastriques, par rapport au lot témoin.

Dans le tableau 15 on résume, à titre d'exemples non limitatifs les résultats obtenus pour deux des produits décrits.

Tableau 15

Cytoprotection de la muqueuse gastrique chez le rat. Protection des lésions induites par l'éthanol.

| Produit | Dose (voie orale) | Nº de rats | Evaluation des lésions ($\bar{x}$ ± écart type de la moyenne) | Inhibition |
|---|---|---|---|---|
| Témoin (gomme arabique) | 10 ml/kg | 16 | 27,6 ± 6,0 | - |
| Exemple 5 | 40 mg/kg | 5 | 4,2 ± 2,1 | 85% |
| Exemple 142 | 40 mg/kg | 6 | 0 | 100% |

Toxicité aiguë chez la souris

Méthode de Litchfield et Wilcoxon (Litchfield, J.T. et Wilcoxon, E.J. : J. Pharmacol. Exp. Therap., 96, 19-113 (1949)).

On administre le produit par voie orale en suspension dans la gomme arabique à 5 % dans l'eau bidistillée. Le volume administré est de 10 ml/kg. On calcule selon la méthode citée la dose léthale cinquante (DL-50).

A titre d'exemples non limitatifs, on résume

les résultats obtenus pour quelques uns des dérivés de la présente invention dans le tableau 16.

Tableau 16

| Produit | Sexe | DL-50 (mg/kg) voie orale |
|---|---|---|
| Exemple 6 | ♂ | 3300 |
| | ♀ | 2600 |
| Exemple 8 | ♂ | 6400 |
| | ♀ | 6400 |
| Exemple 16 | ♂ | 2000 |
| | ♀ | 1500 |
| Exemple 18 | ♂ | 2000 |
| | ♀ | 4000 |
| Exemple 21 | ♂ | >1600 |
| | ♀ | >1600 |
| Exemple 31 | ♂ | >6400 |
| | ♀ | >6400 |
| Exemple 34 | ♂ | >6400 |
| | ♀ | >6400 |
| Exemple 38 | ♂ | >6400 |
| | ♀ | >6400 |

Tableau 16 (suite)

| Produit | Sexe | DL-50 (mg/kg) voie orale |
|---|---|---|
| Exemple 71 | ♂ | 6400 |
| | ♀ | 6400 |
| Exemple 92 | ♂ | 6400 |
| | ♀ | 6400 |
| Exemple 99 | ♂ | >3200 |
| | ♀ | >3200 |
| Exemple 127 | ♂ | >6400 |
| | ♀ | >6400 |
| Exemple 128 | ♂ | >2200 |
| | ♀ | >3200 |
| Exemple 133 | ♂ | >3200 |
| | ♀ | >3200 |
| Exemple 134 | ♂ | >3200 |
| | ♀ | >3200 |
| Exemple 179 | ♂ | >6400 |
| | ♀ | >6400 |

En thérapeutique humaine, la dose d'administration des dérivés de la présente invention est bien sûr fonction de la gravité de l'affection à traiter, elle sera généralement comprise entre environ 30 et environ 60 mg/jour. Les dérivés de l'invention seront par exemple administrés sous la forme de comprimés ou d'ampoules injectables.

On indiquera ci-après, à titre d'exemples, deux formes galéniques particulières des dérivés objet de la présente invention.

Exemple de formule par comprimé

Comprimés de 30 mg

| | |
|---|---|
| Exemple 144 | 0,030 g |
| Lactose | 0,0342 g |
| Amidon | 0,030 g |
| Polyvinylpyrrolidone | 0,006 g |
| Cellulose microcristalline | 0,018 g |
| Dioxyde de silice colloïdal | 0,0012 g |
| Stéarate de magnésium | 0,0006 g |
| | 0,120 g |

Comprimés de 60 mg

| | |
|---|---|
| Exemple 144 | 0,060 g |
| Lactose | 0,0684 g |
| Amidon | 0,060 g |
| Polyvinylpyrrolidone | 0,012 g |
| Cellulose microcristalline | 0,036 g |
| Dioxyde de silice colloïdal | 0,0024 g |
| Stéarate de magnésium | 0,0012 g |
| | 0,240 g |

Exemple de formule par ampoule injectable

Injectables 6 mg/ml

| | |
|---|---|
| Exemple 144 | 0,030 g |
| Chlorure de sodium c.s. | 0,050 g |
| Acide ascorbique | 0,005 g |
| Eau p. injection c.s.p. | 5 ml |

Compte tenu des intéressantes propriétés pharmacologiques attachées aux nouveaux composés de formule générale I, la présente invention s'étend également à l'application de ces composés à titre de médicaments, aux compositions pharmaceutiques les contenant et à leur

utilisation pour la fabrication de médicaments destinés au traitement des maladies gastrointestinales, en particulier pour la fabrication d'agents inhibiteurs de la sécrétion d'acide gastrique, d'agents antiulcéreux et d'agents cytoprotecteurs.

## - REVENDICATIONS -

1 - Dérivés de benzimidazole-2-sulfonamide et d'imidazopyridine-2-sulfonamide répondant à la formule générale I

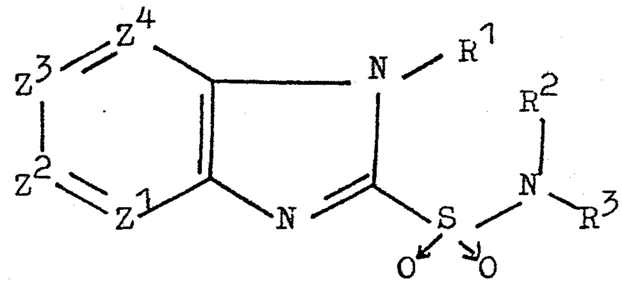

I

et leurs sels pharmaceutiquement acceptables, formule dans laquelle :

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$ peut représenter un atome d'azote;

$R^1$ et $R^2$ représentent indépendamment un atome d'hydrogène, un radical alkyle linéaire ou ramifié, un radical alkylaryle, un radical alkylcarbonylalkyle, un radical alkylcarbonylaryle, un radical alkylcarbonylhétéroaryle, un radical cycloalkyle, un radical carboxyalkyle, un radical acyle, un radical nitro, ou bien $R^1$ et $R^2$ peuvent former ensemble une liaison représentée par un groupe X;

$R^3$ représente un atome d'hydrogène, un radical alkyle linéaire ou ramifié, un radical aryle, un radical

hétéroaryle, un radical alkylaryle, un radical hydroxyalkyle, un radical alkylhétéroaryle, un radical mono-, bi- ou tricycloalkyle, un radical alkylamino, un radical N-alkyl-alkylamino, un radical alkylcarboxyalkyle, un radical acyle, un radical alkylthio (ou sulfinyl, ou sulfonyl)alkyle, un radical alkylthio (ou sulfinyl, ou sulfonyl)aryle, un radical alkylthio (ou sulfinyl, ou sulfonyl)hétéroaryle, un radical alkylthio (ou sulfinyl, ou sulfonyl)alkylaryle, un radical alkylthio (ou sulfinyl, ou sulfonyl)alkylhétéroaryle, un radical haloalkyle, un radical cyanoalkyle, un radical N-alkylaryl-alkylamino, un radical N-alkyl-N-alkylarylalkylamino, un radical N,N-dialkylalkylamino, un radical alkylpipérazinyle, un radical alkylpipéridinyle ou un radical alkylmorpholinyle;

X représente $-(CH\ R^8)_n-(CH\ R^9)_m-$ ou $-CR^8=CR^9-$;

$R^2$ et $R^3$ peuvent former ensemble une liaison représentée par un groupe $-(CH_2)_n-Y_m-CH\ R^{10}-$;

Y représente $-CH_2-O-CH_2-$ ou $-CH_2-NR^{11}-CH_2-$;

n représente 1, 2, 3 ou 4;

m représente 0 ou 1;

$R^4$ et $R^7$ représentent indépendamment un atome d'hydrogène, un atome d'halogène, un radical nitro, un radical amino ou acylamino (tel que acétylamino);

$R^5$ et $R^6$ représentent indépendamment un atome d'hydrogène, un atome d'halogène, un radical nitro, un radical alkyle inférieur, un radical trifluorométhyle, un radical alcoxy, un radical aryloxy, un radical mercapto, un radical alkylthio, un radical alkylsulfinyl, un radical alkylsulfonyl, un radical isothiocyanate, un radical sulfamoyle, un radical alkylcarbonyle, un radical arylcarbonyle, un radical acylamino (tel que acétylamino), un radical cyano, un radical carboxylique, un radical carboxamido ou un radical carboxyalkyle;

$R^8$ et $R^9$ représentent indépendamment un atome d'hydrogène, un radical alkyle inférieur, un radical aryle ou un radical hétéroaryle;

$R^{10}$ représente un atome d'hydrogène, un radical $-CH_2OH$, un radical $-CH_2Cl$ ou un radical méthylène ($-CH_2-$);

$R^1$ peut former une liaison avec $R^{10}$ dans le cas où $R^{10}$ représente un radical méthylène ($-CH_2-$) et lui-même ($R^1$) représente une liaison;

$R^{11}$ représente un atome d'hydrogène, un radical alkyle ou un radical hydroxyalkyle;

Aryle représente un groupe phényle ou un groupe phényle substitué;

Hétéroaryle représente un groupe hétérocyclique aromatique dans lequel l'hétéroatome ou atomes du cycle est/sont sélectionnés parmi O et N; le groupe hétérocyclique peut être substitué,

étant entendu que la formule générale I, peut également désigner dans le cas où $R^1$ représente un atome d'hydrogène, une forme tautomère alternative de formule générale I', dans laquelle les différents substituants ont les mêmes désignations que celles données à propos de la formule générale I,

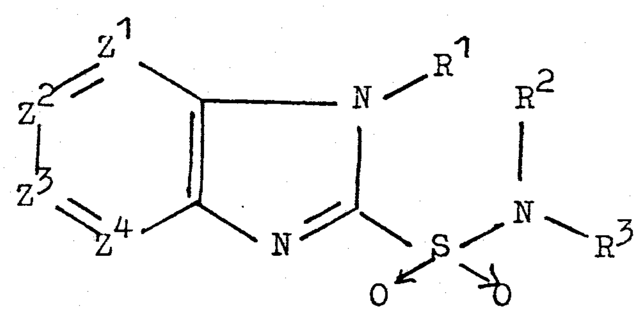

I'

à l'exception toutefois des composés de formule I, dans laquelle :

$Z^1$, $Z^2$, $Z^3$ et $Z^4$ représentent CH ;

$R^1$ et $R^2$ représentent H, et

$R^3$ représente H, phényle, m-chlorophényle, m-tolyle,

éthyle, n-propyle, n-octyle, n-décyle, n-hexadécyle, n-octadécyle;

des composés de formule I, dans laquelle :

$Z^1$, $Z^2$, $Z^3$ et $Z^4$ représentent CH;

$R^1$ représente H;

$R^2$ représente méthyle et $R^3$ représente phényle, ou

$R^2$ représente carboxyéthyle et $R^3$ représente phényle, ou

$R^2$ et $R^3$ représentent méthyle, ou $R^2$ et $R^3$ forment avec le nitrogène ou noyau de morpholine, et

des composés de formule I, dans laquelle :

$Z^1$, $Z^3$ et $Z^4$ représentent CH;

$Z^2$ représente $C-CH_3$;

$R^1$ représente H;

$R^2$ représente H et $R^3$ représente phényle, ou

$R^2$ et $R^3$ forment avec le nitrogène un noyau de morpholine.

2 - Les composés répondant à la formule générale I, selon la revendication 1, représentés par la formule générale Ia

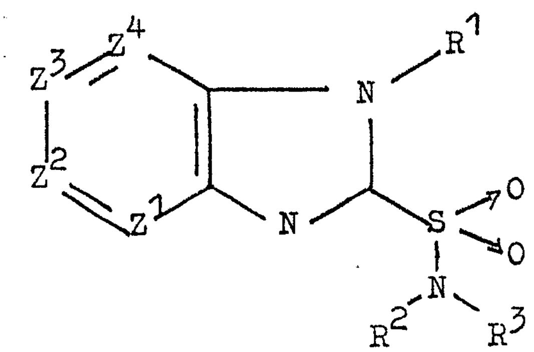

Ia

dans laquelle :

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$, peut représenter un atome d'azote;

$R^1$ représente un atome d'hydrogène, un radical alkyle linéaire ou ramifié, un radical alkylaryle, un radical alkylcarbonylalkyle, un radical alkylcarbonylaryle, un radical alkylcarbonylhétéroaryle, un radical cycloalkyle ou un radical carboxyalkyle;

$R^2$ représente un atome d'hydrogène ou un radical alkyle inférieur linéaire ou ramifié en $C_1$ à $C_5$; un radical nitro ou un radical acyle;

$R^3$ représente un atome d'hydrogène, un radical alkyle linéaire ou ramifié, un radical aryle, un radical hétéroaryle, un radical alkylaryle, un radical alkylhétéroaryle, un radical mono-, bi- ou tricycloalkyle, un radical alkylamino, un radical N-alkyl-alkylamino, un radical alkylcarboxyalkyle, un radical acyle, un radical alkylthio (ou sulfinyl, ou sulfonyl)alkyle, un radical alkylthio (ou sulfinyl, ou sulfonyl)aryle, un radical alkylthio (ou sulfinyl, ou sulfonyl)hétéroaryle, un radical alkylthio (ou sulfinyl, ou sulfonyl)alkylaryle, un radical alkylthio (ou sulfinyl, ou sulfonyl)alkylhétéroaryle, un radical haloalkyle, un radical cyanoalkyle, un radical, N-alkylaryl-alkylamino, un radical N-alkyl-N-alkylaryl-alkylamino, un radical N,N-dialkyl-alkylamino, un radical hydroxyalkyle, un radical alkylpipérazinyle, un radical alkylpipéridinyle ou un radical alkylmorpholinyle;

$R^4$ et $R^7$ représentent indépendamment un atome d'hydrogène, un atome d'halogène, un radical nitro, un radical amino ou acylamino (tel que acétylamino);

$R^5$ et $R^6$ représentent indépendamment un atome d'hydrogène, un atome d'halogène, un radical amino, un radical nitro, un radical alkyle inférieur, un radical

trifluorométhyle, un radical alcoxy, un radical aryloxy, un radical mercapto, un radical alkylthio, un radical alkylsulfinyle, un radical alkylsulfonyle, un radical sulfamoyle, un radical isothiocyanate, un radical alkylcarbonyle, un radical arylcarbonyle, un radical acylamino (tel que acétylamino), un radical cyano, un groupe carboxylique, un groupe carboxamido ou un groupe carboxyalkyle.

3 - Les composés répondant à la formule générale I, selon la revendication 1, représentés par la formule générale Ib

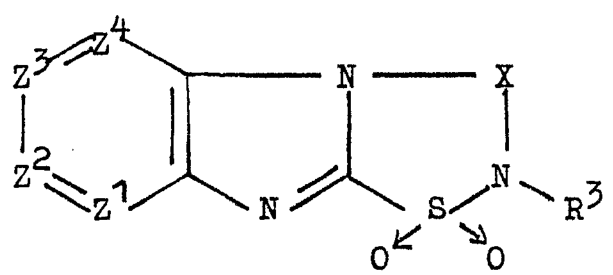

Ib

dans laquelle :

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$, peut représenter un atome d'azote;

$R^3$ représente un atome d'hydrogène, un radical alkyle linéaire ou ramifié, un radical aryle, un radical hétéroaryle, un radical alkylaryle, un radical alkylhétéroaryle, un radical mono-, bi- ou tricycloalkyle, un radical alkylamino, un radical N,N-dialkyl-alkylamino, un radical N-alkyl-alkylamino, un radical alkylcarboxyalkyle, un radical acyle, un radical alkylthio (ou sulfinyl; ou sulfonyl)alkyle, un radical alkylthio (ou sulfinyl, ou sulfonyl)aryle, un radical alkylthio (ou sulfinyl, ou sulfonyl)hétéroaryle, un radical/alkylthio (ou sulfinyl, ou sulfonyl) alkylaryle, un radical alkylthio (ou sulfinyl, ou sulfonyl)alkylhétéroaryle, un radical haloalkyle, un radical cyanoalkyle, un radical N-alkylaryl-alkylamino, un radical N-alkyl-N-alkylaryl-alkylamino, un radical hydroxyalkyle, un radical alkylpipérazinyle, un radical alkylpipéridinyle ou un radical alkylmorpholinyle;

$R^4$ et $R^7$ représentent indépendamment un atome d'hydrogène, un atome d'halogène, un radical nitro, un radical amino ou acylamino (tel que acétylamino);

$R^5$ et $R^6$ représentent indépendamment un atome d'hydrogène, un atome d'halogène, un radical amino, un radical nitro, un radical alkyle inférieur, un radical trifluorométhyle, un radical alcoxy, un radical aryloxy, un radical mercapto, un radical alkylthio, un radical alkylsulfinyle, un radical alkylsulfonyle, un radical sulfamoyle, un radical isothiocyanate, un radical alkylcarbonyle, un radical arylcarbonyle, un radical acylamino (tel que acétylamino), un radical cyano, un groupe carboxylique, un groupe carboxamido ou un groupe carboxyalkyle;

X représente $-(CH\ R^8)_n-(CH\ R^9)_m-$ ou $-CR^8=CR^9-$;

n représente 1, 2, 3 ou 4;

m représente 0 ou 1;

$R^8$ et $R^9$ représentent indépendamment un atome d'hydrogène, un radical alkyle inférieur, un radical aryle ou un radical hétéroaryle.

4 - Les composés répondant à la formule générale I, selon la revendication 1, représentés par la

formule générale Ic

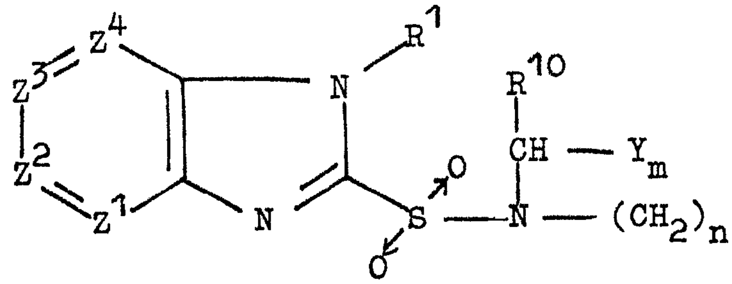

Ic

dans laquelle :

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$, peut représenter un atome d'azote;

$R^1$ représente un atome d'hydrogène, un radical alkyle linéaire ou ramifié, un radical alkylaryle, ou un radical carboxyalkyle;

Y représente $-CH_2-O-CH_2-$ ou $-CH_2-NR^{11}-CH_2-$;

n représente 1, 2, 3 ou 4;

m représente 0 ou 1;

$R^4$ et $R^7$ représentent indépendamment un atome d'hydrogène, un atome d'halogène, un radical nitro, un radical amino ou acylamino (tel que acétylamino);

$R^5$ et $R^6$ représentent indépendamment un atome d'hydrogène, un atome d'halogène, un radical amino, un radical nitro, un radical alkyle inférieur, un radical trifluorométhyle, un radical alcoxy, un radical aryloxy, un radical mercapto, un radical alkylthio, un radical alkylsulfinyle, un radical alkylsulfonyle,

un radical sulfamoyle, un radical isothiocyanate, un radical alkylcarbonyle, un radical arylcarbonyle, un radical acylamino (tel que acétylamino), un radical cyano, un groupe carboxylique, un groupe carboxamido ou un groupe carboxyalkyle;

$R^{10}$ représente un atome d'hydrogène, un radical hydroxyalkyle, un radical haloalkyle ou un radical carboxyalkyle;

$R^{11}$ représente un atome d'hydrogène, un radical alkyle inférieur ou un radical hydroxyalkyle.

5 - Les composés répondant à la formule générale I, selon la revendication 1, représentés par la formule générale Id

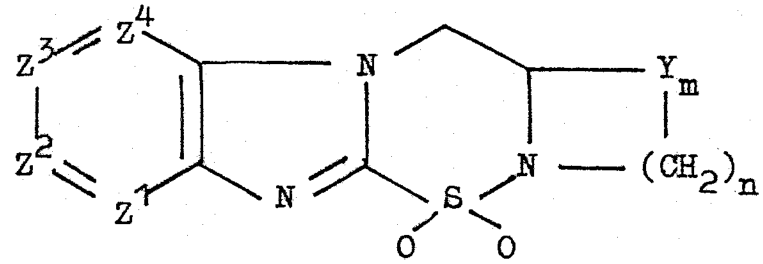

Id

dans laquelle :

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);

$Z^4$ représente un atome d'azote ou un atome de carbone lié à un autre radical $R^7$ ($C-R^7$);

avec la restriction que seulement un des groupes $Z^1$, $Z^2$, $Z^3$ ou $Z^4$, peut représenter un atome d'azote;

Y représente $-CH_2-O-CH_2-$ ou $-CH_2-NR^{11}-CH_2-$;

n représente 1, 2, 3 ou 4;

m représente 0 ou 1;

$R^4$ et $R^7$ représentent indépendamment un atome d'hydrogène,un atome d'halogène, un radical nitro, un radical amino ou acylamino (tel que acétylamino);

$R^5$ et $R^6$ représentent indépendamment un atome d'hydrogène, un atome d'halogène, un radical amino, un radical nitro, un radical alkyle inférieur, un radical trifluorométhyle, un radical alcoxy, un radical aryloxy, un radical mercapto, un radical alkylthio, un radical alkylsulfinyle, un radical alkylsulfonyle, un radical sulfamoyle, un radical isothiocyanate, un radical alkylcarbonyle, un radical arylcarbonyle, un radical acylamino (tel que acétylamino), un radical cyano, un groupe carboxylique, un groupe carboxamido ou un groupe carboxyalkyle;

$R^{11}$ représente un atome d'hydrogène, un radical alkyle inférieur ou un radical hydroxyalkyle.

6 - Les composés répondant à la formule générale I, selon la revendication 1, sélectionnés dans le groupe suivant :

- 5-benzoyl-N-éthyl-1H-benzimidazole-2-sulfonamide
- N-méthyl-1H-benzimidazole-2-sulfonamide
- N-(2-pyridyl)-1H-benzimidazole-2-sulfonamide
- N-[2-(4-morpholinoéthyl)]-1H-benzimidazole-2-sulfonamide
- N-(4-aminobutyl)-1H-benzimidazole-2-sulfonamide
- N-[3-(4-morpholinopropyl]-1H-benzimidazole-2-sulfonamide
- 5-chloro-N-éthyl-1H-benzimidazole-2-sulfonamide
- 5-phénoxy-N-éthyl-1H-benzimidazole-2-sulfonamide
- N-[2-(3,4-diméthoxyphényléthyl)]-1H-benzimidazole-2-sulfonamide
- N,N-diéthyl-1H-benzimidazole-2-sulfonamide
- N-butyl-1H-benzimidazole-2-sulfonamide
- N-éthyl-4,7-dichloro-5,6-diméthyl-1H-benzimidazole-2-sulfonamide

- N-(4-chlorophényl)-1H-benzimidazole-2-sulfonamide
- 5-chloro-N-(4-chlorophényl)-1H-benzimidazole-2-sulfonamide
- 5-chloro-N-(4-diéthylamino-1-méthylbutyl)-1H-benzimidazole-2-sulfonamide
- 4-chloro-5,6-diméthyl-N-éthyl-1H-benzimidazole-2-sulfonamide
- 4-nitro-1H-benzimidazole-2-sulfonamide
- N-éthyl-4-nitro-1H-benzimidazole-2-sulfonamide
- N-(4-diéthylamino-1-méthylbutyl)-1H-benzimidazole-2-sulfonamide
- N-(cyanométhyl)-1H-benzimidazole-2-sulfonamide
- N-cyclohexyl-1H-benzimidazole-2-sulfonamide
- N-(1-naphtyl)-1H-benzimidazole-2-sulfonamide
- N-éthyl-5-trifluorométhyl-1H-benzimidazole-2-sulfonamide
- N-(1-adamantyl)-1H-benzimidazole-2-sulfonamide
- 2-(sulfonyl-2-hydroxyméthyl-1-pipéridinyl)-1H-benzimidazole
- N-(4-méthoxybenzyl)-1H-benzimidazole-2-sulfonamide
- N-(5-méthyl-3-isoxazolyl)-1H-benzimidazole-2-sulfonamide
- N-(2-pyridylméthyl)-1H-benzimidazole-2-sulfonamide
- N-[2-(2-pyridyléthyl)]-1H-benzimidazole-2-sulfonamide
- 5-benzoyl-N-(4-diéthylamino-1-méthylbutyl)-1H-benzimidazole-2-sulfonamide
- 5-nitro-1H-benzimidazole-2-sulfonamide
- N-éthyl-5-nitro-1H-benzimidazole-2-sulfonamide
- N-[2-(4-morpholinoéthyl)]-5-nitro-1H-benzimidazole-2-sulfonamide
- N,N-diéthyl-5-nitro-1H-benzimidazole-2-sulfonamide
- N-[3-(4-morpholinopropyl)]-5-nitro-1H-benzimidazole-2-sulfonamide
- N-butyl-4-nitro-1H-benzimidazole-2-sulfonamide
- N-(2-hydroxy-1-éthyl)-4-nitro-1H-benzimidazole-2-

-sulfonamide

- N-(carboxyéthylméthyl)-4-nitro-1H-benzimidazole-2-sulfonanide
- N-éthyl-N,4-dinitro-1H-benzimidazole-2-sulfonamide
- N-[2-(4-morpholinoéthyl)]-4-nitro-1H-benzimidazole-2-sulfonamide
- N-acétyl-N-éthyl-4-nitro-1H-benzimidazole-2-sulfonamide
- N-acétyl-4-nitro-1H-benzimidazole-2-sulfonamide
- 2-[sulfonyl-4-(2-hydroxy-1-éthyl)-pipérazinyl]-4-nitro-1H-benzimidazole
- N-(4-fluorophényl)-4-nitro-1H-benzimidazole-2-sulfonamide
- 2-[sulfonyl-2-(carboxyméthyl)-pyrrolidinyl]-4-nitro-1H-benzimidazole
- N-méthyl-4-nitro-1H-benzimidazole-2-sulfonamide
- N-propyl-4-nitro-1H-benzimidazole-2-sulfonamide
- N-isopropyl-4-nitro-1H-benzimidazole-2-sulfonamide
- N-éthylthioéthyl-4-nitro-1H-benzimidazole-2-sulfonamide
- N-éthylsulfonyléthyl-4-nitro-1H-benzimidazole-2-sulfonamide
- N-[3-(2-oxohexaméthyléniminyl)]-4-nitro-1H-benzimidazole-2-sulfonamide
- N-[1-[1,3-bis(carboxyéthyl)propyl]]-4-nitro-1H-benzimidazole-2-sulfonamide
- 2-[sulfonyl-4-méthyl-pipérazinyl]-1H-benzimidazole
- 5-chloro-N-cyclohexyl-1H-benzimidazole-2-sulfonamide
- N-(2-diéthylamino-1-éthyl)-1H-benzimidazole-2-sulfonamide
- N-éthyl-1H-imidazo[4,5-b]pyridine-2-sulfonamide
- N-butyl-1H-imidazo[4,5-b]pyridine-2-sulfonamide
- N-butyl-1H-imidazo[4,5-c]pyridine-2-sulfonamide
- 1,N-diéthyl-1H-benzimidazole-2-sulfonamide
- 1,N,N-triéthyl-1H-benzimidazole-2-sulfonamide

- 1,N-diméthyl-N-éthyl-1H-benzimidazole-2-sulfonamide
- 1-benzoylméthyl-N-éthyl-1H-benzimidazole-2-sulfonamide
- 1-acétylméthyl-N-éthyl-1H-benzimidazole-2-sulfonamide
- N-éthyl-1-(2-pyridylcarbonylméthyl)-1H-benzimidazole-2-sulfonamide
- 5-chloro-N-(4-diéthylamino-1-méthylbutyl)-1-éthyl-1H-benzimidazole-2-sulfonamide
- 6-chloro-N-(4-diéthylamino-1-méthylbutyl)-1-éthyl-1H-benzimidazole-2-sulfonamide
- 1,N-diéthyl-4-nitro-1H-benzimidazole-2-sulfonamide
- 1,N,N-triéthyl-6-nitro-1H-benzimidazole-2-sulfonamide
- 1,N,N-triéthyl-5-nitro-1H-benzimidazole-2-sulfonamide
- 5-chloro-1,N-diéthyl-N-(4-diéthylamino-1-méthylbutyl)-1H-benzimidazole-2-sulfonamide
- 6-chloro-1,N-diéthyl-N-(4-diéthylamino-1-méthylbutyl)-1H-benzimidazole-2-sulfonamide
- 1,N,N-triéthyl-4-nitro-1H-benzimidazole-2-sulfonamide
- 1,N,N-triéthyl-7-nitro-1H-benzimidazole-2-sulfonamide
- 1,N-diéthyl-5,N-dinitro-1H-benzimidazole-2-sulfonamide
- 1,N-diéthyl-6,N-dinitro-1H-benzimidazole-2-sulfonamide
- 1,N-diéthyl-5-nitro-1H-benzimidazole-2-sulfonamide
- 1,N-diéthyl-6-nitro-1H-benzimidazole-2-sulfonamide
- 5-amino-1H-benzimidazole-2-sulfonamide
- 4-amino-1H-benzimidazole-2-sulfonamide
- 4-amino-N-(carboxyéthylméthyl)-1H-benzimidazole-2-sulfonamide
- 4-amino-N-éthyl-1H-benzimidazole-2-sulfonamide
- 4-acétylamino-N-éthyl-1H-benzimidazole-2-sulfonamide
- 3,4-dihydro-2H-2-éthyl-[1,2,5]-thiadiazino[5,6-a]-benzimidazole 1,1-dioxyde
- 3,4-dihydro-2H-[1,2,5]-thiadiazino[5,6-a]benzimidazole 1,1-dioxyde
- 3,4-dihydro-2H-2-méthyl-[1,2,5]-thiadiazino[5,6-a]-benzimidazole 1,1-dioxyde
- 3,4,5-trihydro-2H-2-méthyl-[1,2,6]-thiadiazépino[6,7-a]

benzimidazole 1,1-dioxyde

- 3,4,5-trihydro-2H-2-éthyl-[1,2,6]-thiadiazépino-[6,7-a]benzimidazole 1,1-dioxyde
- 3,4,5,6-tétrahydro-2H-2-éthyl-[1,2,7]-thiadiazocino-[7,8-a]benzimidazole 1,1-dioxyde
- 3,4-dihydro-2H-[3-(4-morpholinopropyl)]-[1,2,5]-thiadiazino[5,6-a]benzimidazole 1,1-dioxyde
- 3,4,5-trihydro-2H-[2-(4-morpholinoéthyl)]-[1,2,6]-thiadiazépino[6,7-a]benzimidazole 1,1-dioxyde
- 3,4-dihydro-2H-2-éthyl-8-phénoxy[1,2,5]-thiadiazino-[5,6-a]benzimidazole 1,1-dioxyde
- 3,4-dihydro-2H-2-éthyl-7-phénoxy-[1,2,5]-thiadiazino-[5,6-a]benzimidazole 1,1-dioxyde
- 8-chloro-3,4-dihydro-2H-2-éthyl-7-phénoxy-[1,2,5]-thiadiazino[5,6-a]benzimidazole 1,1-dioxyde
- 7-chloro-3,4-dihydro-2H-2-éthyl-7-phénoxy-[1,2,5]-thiadiazino[5,6-a]benzimidazole 1,1-dioxyde
- 3,4,5-trihydro-2H-[1,2,6]-thiadiazépino[6,7-a]benzimidazole 1,1-dioxyde
- 3,4-dihydro-2H-2-[2-(3,4-diméthoxyphényléthyl)]-[1,2,5]-thiadiazino[5,6-a]benzimidazole 1,1-dioxyde
- 2H-2-butyl-3,4-dihydro-[1,2,5]-thiadiazino[5,6-a]-benzimidazole 1,1-dioxyde
- 6,9-dichloro-3,4-dihydro-7,8-diméthyl-2H-2-éthyl-[1,2,5]-thiadiazino[5,6-a]benzimidazole 1,1-dioxyde
- 8-chloro-3,4-dihydro-2H-2-(4-diéthylamino-1-méthylbutyl)-[1,2,5]-thiadiazino[5,6-a]benzimidazole 1,1-dioxyde
- 7-chloro-3,4-dihydro-2H-2-(4-diéthylamino-1-méthylbutyl)-[1,2,5]-thiadiazino[5,6-a]benzimidazole 1,1-dioxyde
- 2H-2-(4-chlorophényl)-3,4-dihydro-[1,2,5]-thiadiazino-[5,6-a]benzimidazole 1,1-dioxyde
- 3,4-dihydro-2H-2-(5-méthyl-3-isoxazolyl)-[1,2,5]-thiadiazino[5,6-a]benzimidazole 1,1-dioxyde

- 2H-2-(4-diéthylamino-1-méthylbutyl)-3,4-dihydro-[1,2,5]-thiadiazino[5,6-a]benzimidazole 1,1-dioxyde
- 3,4-dihydro-2H-2-éthyl-9-nitro-[1,2,5]-thiadiazino-[5,6-a]benzimidazole 1,1-dioxyde
- 3,4-dihydro-2H-2-éthyl-6-nitro-[1,2,5]-thiadiazino-[5,6-a]benzimidazole 1,1-dioxyde
- 3-hydro-2H-2-méthyl-6-nitro-[1,2,4]-thiadiazolo-[4,5-a]benzimidazole 1,1-dioxyde
- 2H-2-cyclohexyl-3,4-dihydro-[1,2,5]-thiadiazino-[5,6-a]benzimidazole 1,1-dioxyde
- 3,4-dihydro-2H-2-(1-naphtyl)-[1,2,5]-thiadiazino-[5,6-a]benzimidazole 1,1-dioxyde
- 3,4-dihydro-2H-2-éthyl-7-trifluorométhyl-[1,2,5]-thiadiazino[5,6-a]benzimidazole 1,1-dioxyde
- 3,4-dihydro-2H-2-éthyl-8-trifluorométhyl-[1,2,5]-thiadiazino[5,6-a]benzimidazole 1,1-dioxyde
- 2H-2-(1-adamantyl)-3,4-dihydro-[1,2,5]-thiadiazino-[5,6-a]benzimidazole 1,1-dioxyde
- 7-benzoyl-3,4-dihydro-2H-2-éthyl-[1,2,5]-thiadiazino-[5,6-a]benzimidazole 1,1-dioxyde
- 8-benzoyl-3,4-dihydro-2H-2-éthyl-[1,2,5]-thiadiazino-[5,6-a]benzimidazole 1,1-dioxyde
- 3,4-dihydro-2H-2-(4-méthoxybenzyl)-[1,2,5]-thiadiazino-[5,6-a]benzimidazole 1,1-dioxyde
- 3,4-dihydro-2H-2-(2-pyridylméthyl)-[1,2,5]-thiadiazino[5,6-a]benzimidazole 1,1-dioxyde
- 3,4-dihydro-2H-2-[2-(2-pyridyléthyl)]-[1,2,5]-thiadiazino[5,6-a]benzimidazole 1,1-dioxyde
- 8-chloro-3,4-dihydro-2H-2-cyclohexyl-[1,2,5]-thiadiazino[5,6-a]benzimidazole 1,1-dioxyde
- 7-chloro-3,4-dihydro-2H-2-cyclohexyl-[1,2,5]-thiadiazino[5,6-a]benzimidazole 1,1-dioxyde
- 2-(2-diéthylamino-1-éthyl)-3,4-dihydro-2H-[1,2,5]-thiadiazino[5,6-a]benzimidazole 1,1-dioxyde
- 8-chloro-3,4-dihydro-2H-[1,2,5]-thiadiazino[5,6-a]-

benzimidazole 1,1-dioxyde
- 7-chloro-3,4-dihydro-2H-[1,2,5]-thiadiazino[5,6-a]-benzimidazole 1,1-dioxyde
- 7,8-dichloro-3,4-dihydro-2H-[1,2,5]-thiadiazino-[5,6-a]benzimidazole 1,1-dioxyde
- 2-butyl-7,8-dichloro-3,4-dihydro-2H-9-nitro-[1,2,5]-thiadiazino[5,6-a]benzimidazole 1,1-dioxyde
- 3,4-dihydro-2H-2-éthyl-7-nitro-[1,2,5]-thiadiazino-[5,6-a]benzimidazole 1,1-dioxyde
- 3,4-dihydro-2H-2-éthyl-8-nitro-[1,2,5]-thiadiazino-[5,6-a]benzimidazole 1,1-dioxyde
- 3,4-dihydro-2H-2-méthyl-7-nitro-[1,2,5]-thiadiazino-[5,6-a]benzimidazole 1,1-dioxyde
- 3,4-dihydro-2H-2-méthyl-8-nitro-[1,2,5]-thiadiazino-[5,6-a]benzimidazole 1,1-dioxyde
- 3,4,5-trihydro-2H-2-éthyl-8-nitro-[1,2,6]-thiadiazépino[6,7-a]benzimidazole 1,1-dioxyde
- 3,4,5-trihydro-2H-2-éthyl-9-nitro-[1,2,6]-thiadiazépino[6,7-a]benzimidazole 1,1-dioxyde
- 2H-2-butyl-3,4-dihydro-7-nitro-[1,2,5]-thiadiazino-[5,6-a]benzimidazole 1,1-dioxyde
- 2H-2-butyl-3,4-dihydro-8-nitro-[1,2,5]-thiadiazino-[5,6-a]benzimidazole 1,1-dioxyde
- 3,4-dihydro-2H-8-nitro-2-[2-(2-pyridyléthyl)]-[1,2,5]-thiadiazino[5,6-a]benzimidazole 1,1-dioxyde
- 3,4-dihydro-2H-7-nitro-2-[2-(2-pyridyléthyl)]-[1,2,5]-thiadiazino[5,6-a]benzimidazole 1,1-dioxyde
- 3,4-dihydro-2H-8-nitro-2-(2-pyridylméthyl)-[1,2,5]-thiadiazino[5,6-a]benzimidazole 1,1-dioxyde
- 3,4-dihydro-2H-7-nitro-2-(2-pyridylméthyl)-[1,2,5]-thiadiazino[5,6-a]benzimidazole 1,1-dioxyde
- 3,4,5,6-tétrahydro-2H-2-éthyl-9-nitro-[1,2,7]-thiadiazozino[7,8-a]benzimidazole 1,1-dioxyde
- 3,4,5,6-tétrahydro-2H-2-éthyl-10-nitro-[1,2,7]-thiadiazozino[7,8-a]benzimidazole 1,1-dioxyde

- 3,4-dihydro-2H-2-[3-(4-morpholinopropyl)]-7-nitro-[1,2,5]-thiadiazino[5,6-a]benzimidazole 1,1-dioxyde
- 3,4-dihydro-2H-2-[3-(4-morpholinopropyl)]-8-nitro-[1,2,5]-thiadiazino[5,6-a]benzimidazole 1,1-dioxyde
- 2H-2-(4-diéthylamino-1-méthylbutyl)-3,4-dihydro-7-nitro-[1,2,5]-thiadiazino[5,6-a]benzimidazole 1,1-dioxyde
- 2H-2-(4-diéthylamino-1-méthylbutyl)-3,4-dihydro-8-nitro-[1,2,5]-thiadiazino[5,6-a]benzimidazole 1,1-dioxyde
- 3,4-dihydro-2H-7-nitro-[1,2,5]-thiadiazino[5,6-a]-benzimidazole 1,1-dioxyde
- 3,4-dihydro-2H-8-nitro-[1,2,5]-thiadiazino[5,6-a]-benzimidazole 1,1-dioxyde
- 2H-2-(1-adamantyl)-3,4-dihydro-7-nitro-[1,2,5]-thiadiazino[5,6-a]benzimidazole 1,1-dioxyde
- 2H-2-(1-adamantyl)-3,4-dihydro-8-nitro-[1,2,5]-thiadiazino[5,6-a]benzimidazole 1,1-dioxyde
- 7-amino-3,4-dihydro-2H-2-éthyl-[1,2,5]-thiadiazino-[5,6-a]benzimidazole 1,1-dioxyde
- 3,4-dihydro-2H-2-éthyl-7-isothiocyanato-[1,2,5]-thiadiazino[5,6-a]benzimidazole 1,1-dioxyde
- 7-acétylamino-3,4-dihydro-2H-2-éthyl-[1,2,5]-thiadiazino[5,6-a]benzimidazole 1,1-dioxyde
- 2-carboxyéthylméthyl-8-chloro-3,4-dihydro-2H-[1,2,5]-thiadiazino[5,6-a]benzimidazole 1,1-dioxyde
- 2-carboxyéthylméthyl-7-chloro-3,4-dihydro-2H-[1,2,5]-thiadiazino[5,6-a]benzimidazole 1,1-dioxyde
- 8-chloro-2-(6-chloro-1-hexyl)-3,4-dihydro-2H-[1,2,5]-thiadiazino[5,6-a]benzimidazole 1,1-dioxyde
- 7-chloro-2-(6-chloro-1-hexyl)-3,4-dihydro-2H-[1,2,5]-thiadiazino[5,6-a]benzimidazole 1,1-dioxyde
- 8-chloro-2-(2-chloro-1-éthyl)-3,4-dihydro-2H-[1,2,5]-thiadiazino[5,6-a]benzimidazole 1,1-dioxyde
- 7-chloro-2-(2-chloro-1-éthyl)-3,4-dihydro-2H-[1,2,5]-

thiadiazino[5,6-a]benzimidazole 1,1-dioxyde

- 2-(3-chloro-1-propyl)-3,4-dihydro-2H-7-nitro-[1,2,5]-thiadiazino[5,6-a]benzimidazole 1,1-dioxyde
- 8-chloro-2-(4-cyano-1-butyl)-3,4-dihydro-2H-[1,2,5]-thiadiazino[5,6-a]benzimidazole 1,1-dioxyde
- 7-chloro-2-(4-cyano-1-butyl)-3,4-dihydro-2H-[1,2,5]-thiadiazino[5,6-a]benzimidazole 1,1-dioxyde
- 2-{3-[N-méthyl-N-[2-(3,4-diméthoxyphényl)éthyl]]-propylamino}-3,4-dihydro-2H-7-nitro-[1,2,5]-thiadiazino[5,6-a]benzimidazole 1,1-dioxyde
- 8-chloro-2-(6-diéthylamino-1-hexyl)-3,4-dihydro-2H-[1,2,5]-thiadiazino[5,6-a]benzimidazole 1,1-dioxyde
- 7-chloro-2-(6-diéthylamino-1-hexyl)-3,4-dihydro-2H-[1,2,5]-thiadiazino[5,6-a]benzimidazole 1,1-dioxyde
- 8-chloro-2-cyanométhyl-3,4-dihydro-2H-[1,2,5]-thiadiazino[5,6-a]benzimidazole 1,1-dioxyde
- 7-chloro-2-cyanométhyl-3,4-dihydro-2H-[1,2,5]-thiadiazino[5,6-a]benzimidazole 1,1-dioxyde
- 8-chloro-2-(2-diéthylamino-1-éthyl)-3,4-dihydro-2H-[1,2,5]-thiadiazino[5,6-a]benzimidazole 1,1-dioxyde
- 7-chloro-2-(2-diéthylamino-1-éthyl)-3,4-dihydro-2H-[1,2,5]-thiadiazino[5,6-a]benzimidazole 1,1-dioxyde
- 7-chloro-2-(6-éthylamino-1-hexyl)-3,4-dihydro-2H-[1,2,5]-thiadiazino[5,6-a]benzimidazole 1,1-dioxyde
- 7-chloro-2-(2-pyridyl)-3,4-dihydro-2H-[1,2,5]-thiadiazino[5,6-a]benzimidazole 1,1-dioxyde
- 3,4-dihydro-2H-2-éthyl-3-méthyl-[1,2,5]-thiadiazino-[5,6-a]benzimidazole 1,1-dioxyde
- 3,4-dihydro-2H-2-éthyl-3-méthyl-7-nitro-[1,2,5]-thiadiazino[5,6-a]benzimidazole 1,1-dioxyde
- 3,4-dihydro-2H-2-éthyl-3-méthyl-8-nitro-[1,2,5]-thiadiazino[5,6-a]benzimidazole 1,1-dioxyde
- 2H-2-butyl-3,4-dihydro-pyrido[3',2':4,5]imidazo[1,2-e][1,2,5]-thiadiazine 1,1-dioxyde
- 2-butyl-3,4-dihydro-2H-pyrido[3',4':4,5]imidazo[1,2-e]

[1,2,5]-thiadiazine 1,1-dioxyde

- 1,2,3,4,13,14-hexahydro-pyrido[1',2':2,3][1,2,5]-thiadiazino[5,6-a]benzimidazole 6,6-dioxyde
- 1,2,3,4,13,14-hexahydro-9-nitro-pyrido[1',2':2,3][1,2,5]-thiadiazino[5,6-a]benzimidazole 6,6-dioxyde
- 1,2,3,4,13,14-hexahydro-10-nitro-pyrido[1',2':2,3][1,2,5]-thiadiazino[5,6-a]benzimidazole 6,6-dioxyde
- 2H-2-éthyl-[1,2,5]-thiadiazino[5,6-a]benzimidazole 1,1-dioxyde
- 2H-2-éthyl-3-méthyl-[1,2,5]-thiadiazino[5,6-a]benzimidazole 1,1-dioxyde
- 2H-2-éthyl-3-phényl-[1,2,5]-thiadiazino[5,6-a]benzimidazole 1,1-dioxyde
- 2H-2-éthyl-7-nitro-3-(4-nitrophényl)-[1,2,5]-thiadiazino[5,6-a]benzimidazole 1,1-dioxyde.

7 - Procédé de préparation des composés selon l'une des revendications 2, 4 et 6, caractérisé par la mise en oeuvre des opérations suivantes :

par réaction d'un composé de formule générale II en suspension dans l'eau ou dans l'acide acétique de 10 à 90 % dans l'eau

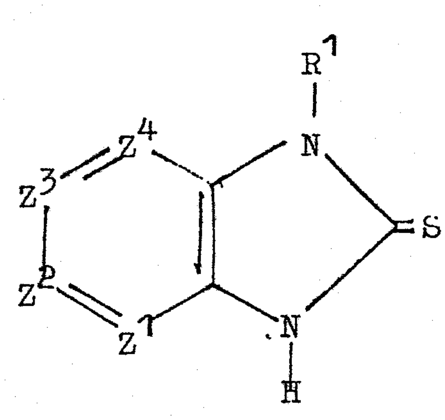

II

dans laquelle

$Z^1$ représente un atome d'azote ou un atome de carbone lié à un autre radical $R^4$ ($C-R^4$);

$Z^2$ représente un atome d'azote ou un atome de carbone lié à un autre radical $R^5$ ($C-R^5$);

$Z^3$ représente un atome d'azote ou un atome de carbone lié à un autre radical $R^6$ ($C-R^6$);
$Z^4$ représente un atome d'azote ou un atome de carbone lié à un autre radical $R^7$ ($C-R^7$);
avec la restriction que seulement un des groupes $Z^1$, $Z^2$, $Z^3$ ou $Z^4$, peut représenter un atome d'azote;
$R^1$ représente un atome d'hydrogène, un radical alkyle linéaire ou ramifié, un radical alkylaryle, un radical alkylcarbonylalkyle, un radical alkylcarbonylaryle, un radical alkylcarbonylhétéroaryle ou un radical cycloalkyle;
$R^4$ et $R^7$ représentent indépendamment un atome d'hydrogène, un atome d'halogène, un radical nitro, un radical amino ou acylamino (tel que acétylamino);
$R^5$ et $R^6$ représentent indépendamment un atome d'hydrogène, un atome d'halogène, un radical amino, un radical nitro, un radical alkyle inférieur, un radical trifluorométhyle, un radical alcoxy, un radical aryloxy, un radical mercapto, un radical alkylthio, un radical alkylsulfinyle, un radical alkylsulfonyle, un radical sulfamoyle, un radical isothiocyanate, un radical alkylcarbonyle, un radical arylcarbonyle, un radical acylamino (tel que acétylamino), un radical cyano, un groupe carboxylique, un groupe carboxamido ou un groupe carboxyalkyle;

avec un courant de chlore gazeux pendant un temps compris entre 10 minutes et deux heures on obtient le chlorure d'acide sulfonique correspondant. La réaction s'effectue à des températures comprises entre 0°C et 15°C, en pouvant utiliser dans certains cas comme catalyseur un acide de Lewis, tel que le chlorure ferrique, le chlorure de zinc ou le chlorure d'étain (IV). On filtre le chlorure de l'acide formé et immédiatement on l'additionne à une solution du composé de formule générale III dans un solvant approprié, tel que l'eau,

les alcools, l'acétone ou les mélanges de ceux-ci

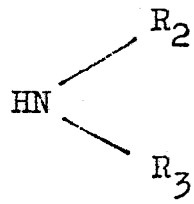

III

dans laquelle

$R^2$ représente un atome d'hydrogène ou un radical alkyle inférieur linéaire ou ramifié en $C_1$ à $C_5$;

$R^3$ représente un atome d'hydrogène, un radical alkyle linéaire ou ramifié, un radical aryle, un radical hétéroaryle, un radical alkylaryle, un radical alkylhétéroaryle, un radical mono-, bi- ou tricycloalkyle, un radical alkylamino, un radical N-alkyl-alkylamino, un radical alkylcarboxyalkyle, un radical acyle, un radical alkylthio (ou sulfinyl, ou sulfonyl)alkyle, un radical alkylthio (ou sulfinyl, ou sulfonyl)aryle, un radical alkylthio (ou sulfinyl, ou sulfonyl)hétéroaryle, un radical alkylthio (ou sulfinyl, ou sulfonyl)alkylaryle, un radical alkylthio (ou sulfinyl, ou sulfonyl)alkylhétéroaryle, un radical haloalkyle, un radical cyanoalkyle, un radical N-alkylaryl-alkylamino, un radical N-alkyl-N-alkylaryl-alkylamino,un radical N,N-dialkyl-alkylamino, un radical hydroxyalkyle, un radical alkylpipérazinyle, un radical alkylpipéridinyle ou un radical alkylmorpholinyle;

$R^2$ et $R^3$ peuvent former ensemble une liaison représentée par un groupe $-(CH_2)_n-Y_m-CH\ R^{10}-$;

Y représente $-CH_2-O-CH_2-$ ou $-CH_2-NR^{11}-CH_2-$;

n représente 1, 2, 3 ou 4;

m représente 0 ou 1;

$R^{10}$ représente un atome d'hydrogène, un radical hydroxyalkyle, un radical haloalkyle ou un radical carboxyalkyle;

$R^{11}$ représente un atome d'hydrogène, un radical alkyle

inférieur ou un radical hydroxyalkyle

La réaction s'effectue à des températures comprises entre -10°C et 40°C pendant 1 à 4 heures. Dans les composés préparés de formules générales Ia et Ic, $Z^1$, $Z^2$, $Z^3$, $Z^4$, $R^1$ à $R^7$, Y, n, m, $R^{10}$ et $R^{11}$ ont les significations mentionnées dans les revendications 2 et 4.

8 - Procédé de préparation des composés selon l'une des revendications 3 et 6, caractérisé par la mise en oeuvre d'au moins l'une des opérations suivantes :

a) Par réaction d'un composé de formule générale Ia, dans laquelle $Z^1$, $Z^2$, $Z^3$, $Z^4$, $R^3$ à $R^7$ ont les significations mentionnées dans la revendication 2 et $R^1$ et $R^2$ représentent un atome d'hydrogène, avec un composé de formule générale IV ou avec un composé de formule générale V

$$A^1-(CHR^8)_m-(CHR^8)_{n-1}-(CHR^9)_m-A^2 \qquad IV$$

$$A^1 - CR^8 = CR^9 - A^2 \qquad V$$

dans lesquelles

$A^1$ et $A^2$ représentent indépendamment un atome de chlore, un atome de brome ou un atome d'iode;

$R^8$ et $R^9$ représentent indépendamment un atome d'hydrogène, un radical alkyle inférieur, un radical aryle ou un radical hétéroaryle;

n représente 1, 2, 3 ou 4;

m représente 0 ou 1;

dans les composés préparés, de formule générale Ib, $Z^1$, $Z^2$, $Z^3$, $Z^4$, X et $R^3$ à $R^9$ ont les significations mentionnées dans la revendication 3,

b) Par réaction d'un composé de formule générale Ia, dans laquelle $Z^1$, $Z^2$, $Z^3$, $Z^4$, Y, n, m, $R^3$ à $R^7$ et $R^{11}$ ont les significations mentionnées dans la revendi-

cation 2, $R^1$ représente un radical alkylcarbonylalkyle, un radical alkylcarbonylaryle ou un radical alkylcarbonylhétéroaryle et $R^2$ représente un atome d'hydrogène, avec un composé deshydratant tel que l'acide polyphosphorique, le chlorure de thionyle, l'acide p-toluènesulfonique, le tétrachlorure de titanium ou l'oxychlorure de phosphore;

dans les composés préparés de formule générale Ib, $Z^1$, $Z^2$, $Z^3$, $Z^4$, Y, n, m, $R^3$ à $R^7$ et $R^{11}$ ont les significations mentionnées dans la revendication 3, X représente $-CR^8{=}CR^9-$, $R^8$ représente un atome d'hydrogène et $R^9$ représente un radical alkyle inférieur, un radical aryle ou un radical hétéroaryle;

c) Par réaction d'un composé de formule générale Ib, dans laquelle $Z^1$ à $Z^4$ et $R^4$ à $R^9$ ont les significations mentionnées dans la revendication 3, X représente $-(CHR^8)_n-(CHR^9)_m-$, n représente 1, 2, 3 ou 4, m représente 0 ou 1 et $R^3$ représente un atome d'hydrogène, avec un composé de formule générale VIII

$$A^1 - R^3 \qquad \text{VIII}$$

dans laquelle

$A^1$ représente un atome de fluor, un atome de chlore, un atome de brome ou un atome d'iode;

$R^3$ représente un radical alkyle linéaire ou ramifié, un radical hétéroaryle, un radical alkylaryle, un radical alkylhétéroaryle, un radical mono-, bi- ou tricycloalkyle, un radical alkylamino, un radical N-alkyl-alkylamino, un radical alkylcarboxyalkyle, un radical acyle, un radical alkylthio (ou sulfinyl, ou sulfonyl)alkyle, un radical alkylthio (ou sulfinyl, ou sulfonyl)aryle, un radical alkylthio (ou sulfinyl, ou sulfonyl)hétéroaryle, un radical alkylthio (ou sulfinyl, ou sulfonyl)alkylaryle, un radical alkylthio (ou sulfinyl, ou sulfonyle)alkylhétéroaryle, un radical haloalkyle, un radical cyanoalkyle, un radical N-alkylaryl-alkylamino, un radical N-alkyl-N-alkylaryl-alkylamino, un radical N,N-dialkyl-alkylamino, un radical hydroxyalkyle, un radical alkylpipérazinyle, un radical alkylpipéridinyle ou un radical alkylmorpholinyle;

Dans les composés préparés de formule générale Ib, $Z^1$ à $Z^4$ et $R^4$ à $R^9$ ont les significations mentionnées dans la revendication 3 et $R^3$ a la signification mentionnée dans la revendication 3 excepté hydrogène.

9 - Procédé de préparation des composés selon l'une des revendications 5 et 6, caractérisé par la mise en oeuvre de la réaction suivante :

Par réaction d'un composé de formule générale Ic, dans laquelle $Z^1$, $Z^2$, $Z^3$, $Z^4$, Y, n, m, $R^4$ à $R^7$ et $R^{11}$ ont les significations mentionnées dans la revendication 4; $R^1$ représente un atome d'hydrogène et $R^{10}$ représente un groupe hydroxyméthyle ($CH_2OH$), avec un composé déshydratant tel que l'acide polyphosphorique ou le chlorure de thionyle;
dans les composés préparés de formule générale Id, $Z^1$, $Z^2$, $Z^3$, $Z^4$, Y, n, m, $R^4$ à $R^7$ et $R^{11}$ ont les significations mentionnées dans la revendication 5.

10 - Procédé de préparation des composés selon l'une des revendications 2 et 6, caractérisé par la mise en oeuvre d'au moins l'une des opérations suivantes :

a) Par réaction d'un composé de formule générale Ia, dans laquelle $Z^1$ à $Z^4$ et $R^2$ à $R^7$ ont les significations mentionnées dans la revendication 2 et $R^1$ représente un atome d'hydrogène, avec un composé de formule générale VI

$$A^1 - R^1 \qquad \text{VI}$$

dans laquelle
$A^1$ représente un atome de chlore, un atome de brome ou un atome d'iode;

$R^1$ représente un radical alkyle linéaire ou ramifié en $C_1$ à $C_5$, un radical cycloalkyle, un radical alkylaryle, un radical alkylcarbonylalkyle, un radical carboxyalkyle, un radical alkylcarbonylaryle ou un radical alkylcarbonylhétéroaryle;

dans les composés préparés de formule générale Ia, $Z^1$ à $Z^4$ et $R^2$ à $R^7$ ont les significations mentionnées dans la revendication 2 et $R^1$ a les significations mentionnées excepté hydrogène.

b) Par réaction d'un composé de formule générale Ia, dans laquelle $Z^1$ à $Z^4$ et $R^3$ à $R^7$ ont les significations mentionnées dans la revendication 2, $R^2$ représente un atome d'hydrogène et $R^1$ représente un atome d'hydrogène, un radical alkyle linéaire ou ramifié en $C_1$ à $C_5$, un radical cycloalkyle, un radical alkylaryle, un radical alkylcarbonylalkyle, un radical carboxyalkyle, un radical alkylcarbonylaryle ou un radical alkylcarbonylhétéroaryle, avec un composé de formule générale VII

$$A^1 - R^2 \qquad \text{VII}$$

dans laquelle

$A^1$ représente un atome de chlore, un atome de brome, un atome d'iode ou un radical acyloxy;

$R^2$ représente un radical alkyle inférieur linéaire ou ramifié en $C_1$ à $C_5$ ou un radical acyle.

dans les composés préparés de formule générale Ia, $Z^1$ à $Z^4$ et $R^3$ à $R^7$ ont les significations mentionnées dans la revendication 2 et $R^1$ et $R^2$ ont les significations mentionnées précédemment excepté hydrogène.

11 - Procédé de préparation des composés selon l'une des revendications 1 et 6, caractérisé par la mise en oeuvre d'au moins l'une des opérations suivantes :

a) Par nitration d'un composé de formule générale I, dans laquelle $Z^1$ représente C-$R^4$, $Z^2$ représente C-$R^5$, $Z^3$ représente C-$R^6$, $Z^4$ représente C-$R^7$, l'un des deux

radicaux $R^5$ et $R^6$ représente un atome d'hydrogène et l'autre représente un atome d'hydrogène, un atome d'halogène, un radical nitro, un radical alkyle inférieur, un radical trifluorométhyle, un radical alcoxy, un radical aryloxy ou un radical acylamino et $R^1$ à $R^4$, $R^7$ à $R^{11}$, X, Y, n et m ont les significations mentionnées dans la revendication 1, avec un agent de nitration tel qu'un mélange d'acide nitrique et un autre acide fort comme par exemple l'acide sulfurique ou avec le tétrafluoroborate de nitrosium;
dans les composés préparés de formule générale I, $Z^1$ représente C-$R^4$, $Z^2$ représente C-$R^5$, $Z^3$ représente C-$R^6$, $Z^4$ représente C-$R^7$, l'un des deux radicaux $R^5$ et $R^6$ représente un groupe nitro et l'autre représente un atome d'hydrogène, un atome d'halogène, un radical nitro, un radical alkyle inférieur, un radical trifluorométhyle, un radical alcoxy, un radical aryloxy ou un radical acylamino et $R^1$ à $R^4$, $R^7$ à $R^{11}$, X, Y, n et m ont les significations mentionnées dans la revendication 1.

b) Par réduction d'un composé de formule générale I, dans laquelle $Z^1$ représente C-$R^4$, $Z^2$ représente C-$R^5$, $Z^3$ représente C-$R^6$, $Z^4$ représente C-$R^7$, l'un des quatre radicaux $R^4$, $R^5$, $R^6$ ou $R^7$ représente un groupe nitro et les autres représentent un atome d'hydrogène et $R^1$ à $R^3$, $R^8$ à $R^{11}$, X, Y, n et m ont les significations mentionnées dans la revendication 1, avec un agent réducteur approprié,
dans les composés préparés de formule générale I, $Z^1$ représente C-$R^4$, $Z^2$ représente C-$R^5$, $Z^3$ représente C-$R^6$, $Z^4$ représente C-$R^7$, l'un des quatre radicaux $R^4$, $R^5$, $R^6$ ou $R^7$ représente un groupe amino et les autres représentent un atome d'hydrogène et $R^1$ à $R^3$, $R^8$ à $R^{11}$, X, Y, n et m ont les significations mentionnées dans la revendication 1;

c) Par acylation d'un composé de formule géné-

rale I, dans laquelle $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un groupe amino et les autres représentent un atome d'hydrogène et $R^1$ à $R^3$, $R^8$ à $R^{11}$, X, Y, n et m ont les significations mentionnées dans la revendication 1, avec un halogénure d'acide ou un anhydride,
dans les composés préparés de formule générale I, $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un groupe acylamino et les autres représentent un atome d'hydrogène et $R^1$ à $R^3$, $R^8$ à $R^{11}$, X, Y, n et m ont les significations mentionnées dans la revendication 1;

d) Par réaction d'un composé de formule générale I, dans laquelle $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un groupe amino et les autres représentent un atome d'hydrogène et $R^1$ à $R^3$, $R^8$ à $R^{11}$, X, Y, n et m ont les significations mentionnées dans la revendication 1, avec le thiophosgène ($Cl_2CS$);
dans les composés préparés de formule générale I, $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un groupe isothiocyanate (-N=C=S) et les autres représentent un atome d'hydrogène et $R^1$ à $R^3$, $R^8$ à $R^{11}$, X, Y, n et m ont les significations mentionnées dans la revendication 1;

e) Par réaction d'un composé de formule générale I, dans laquelle $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un groupe amino et les autres représentent un atome d'hydrogène et $R^1$ à $R^3$, $R^8$ à $R^{11}$, X, Y, n et m ont les significations mentionnées revendication 1, avec le nitrite de sodium dans une solution aqueuse d'acide chlorhydrique ou tétrafluoroborique ou dans de l'acide acétique saturé d'acide chlorhydrique gazeux à une température entre -10°C et 5°C, on obtient le sel correspondant de diazonium que l'on verse sur une solution aqueuse de chlorure cuivreux ou de cyanure cuivreux ou bien sur une solution d'acide acétique saturé d'anhydride sulfureux et une quantité catalytique de chlorure cuivreux et de chlorure cuivrique; dans ce dernier cas on fait réagir le chlorure de l'acide sulfonique obtenu avec de l'ammoniaque dans les conditions habituelles de formation d'une sulfamide;
dans les composés préparés de formule générale I, $Z^1$ représente $C-R^4$, $Z^2$ représente $C-R^5$, $Z^3$ représente $C-R^6$, $Z^4$ représente $C-R^7$, l'un des quatre radicaux $R^4$, $R^5$, $R^6$ ou $R^7$ représente un atome de chlore, un groupe cyano ($C\equiv N$) ou un groupe sulfamoyle ($-SO_2NH_2$) et les autres représentent un atome d'hydrogène, et $R^1$ à $R^3$, $R^8$ à $R^{11}$, X, Y, n et m ont les significations mentionnées dans la revendication 1.

12 - A titre de médicaments, les composés de formule générale I et leurs sels thérapeutiquement acceptables selon l'une des revendications 1 à 6, en particulier à titre de médicaments destinés au traitement des maladies gastro-intestinales principalement indiqués comme inhibiteurs de la sécrétion d'acide gastrique, comme agents antiulcéreux et comme agents cytoprotecteurs.

13 - Compositions pharmaceutiques, caractérisées par le fait qu'elles contiennent, outre un support pharmaceutiquement acceptable, au moins un composé de formule générale I ou l'un de leurs sels physiologiquement acceptables, selon l'une des revendications 1 à 6.

14 - Utilisation des composés de formule générale I et de leurs sels physiologiquement acceptables selon l'une des revendications 1 à 6, pour la fabrication de médicaments destinés au traitement des maladies gastrointestinales, en particulier pour la fabrication d'agents inhibiteurs de la sécrétion d'acide gastrique, d'agents antiulcéreux et d'agents cytoprotecteurs.

Office européen des brevets

0246126

Numero de la demande

# RAPPORT DE RECHERCHE EUROPEENNE

EP 87 40 0875

## DOCUMENTS CONSIDERES COMME PERTINENTS

| Catégorie | Citation du document avec indication, en cas de besoin des parties pertinentes | Revendication concernee | CLASSEMENT DE LA DEMANDE (Int Cl 4) |
|---|---|---|---|
| X | DE-A-2 726 625 (CIBA-GEIGY)<br>* Pages 5-9 * | 1,2,6,7 | C 07 D 235/28<br>C 07 D 401/12<br>C 07 D 413/12<br>C 07 D 471/04<br>C 07 D 513/04<br>C 07 D 513/14<br>C 07 D 403/12 |
| A | JOURNAL OF MEDICINAL CHEMISTRY, vol. 13, no. 1, janvier 1970, pages 143-144, Washington D.C., US; S.M. DESHPANDE et al.: "Potential antidiabetics. Benzimidazole-2-sulfonylglycamide derivatives" | | |
| D,A | GB-A- 833 049 (OMIKRON-GAGLIARDI) | | |
| D,A | GB-A- 687 760 (AMERICAN CYANAMID) | | |

DOMAINES TECHNIQUES RECHERCHES (Int Cl 4)

C 07 D 235/00
C 07 D 401/00
C 07 D 413/00
C 07 D 471/00
C 07 D 513/00
C 07 D 403/12

Le présent rapport de recherche a été etabli pour toutes les revendications

| Lieu de la recherche | Date d achèvement de la recherche | Examinateur |
|---|---|---|
| LA HAYE | 29-07-1987 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publie a la date de dépôt ou après cette date
D : cité dans la demande
L : cité pour d'autres raisons

& : membre de la même famille, document correspondant

OEB Form 1503 03 82